# EUROPEAN PATENT SPECIFICATION

(11) **EP 2 454 374 B1**
(45) Date of publication and mention of the grant of the patent: **10.02.2016**
(21) Application number: 10800409.4
(22) Date of filing: 13.07.2010
(51) Int. Cl.: C12N 15/74

(54) **ORAL VACCINES PRODUCED AND ADMINISTERED USING EDIBLE MICRO-ORGANISM**
ORALE IMPFSTOFFE DIE UNTER VERWENDUNG VON ESSBAREM MIKROORGANISMUS HERGESTELLT UND VERABREICHT WERDEN
VACCINS ORAUX PRODUITS ET ADMINISTRÉS À L'AIDE D'UN MICROORGANISME COMESTIBLE

(30) Priority: 13.07.2009 US 224973 P; 20.11.2009 US 263215 P
(43) Date of publication of application: 23.05.2012
(73) Proprietor: Vaxgene Corporation, Hong Kong (CN)
(72) Inventor: LAM, Dominic, Man-Kit, Hong Kong (CN); XU, Yuhong, Shanghai 20030 (CN)
(74) Representative: Lecca, Patricia S.
(86) International application number: PCT/US2010/041792
(87) International publication number: WO 2011/008735

(56) References cited:
- EP-A1- 2 133 092
- US-A1- 2004 101 531
- US-A1- 2004 234 548
- US-A1- 2005 232 949
- US-A1- 2008 124 355
- LEE JONG-SOO ET AL: "Mucosal immunization with surface-displayed severe acute respiratory syndrome coronavirus spike protein on Lactobacillus casei induces neutralizing antibodies in mice", JOURNAL OF VIROLOGY, THE AMERICAN SOCIETY FOR MICROBIOLOGY, US, vol. 80, no. 8, 1 April 2006 (2006-04-01), pages 4079-4087, XP009130248, ISSN: 0022-538X, DOI: 10.1128/JVI.80.8.4079-4087.2006
- CHO ET AL: "Induction of mucosal and systemic immune responses following oral immunization of mice with Lactococcus lactis expressing human papillomavirus type 16 L1", VACCINE, ELSEVIER LTD, GB, vol. 25, no. 47, 26 October 2007 (2007-10-26), pages 8049-8057, XP022317481, ISSN: 0264-410X, DOI: 10.1016/J.VACCINE.2007.09.024
- OLIVEIRA M L S ET AL: "Induction of systemic and mucosal immune response and decrease in Streptococcus pneumoniae colonization by nasal inoculation of mice with recombinant lactic acid bacteria expressing pneumococcal surface antigen A", MICROBES AND INFECTION, ELSEVIER, PARIS, FR, vol. 8, no. 4, 1 April 2006 (2006-04-01), pages 1016-1024, XP025243225, ISSN: 1286-4579, DOI: 10.1016/J.MICINF.2005.10.020 [retrieved on 2006-04-01]
- SEEGERS J F M L: "Lactobacilli as live vaccine delivery vectors: progress and prospects", TRENDS IN BIOTECHNOLOGY, ELSEVIER PUBLICATIONS, CAMBRIDGE, GB, vol. 20, no. 12, 1 December 2002 (2002-12-01), pages 508-515, XP004393459, ISSN: 0167-7799, DOI: 10.1016/S0167-7799(02)02075-9

### SEQUENCE LISTING

<110> Lam, Dominic Man-Kit Xu, Yuhong
<120> ORAL VACCINES PRODUCED AND ADMINISTERED USING EDIBLE MICRO-ORGANISM
<130> 1415-A-PCT
<140> PCT/US2010/041792
   <141> 2010-07-13
<150> 61/263,215
   <151> 2009-11-20
<150> 61/224,973
   <151> 2009-07-13
<160> 52
<210> 1
   <211> 4
   <212> PRT
   <213> Artificial sequence
<220>
   <223> Synthetic construct
<400> 1
   Lys Asp Glu Leu
   1
<210> 2
   <211> 4
   <212> PRT
   <213> Artificial sequence
<220>
   <223> Synthetic construct
<400> 2
   His Asp Glu Leu
   1
<210> 3
   <211> 2629
   <212> DNA
   <213> Unknown
<220>
   <223> Pestivirus genus of the Flaviviridae
<220>
   <223> DNA Sequences of HCV2.5
<400> 3
   atgaaagccc tattggcatg ggcagtgata gcaatcgcgc tgtaccagcc tgtagcagcc      60
   gagaatataa ctcaatggaa cctgagtgac aacggcacaa gcggcatcca gcgagctatg     120
   tatctcagag gggtcaatag gagcttacat gggatctggc ctgaaaaaat atgcaagggg      180
   gtccccactc atctggccac tgatacggaa ctgacagaga tacgcgggat gatggacgcc     240
   agtgagagga cgaactatac gtgttgtagg ttgcagagac acgaatggaa taaacatgga     300
   tggtgtaact ggtacaacat agacccttgg attcagttaa tgaacaggac ccaagcaaat     360
   ttgacagaag gccctccaaa taaagagtgc gctgtgactt gcaggtatga caacaatacc     420
   gacgttaacg tggttaccca ggccaggaat agaccaacca ctctgactgg ctgcaagaaa     480
   gggaaaaatt tttcatttgc aggtacggtc atagagggcc catgcaattt caacgtatcc     540
   gtggaggaca tcttatatgg agaccatgag tgtggcagcc tgtttcagga cacggctctg     600
   tacctattag atggaatgac caacactata gagaaagcca ggcagggtgc ggcaagagtc     660
   acatcttggc tagggaggca actcagtacc acagggaaga agttggagag aggaagcaaa     720
   acctggttcg gtgcctatgc cctgtcacct tattgcaatg taacaaggaa aatagggtac     780
   atatggtaca cgaacaattg cactccggca tgcctcccaa aaaacacaaa aataataggc     840
   ccaggaaaat ttgataccaa cgcagaagac gggaagattc ttcatgaaat gggggccacc     900
   tatcagagtt cttgttcgtt tctctggtaa tcctgtctga cttcgcccca gaaacagcca     960
   gtacgttata cctaatctta cactatgcaa tccctcagtc ctatgaagaa cctgagggtt    1020
   gcgatatgaa ccagctcaac ctaacggtgg gacttaggac agaagatgtg gtaccatcat    1080
   cagtctggaa tattggtaaa tatgtgtgtg ttagaccaga ttggtggccg tatgaaacta    1140
   aggtggctct actgtttgaa gaggtaggac aggttataaa gctagctcta cgggcactga    1200
   gggatttaac tagagtctgg aacagtgcat caaccactgc attcctcatc tgcttgataa    1260
   aaatattaag aggacaggtt gtgcaaggta taatatggct gctgctggtg accggggcac    1320
   aagggcggtt gtcctgcaag gaagactaca ggtatgcgat atcatcaacc aatgagatag    1380
   ggccgctagg ggctgaaggt ctcactacca cctggaaaga atacaaccac ggtttgcagc    1440
   tggatgacgg gacagtcagg gccatctgca ctgcagggtc ctttaaagtt atagcactta    1500
   atgtggtcag taggagatac ctggcatcat tacacaagag ggctttgccc acctcagtaa    1560
   catttgaact cctatttgat gggactagtc cagcaataga ggagatggga gatgactttg    1620
   gatttgggct gtgccctttt gacacaaccc ccgtggtaaa agggaagtac aataccactt    1680
   tgttaaacgg tagtgctttc tacctagtct gcccaatagg atggacgggt gtcatagagt    1740
   gcacggcagt aagcctcaca accttgagaa cagaagtggt gaagaccttc aagagagaga    1800
   agcctttccc gcacagagtg gattgcgtga ccactatagt agaaaaagaa gacctgttct    1860
   actgcaagtt gggggtaatt ggacatgtgt gaaaggcaac ccggtgacct acatgggggg 1920
   gcaagtaaaa caatgcaggt ggtgcggttt tgacttcaag gagcccgatg ggctcccaca 1980
   ctaccccata ggcaagtgca tcctagcaaa tgagacgggt tacagggtag tggattccac 2040
   agactgcaac agagatggcg tcgttatcag cactgaagga gaacacgagt gcttgattgg 2100
   caacaccacc gtcaaggtac acgcgttgga tggaagactg ggccccatgc cgtgcagacc 2160
   caaagaaatt gtctctagtg cgggacctgt aaggaaaact tcctgcactt tcaattacac 2220
   aaagacacta agaaataagt actatgagcc caaggacagc tatttccagc aatatatgct 2280
   taagggcgag taccaatact ggtttgatct ggacgtgact gaccaccaca cagactactt 2340
   tgccgaattt gttgtcttgg tggtagtggc actattaggg gggaggtacg ttctgtggct 2400
   aatagtgacc tatatagttc taacagagca actcgctgct ggtttacagc taggccaagg 2460
   cgaggcggta ctgataggga acttaatcac ccacacggac aatgaggtgg tggtatactt 2520
   cttactgctc tacctgataa taagagacga gcccataaag aaatggatac tactgctgtt 2580
   tcatggaatt ctgcagatat ccagcacagt ggcggccgct cgagtctag 2629
<210> 4
   <211> 1308
   <212> DNA
   <213> Unknown
<220>
   <223> Pestivirus genus of the Flaviviridae
<220>
   <223> DNA Sequences of HCV1.25
<400> 4
   atgcggctag cctgcaagga agattacagg tatgcgatat catcaaccaa tgagataggg 60
   ccgctagggg ctgaaggtct cactaccacc tggaaagaat acaaccatgg tttgcagctg 120
   gatgacggga cagtcagggc catctgcact gcagggtcct ttaaagttat agcacttaat 180
   gtggtcagta ggagatacct ggcatcatta cacaagaggg ctttgcccac ctcagtaaca 240
   tttgaactcc tatttgatgg gactagtcca gcaatagagg agatgggaga tgactttgga 300
   tttgggctgt gcccttttga cacaaccccc gtggtaaaag ggaagtacaa taccactttg 360
   ttaaacggta gtgctttcta cctagtctgc ccaataggat ggacgggtgt catagagtgc 420
   acggcagtaa gcctcacaac cttgagaaca gaagtggtga agaccttcaa gagagagaag 480
   cctttcccgc acagagtgga ttgcgtgacc actatagtag aaaaagaaga cctgttctac 540
   tgcaagttgg ggggtaattg gacatgtgtg aaaggcaacc cggtgaccta catggggggg 600
   caagtaaaac aatgcaggtg gtgcggtttt gacttcaagg agcccgatgg gctcccacac 660
   taccccatag gcaagtgcat cctagcaaat gagacgggtt acagggtagt ggattccaca 720
   gactgcaaca gagatggcgt cgttatcagc actgaaggag aacacgagtg cttgattggc 780
   aacaccaccg tcaaggtaca cgcgttggat ggaagactgg gccccatgcc gtgcagaccc 840
   aaagaaattg tctctagtgc gggacctgta aggaaaactt cctgcacttt caattacaca 900
   aagacactaa gaaataagta ctatgagccc aaggacagct atttccagca atatatgctt 960
   aagggcgagt accaatactg atttgatctg gacgtgactg accaccacac agactacttt 1020
   gccgaatttg ttgtcttggt ggtagtggca ctattagggg ggaggtacgt tctgtggcta 1080
   atagtgacct atatagttct aacagagcaa ctcgctgctg gtttacagct aggccaaggc 1140
   gaggcggtac tgatagggaa cttaatcacc cacacggaca atgaggtggt ggtatacttc 1200
   ttactgctct acctgataat aagagacgag cccataaaga aatggatact actgctgttt 1260
   catggaattc tgcagatatc cagcacagtg gcggccgctc gagtctag 1308
<210> 5
   <211> 1266
   <212> DNA
   <213> Unknown
<220>
   <223> Pestivirus genus of the Flaviviridae
<220>
   <223> DNA Sequences of HCV1.25
<400> 5
   atgcggctag cctgcaagga agattacagg tatgcgatat catcaaccaa tgagataggg      60
   ccgctagggg ctgaaggtct cactaccacc tggaaagaat acaaccatgg tttgcagctg     120
   gatgacggga cagtcaaggc catctgcact gcagggtcct ttaaagttat agcacttaat     180
   gtggtcagta ggagatacct ggcatcatta cacaagaggg ctttgcccac ctcagtaaca     240
   tttgaactcc tatttgatgg gactagtcca gcaatagagg agatgggaga tgactttgga     300
   tttgggctgt gcccttttga cacaaccccc gtggtaaaag ggaagtacaa taccactttg     360
   ttaaacggta gtgctttcta cctagtctgc ccaataggat ggacgggtgt catagagtgc     420
   acggcagtaa gcctcacaac cttgagaaca gaagtggtga agaccttcaa gagagagaag     480
   cctttcccgc acagagtgga ttgcgtgacc actatagtag aaaaagaaga cctgttctac     540
   tgcaagttgg ggggtaattg gacatgtgtg aaaggcaacc cggtgaccta catggggggg     600
   caagtaaaac aatgcaggtg gtgcggtttt gacttcaagg agcccgatgg gctcccacac     660
   taccccatag gcaagtgcat cctagcaaat gagacgggtt acagggtagt ggattccaca     720
   gactgcaaca gagatggcgt cgttatcagc actgaaggag aacacgagtg cttgattggc     780
   aacaccaccg tcaaggtaca cgcgttggat ggaagactgg gccccatgcc gtgcagaccc     840
   aaagaaattg tctctagtgc gggacctgta aggaaaactt cctgcacttt caattacaca     900
   aagacactaa gaaataagta ctatgagccc aaggacagct atttccagca atatatgctt     960
   aagggcgagt accaatactg gtttgatctg gacgtgactg accaccacac agactacttt    1020
   gccgaatttg ttgtcttggt ggtagtggca ctattagggg ggaggtacgt tctgtggcta    1080
   atagtgacct atatagttct aacagagcaa ctcgctgctg gtttacagct aggccaaggc    1140
   gaggcggtac tgatagggaa cttaatcacc cacacggaca atgaggtggt ggtatacttc    1200
   ttactgctct acctgataat aagagacgag cccataaaga aatggatact actgctgttt    1260
   cattag                                                              1266
<210> 6
   <211> 1266
   <212> DNA
   <213> Unknown
<220>
   <223> Pestivirus genus of the Flaviviridae
<220>
   <223> DNA Sequences of HCV1.25
<400> 6
   atgcggcttg cctgtaagga agattacagg tatgcgatat catcaaccaa tgagataggg      60
   ccgctagggg ctgaaggtct cactaccacc tggaaagaat acaaccatgg tttgcagctg     120
   gatgacggga cagtcagggc catctgcact gcagggtcct ttaaagttat agcacttaat     180
   gtggtcagta ggagatacct ggcatcatta cacaagaggg ctttgcccac ctcagtaaca     240
   tttgaactcc tatttgatgg gactagtcca gcaatagagg agatgggaga tgactttgga     300
   tttggacttt gtccttttga cacaacccca gtggttaaag gtaagtacaa taccactttg     360
   ttaaacggta gtgctttcta cctagtctgc ccaataggat ggacgggtgt catagagtgc     420
   acggcagtaa gcctcacaac cttgagaaca gaagtggtga agaccttcaa gagagagaag     480
   cctttcccgc acagagtgga ttgcgtgacc actatagtag aaaaagaaga cctgttctac     540
   tgcaagttgg ggggtaattg gacatgtgtg aaaggcaacc cggtgaccta catgggtgga     600
   caagttaaac aatgtaggtg gtgtggtttt gacttcaagg agcccgattg gctctcacac     660
   taccctattg gtaagtgcat cctagcaaat gagacgggtt acagggtagt ggattccaca     720
   gactgcaaca gagatggcgt cgttatcagc actgaaggag aacacgagtg cttgattggc     780
   aacaccaccg tcaaggtaca cgcgttggat ggaagacttg gtccaatgcc gtgtagaccg     840
   aaagaaattg tctctagtgc gggacctgta aggaaaactt cctgcacttt caattacaca     900
   aagacactaa gaaataagta ctatgagccc aaggacagct atttccagca atatatgctt     960
   aagggcgagt accaatactg gtttgatctg gacgtgactg accaccacac agactacttt    1020
   gccgaatttg ttgtcttggt ggttgtggca cttttaggag gtaggtacgt tctttggcta    1080
   atagtgacct atatagttct aacagagcaa ctcgctgctg gtttacagct aggccaaggc    1140
   gaggcggtac tgatagggaa cttaatcacc cacacggaca atgaggtggt ggtatacttc    1200
   ttactgctct acctgataat aagagacgag cccataaaga aatggattct tcttctcttt    1260
   cattag                                                               1266
<210> 7
   <211> 20
   <212> DNA
   <213> Unknown
<220>
   <223> Arabidopsis thaliana
<220>
   <223> Synthetic Oligonucleotide
<400> 7
   atcttccatg tcgcaactcc 20
<210> 8
   <211> 20
   <212> DNA
   <213> Unknown
<220>
   <223> Arabidopsis thaliana
<220>
   <223> Synthetic Oligonucleotide
<400> 8
   caaatgtagc gaccagaagc 20
<210> 9
   <211> 28
   <212> DNA
   <213> Unknown
<220>
   <223> Pestivirus genus of the Falviviridae
<220>
   <223> DNA Sequences of HCV1.25
<400> 9
   gtaggatcca tgcggcttgc ctgtaagg 28
<210> 10
   <211> 40
   <212> DNA
   <213> Unknown
<220>
   <223> Pestivirus genus of the Falviviridae
<220>
   <223> DNA Sequences of HCV
<400> 10
   aaaaccacac cacctacatt gtttaacttg tccacccatg 40
<210> 11
   <211> 39
   <212> DNA
   <213> Unknown
<220>
   <223> Pestivirus genus of the Falviviridae
<220>
   <223> DNA Sequences of HCV
<400> 11
   ataggatcca tgaaagccct attggcatgg gcagtgatg 39
<210> 12
   <211> 39
   <212> DNA
   <213> Unknown
<220>
   <223> Pestivirus genus of the Falviviridae
<220>
   <223> DNA Sequences of HCV
<400> 12
   cccgggaatt ccatgaaaca gcagtagtat ccatttctt 39
<210> 13
   <211> 36
   <212> DNA
   <213> Unknown
<220>
   <223> Pestivirus genus of the Falviviridae
<220>
   <223> DNA Sequences of HCV
<400> 13
   attggatcca tgcggctagc ctgcaaggaa gattac 36
<210> 14
   <211> 22
   <212> DNA
   <213> Unknown
<220>
   <223> Pestivirus genus of the Falviviridae
<220>
   <223> DNA Sequences of HCV
<400> 14
   gacagcatca ttacacaaga gg 22
<210> 15
   <211> 48
   <212> DNA
   <213> Unknown
<220>
   <223> Pestivirus genus of the Falviviridae
<220>
   <223> DNA Sequences of HCV
<400> 15
   gtacttacct ttaaccactg gggttgtgtc aaaaggacaa agtccaaa 48
<210> 16
   <211> 33
   <212> DNA
   <213> Unknown
<220>
   <223> Pestivirus genus of the Falviviridae
<220>
   <223> DNA Sequences of HCV
<400> 16
   tctttcggtc tacacggcat tggaccaagt ctt 33
<210> 17
   <211> 18
   <212> DNA
   <213> Unknown
<220>
   <223> Genus Anterivirus of Arteriviridae
<400> 17
   gtatgaactt gcaggatg 18
<210> 18
   <211> 20
   <212> DNA
   <213> Unknown
<220>
   <223> Genus Anterivirus of Arteriviridae
<400> 18
   gccgacaata ccatgtgctg 20
<210> 19
   <211> 18
   <212> DNA
   <213> Unknown
<220>
   <223> Genus Anterivirus of Arteriviridae
<400> 19
   ggcgcagtga ctaagaga 18
<210> 20
   <211> 21
   <212> DNA
   <213> Unknown
<220>
   <223> Genus Anterivirus of Arteriviridae
<400> 20
   gtaactgaac accatatgct g 21
<210> 21
   <211> 18
   <212> DNA
   <213> Unknown
<220>
   <223> Genus Anterivirus of Arteriviridae
<400> 21
   cctcctgtat gaacttgc 18
<210> 22
   <211> 20
   <212> DNA
   <213> Unknown
<220>
   <223> Genus Anterivirus of Arteriviridae
<400> 22
   aggtcctcga acttgagctg 20
<210> 23
   <211> 89
   <212> DNA
   <213> Unknown
<220>
   <223> Genus Anterivirus of Arteriviridae
<400> 23
   ggcctgtcgt ccggcgaccc gatcacctct gtgtctaaca ccatttacag tttggtgatc 60
   tacgcacagc atatggtgtt cagttacat 89
<210> 24
   <211> 88
   <212> DNA
   <213> Unknown
<220>
   <223> Genus Anterivirus of Arteriviridae
<400> 24
   ggcctgtcgt ctggcgaccc gatcacctct gtgtctaaca ccatttatag tttggtgata 60
   tatgcacagc atatggtgtt cagttaca 88
<210> 25
   <211> 89
   <212> DNA
   <213> Unknown
<220>
   <223> Genus Anterivirus of Arteriviridae
<400> 25
   ggcctgtcgt ccggcgaccc gatcagctct gtgtctaaca ccatttacag tttggtgatc 60
   tacgcacagc atatcctctt cagttacat 89
<210> 26
   <211> 88
   <212> DNA
   <213> Unknown
<220>
   <223> Genus Anterivirus of Arteriviridae
<400> 26
   gttttcataa aaaaaggtta gaaaataaaa ataaaatctc catagctggt ggttatttta 60
   aaagttagaa tgagactata agcaatgg 88
<210> 27
   <211> 89
   <212> DNA
   <213> Unknown
<220>
   <223> Genus Anterivirus of Arteriviridae
<400> 27
   ggcctgtcgt ccggcgaccc gatcacctct gtgtctaaca ccatttacag tttggtgatc 60
   tacgcacagc atatggtgtt cagttacat 89
<210> 28
   <211> 89
   <212> DNA
   <213> Unknown
<220>
   <223> Genus Anterivirus of Arteriviridae
<400> 28
   ggcctgtcgt ctggcgaccc gatcacctct gtgtctaaca ccatttacag tttggtgatc 60
   tacgcacagc atatggtgct cagttactt 89
<210> 29
   <211> 28
   <212> DNA
   <213> Unknown
<220>
   <223> Chicken Infectious Bronchitis Virus (IBV)
<220>
   <223> Coronaviridae family, genus coronavirus
<400> 29
   ccgaattcgc tatgaaaact gaacaaaa 28
<210> 30
   <211> 28
   <212> DNA
   <213> Unknown
<220>
   <223> Chicken Infectious Bronchitis Virus (IBV)
<220>
   <223> Coronaviridae family, genus coronavirus
<400> 30
   gggtcgacat ccataactaa cacaaggg 28
<210> 31
   <211> 20
   <212> DNA
   <213> Unknown
<220>
   <223> Chicken Infectious Bronchitis Virus (IBV)
<220>
   <223> Coronaviridae family, genus coronavirus
<400> 31
   tcaaagcttc anggngcgta 20
<210> 32
   <211> 22
   <212> DNA
   <213> Unknown
<220>
   <223> Chicken Infectious Bronchitis Virus (IBV)
<220>
   <223> Coronaviridae family, genus coronavirus
<400> 32
   ctcgaattcc ngtrtaytgr ca 22
<210> 33
   <211> 18
   <212> DNA
   <213> Unknown
<220>
   <223> Chicken Infectious Bronchitis Virus (IBV)
<220>
   <223> Coronaviridae family, genus coronavirus
<400> 33
   gtattctgct ttaaaaag 18
<210> 34
   <211> 18
   <212> DNA
   <213> Unknown
<220>
   <223> Chicken Infectious Bronchitis Virus (IBV)
<220>
   <223> Coronaviridae family, genus coronavirus
<400> 34
   agctcaccac tataaaca 18
<210> 35
   <211> 5
   <212> PRT
   <213> Unknown
<220>
   <223> Chicken Infectious Bronchitis Virus (IBV)
<220>
   <223> Coronaviridae family, genus coronavirus
<400> 35
   His Gly Gly Ala Tyr
   1               5
<210> 36
   <211> 14
   <212> PRT
   <213> Unknown
<220>
   <223> Chicken Infectious Bronchitis Virus (IBV)
<220>
   <223> Coronaviridae family, genus coronavirus
<400> 36
   Ser Glu Asn Asn Ala Gly Thr Ala Pro Ser Cys Thr Ala Gly
   1               5                   10
<210> 37
   <211> 32
   <212> PRT
   <213> Unknown
<220>
   <223> Chicken Infectious Bronchitis Virus (IBV)
<220>
   <223> Coronaviridae family, genus coronavirus
<400> 37
   Lys Ser Gly Ser Asn Ser Cys Pro Leu Thr Gly Leu Ile Pro Lys
   1               5                   10                  15
   Gly Gln Ile Arg Ile Ser Ala Met Arg Ser Val Asn Ser Arg Leu
                   20                  25                  30
   His Ile
<210> 38
   <211> 6
   <212> PRT
   <213> Unknown
<220>
   <223> Chicken Infectious Bronchitis Virus (IBV)
<220>
   <223> Coronaviridae family, genus coronavirus
<400> 38
   Cys Gln Tyr Asn Thr Gly
   1               5
<210> 39
   <211> 13
   <212> PRT
   <213> Unknown
<220>
   <223> Chicken Infectious Bronchitis Virus (IBV)
<220>
   <223> Coronaviridae family, genus coronavirus
<400> 39
   Thr Ile Ser Tyr Asn Ala Gly Thr Ala Cys Thr Ala Gly
   1               5                   10
<210> 40
   <211> 27
   <212> PRT
   <213> Unknown
<220>
   <223> Chicken Infectious Bronchitis Virus (IBV)
<220>
   <223> Coronaviridae family, genus coronavirus
<400> 40
   Lys Ala Gly Ser Asn Gly Leu Ile Pro Lys Gly Gln Ile Arg Ile
   1 5 10 15
   Ser Ala Met Arg Ser Val Asn Ser Arg Leu His Leu
   20 25
<210> 41
   <211> 14
   <212> PRT
   <213> Unknown
<220>
   <223> Chicken Ifectious Bronchitis Virus (IBV)
<220>
   <223> Coronaviridae family, genus coronavirus
<400> 41
   Ser Glu Asn Asn Ala Gly Thr Ala Pro Ser Cys Thr Ala Gly
   1 5 10
<210> 42
   <211> 32
   <212> PRT
   <213> Unknown
<220>
   <223> Chicken Infectious Bronchitis Virus (IBV)
<220>
   <223> Coronaviridae family, genus coronavirus
<400> 42
   Lys Ser Gly Ser Asn Ser Cys Pro Leu Thr Gly Leu Ile Pro Lys
   1 5 10 15
   Gly Gln Ile Arg Ile Ser Ala Met Arg Ser Val Asn Ser Arg Leu
   20 25 30
   His Phe
<210> 43
   <211> 13
   <212> PRT
   <213> Unknown
<220>
   <223> Chicken Infectious Bronchitis Virus (IBV)
<220>
   <223> Coronaviridae family, genus coronavirus
<400> 43
   Ser Glu Asn Asn Ala Gly Ala Pro Ser Cys Thr Ala Gly
   1 5 10
<210> 44
   <211> 31
   <212> PRT
   <213> Unknown
<220>
   <223> Chicken Infectious Bronchitis Virus (IBV)
<220>
   <223> Coronaviridae family, genus coronavirus
<400> 44
   Lys Ser Gly Ser Asn Ser Cys Pro Leu Thr Gly Leu Ile Pro Lys
   1               5                   10                  15
   Gly Gln Ile Arg Ile Arg Met Ala Ser Val Asn Ser Arg Leu Thr
                   20                  25                  30
   Ile
<210> 45
   <211> 14
   <212> PRT
   <213> Unknown
<220>
   <223> Chicken Infectious Bronchitis Virus (IBV)
<220>
   <223> Coronaviridae family, genus coronavirus
<400> 45
   Ser Glu Asn Asn Ala Gly Thr Ala Pro Ser Cys Thr Ala Gly
   1               5                   10
<210> 46
   <211> 32
   <212> PRT
   <213> Unknown
<220>
   <223> Chicken Infectious Bronchitis Virus (IBV)
<220>
   <223> Coronaviridae family, genus coronavirus
<400> 46
   Lys Ala Gly Ser Asn Ser Cys Pro Leu Thr Gly Leu Ile Pro Lys
   1               5                   10                  15
   Gly Gln Ile Arg Ile Ser Ala Met Arg Ser Val Asn Ser Arg Leu
                   20                  25                  30
   His Ile
<210> 47
   <211> 14
   <212> PRT
   <213> Unknown
<220>
   <223> Chicken Infectious Bronchitis Virus (IBV)
<220>
   <223> Coronaviridae family, genus coronavirus
<400> 47
   Ser Glu Asn Asn Ala Pro Thr Gln Tyr Ser Cys Thr Ala Gly
   1               5                   10
<210> 48
   <211> 30
   <212> PRT
   <213> Unknown
<220>
   <223> Chicken Infectious Bronchitis Virus (IBV)
<220>
   <223> Coronaviridae family, genus coronavirus
<400> 48
   Lys Ser Gly Ser Asn Cys Pro Leu Thr Gly Leu Ile Pro Lys Gly
   1               5                   10                  15
   Gln Ile Arg Ile Ser Ala Met Arg Ser Val Asn Ser Arg Leu Phe
                   20                  25                  30
<210> 49
   <211> 12
   <212> PRT
   <213> Unknown
<220>
   <223> Chicken Infectious Bronchitis Virus (IBV)
<220>
   <223> Coronaviridae family, genus coronavirus
<400> 49
   Thr Ile Ser Tyr Thr Ala Pro Ser Cys Thr Ala Gly
   1               5                   10
<210> 50
   <211> 28
   <212> PRT
   <213> Unknown
<220>
   <223> Chicken Infectious Bronchitis Virus (IBV)
<220>
   <223> Coronaviridae family, genus coronavirus
<400> 50
   Ile Ser Gly Ser Asn Ser Cys Pro Leu Thr Gly Leu Ile Pro Arg
   1               5                   10                  15
   Ile Ser Ala Met Arg Asn Val Asn Ser Arg Leu Leu Ile
                   20                  25
<210> 51
   <211> 11
   <212> PRT
   <213> Unknown
<220>
   <223> Chicken Infectious Bronchitis Virus (IBV)
<220>
   <223> Coronaviridae family, genus coronavirus
<400> 51
   Ser Glu Ile Asn Ala Gly Ser Cys Thr Ala Gly
   1               5                   10
<210> 52
   <211> 28
   <212> PRT
   <213> Unknown
<220>
   <223> Chicken Infectious Bronchitis Virus (IBV)
<220>
   <223> Coronaviridae family, genus coronavirus
<400> 52
   Lys Ala Gly Ser Asn Ser Cys Leu Ile Pro Lys Gly Gln Ile Arg
   1               5                   10                  15
   Ile Ser Ala Met Arg Ser Val Asn Ser Arg Leu His Ala
                   20                  25

## Description

This application claims the benefit of U.S. Serial No. 61/263,215, filed November 20, 2009, and U.S. Serial No. 61/224,973, filed July 13, 2009 the entire contents and disclosures of which are incorporated by reference into this application.

### FIELD OF THE INVENTION

This invention pertains to vaccines against animal viruses, bacteria, other pathogenic organisms and/or antigenic agents. This invention also concerns methods of preparing such vaccines. The invention further concerns expression of exogenous antigens in microorganisms such as bacteria, and use of such microorganisms as a vaccine. In one embodiment, the present invention provides for a method of using genetically modified *Lactococcus lactis* strains expressing the avian influenza HA gene as an oral vaccine for protection against H5N1 virus infection. The invention further pertains to methods of preparing and administering such microorganism derived vaccines.

### BACKGROUND OF THE INVENTION

Domestic animal diseases annually cause reductions of substantial proportions and drastic commercial impact. In developed countries where veterinary services and injected vaccines are more readily obtained, such diseases while critical are often ameliorated. This is especially true due to larger herd/flock sizes and excess food-producing capability in these countries. In the lesser developed countries, the lack of veterinary services and drugs for such diseases and the reduced food-producing capacity has a much more substantial impact on the human population, leading to food shortages and human health problems.

The usefulness of antibiotics to effectively control bacterial pathogens is becoming increasingly difficult, because of the increased occurrence of antibiotic-resistant pathogens. Because of this, the voluntary reduction of antibiotic additives to animal feeds is practiced by many producers. However, since prevention of infectious diseases is more cost effective than the ultimate treatment of the disease once it has occurred, increased attention is being focused on the development of vaccines.

Vaccines are administered to animals to induce their immune systems to produce antibodies against viruses, bacteria, and other pathogenic organisms. In the economically advanced countries of the world, vaccines have brought many diseases under control. In particular, many viral diseases are now prevented due to the development of immunization programs.

But many vaccines for such diseases as rabies, foot and mouth disease, etc. are still too expensive for the lesser developed countries to provide to their large herd/flock animal populations. Lack of these preventative measures for animal populations routinely worsens the human condition by creating food shortages in these countries.

Microbial pathogens infect a host by: entering through a break in the integument induced by trauma; introduced by vector transmission; or by interacting with a mucosal surface.

The majority of animal pathogens initiate disease by the last mechanism, i.e., following interaction with mucosal surfaces. Bacterial and viral pathogens that act through this mechanism first make contact with the mucosal surface where they may attach and then colonize, or be taken up by specialized absorptive cells (M cells) in the epithelium that overly Peyer's patches and other lymphoid follicles. Organisms that enter the lymphoid tissues may be readily killed within the lymphoid follicles, thereby provoking a potentially protective immunological response as antigens are delivered to immune cells within the follicles. Alternatively, pathogenic organisms capable of surviving local defense mechanisms may spread from the follicles and subsequently cause local or systemic disease (e.g. Salmonella spp.).

Most pathogens enter on or through a mucosal surface, with exception of insect-borne pathogens or pathogens entering the body through wounds. Pathogens that enter, through mucosal surfaces include, without limitation, *Actinomyces*, *Aeromonas, Bacillus, Bacteroides, Bordetella, Brucella, Campylobacter, Capnbocylophaga, Clanrdia, Clostridium*, *Corynebacterium, Eiketiella, Erysipelothriz*, *Escherichia, Fusobacterium, Hemophilus*, *Klebsiella, Legionella, Leptospira, Lisleria, Mycobacterium, Mcoplasma, Neisseria, Nocardia, Pasteurella, Proteus*, *Pseudomonas, Rickettsia, Salmonella, Selenomonas, Shigelia, Staphylococcus, Streptococcus, Treponema, Bibro, and Yersinia, pathogenic vital strains from the groups Adetiovirus, Coronavirus, Herpesvirus, Orthomyxovirus*, *Picornovirus, Poxvirus*, *Reovirus*, *Retrovirus*, *and Rotavirus, pathogen fungi from the general Aspercillus, Blastomyces, Candida, Coccoidiodes*, *Cryptococcus Histoplasma and Phycomyces*, *and pathogenic parasites in the general Eimeria, Entamoeba, and Trichomonas.*

Mammalian hosts infected by a pathogen mount an immune response in an attempt to overcome the pathogen. The immune system consists of three branches: mucosal, humoral, and cellular. Mucosal immunity results from the production of secretory (sigA) antibodies in secretions that bathe all mucosal surfaces including the respiratory tract, gastrointestinal tract, and the genitourinary tract and in secretions from all secretory glands. Secretory IgA antibodies prevent colonization of pathogens on the mucosal surfaces and are a first line of defense against colonization and invasion of a pathogen through the mucosal surfaces. The production of sIgA can be stimulated wither by local immunization of the secretory gland or tissue or by presentation of an antigen to either the gut-associated lymphoid tissue (GALT or Peyer's patches) or the bronchial-associated lymphoid tissue (BALT).

Membranous microfold cells, otherwise known as M cells, cover the surface of the GALT and BALT and may be associated with other secretory mucosal surfaces. M cells act to sample antigens from the lumenal space adjacent to the mucosal surface and transfer such antigens to antigen-presenting cells (dendritic cells and macrophages), which in turn present the antigen to T lymphocytes (in the case of T-dependent antigens), which process the antigen for presentation to committed B cells. B cells are then stimulated to proliferate, migrate, and ultimately transformed into antibody-secreting plasma cells producing IgA against the presented antigen.

When the antigen is taken up by M cells overlying the GALT and BALT, a generalized mucosal immunity results with sIgA against the antigen being produced by all secretory tissues in the body. Because most pathogens enter through mucosal surfaces and such surfaces make up the first line of defense to infection and facilitate the body's immune response, vaccines that can be orally administered represent an important route to stimulating a generalized mucosal immune response leading to local stimulation of a secretory immune response in the oral cavity and in the gastrointestinal tract.

Secretory IgA antibodies directly inhibit the adherence of microorganisms to mucosal epithelial cells and to the teeth of the host. This inhibition may be the result of agglutination of microorganisms, reduction of hydrophobicity or negative charge, and blockage of microbial adhesions. These anti-adherence effects are amplified by other factors such as secretory glycoproteins, continuous desquamation of surface epithelium and floral competition.

Clinical experience with human peroral poliovirus vaccine and several peroral or intranasal virus vaccines applied in veterinary medicine shown that sIgA plays a decisive role in the protective effect by the mucosal immune system against respiratory and enteric viral infections. The effect of sIgA appears to be that of inhibiting the entry of viruses into host cells rather than prevention of attachment.

Secretory IgA antibodies directed against specific virulence determinants of infecting organism play an important role in overall mucosal immunity. In many cases, it is possible to prevent the initial infection of mucosal surfaces by stimulating production of mucosal sIgA levels directed against relevant virulence determinants of an infecting organism. Secretory IgA may prevent the initial interaction of the pathogen with the mucosal surface by blocking attachment and/or colonization, neutralizing surface acting toxins, or preventing invasion of the host cells.

Parenterally administered inactivated whole-cell and whole-virus preparations are effective at eliciting protective serum IgG and delayed type hypersensitivity reactions against organisms that have a signifcant serum phase in their pathogenesis (e.g, human and animal pathogens such as Salmonella typhi and Hepatitis B). However, parenteral vaccines are not effective at eliciting mucosal sIgA responses and are inefective against bacteria that interact with mucosal surfaces and do not invade (e.g., human and animal pathogens such as Vibrio cholerae).

Oral immunization can be effective for induction of specific sIgA responses if the antigens are presented to the T and B lymphocytes and accessory cells contained within the Peyer's patches where preferential IgA B-cell development is initiated. The Peyer's patches contain helper T cells (TH) that mediate B-cell isotype switching directly from IgM cells to IgA B cells then migrate to the mesentric lymph nodes and undergo differentiation, enter the thoracic duct, then the general circulation, and subsequently seed all of the secretory tissues of the body, including the lamina propria of the gut and respiratory tract. IgA is then produced by the mature plasma cells, complexes with membrane-bound Secretory Component, and transported onto the mucosal surface where it is available to interact with invading pathogens. The existence of this common mucosal immune system explains in part the potential of live oral vaccines and oral immunization for protection against pathogenic organisms that initiate infection by frst interacting with mucosal surfaces.

Because of simplicity of oral delivery, there is great current interest in discovering new oral vaccine technology. Appropriately delivered oral immunogens can stimulate both humoral and cellular immunity and have the potential to provide cost-effective, safe vaccines for use in developing countries where large-scale parenteral immunization of herd or other commercially produced animals is not practical or extremely difficult to implement. Such vaccines may be based upon bacterial or viral vector systems expressing protective epitopes from diverse pathogens (multivalent vaccines) or may be based upon purified antigens delivered singularly or in combination with relevant antigens or other pathogens.

A number of strategies have been developed for oral immunization, including the use of attenuated mutants of bacteria (e.g., Salmonella spp.) as carriers of heterologous antigens, encapsulation of antigens into microspheres composed of poly-DL-lactide-glycolide (PGL), protein-like polymers-proteinoids, gelatin capsules, different formulations of liposomes, adsorption onto nanoparticles, use of lipophilic immune stimulating complexes, and addition of bacterial products with known adjuvant properties.

Underlying the development of most current vaccines is the ability to grow the disease-causing agent in large quantities. At present, vaccines are usually produced from killed or live attenuated pathogens. If the pathogen is a virus, large amounts of the virus must be grown in an animal host or cultured animal cells. If a live attenuated virus is utilized, it must be clearly proven to lack virulence while retaining the ability to establish infection and induce humoral and cellular immunity. If a killed virus is utilized, the vaccine must demonstrate the lack of capacity of surviving antigens to induce immunization. Additionally, surface antigens, the major viral particles that induce immunity, may be isolated and administered to induce immunity in lieu of utilizing live attenuated or killed viruses.

Vaccine manufacturing often employs complex technologies entailing high costs for both the development and production of the vaccine. Concentration and purification of the vaccine is required, whether it is made from cell cultures, whole bacteria, viruses, other pathogenic organisms or sub-units thereof Even after these precautions, problems can and do arise. With killed bacterial cells, viruses or other pathogenic organisms, there is always a chance that live pathogens survive and vaccination may lead to isolated cases of the disease. Moreover, the vaccines may sometimes be contaminated with cellular material from the culture material from which it was derived. These contaminates can cause adverse reactions in the vaccine recipient animal and sometimes even death.

Direct injection of plasmid DNA has been used as a vaccine strategy, with reports of protective immunity and cytotoxic T lymphocyte (CTL) induction in mice afer i.m. injection of a DNA plasmid. The use of DNA vaccines in preclinical studies has become well established, with report of protective immunity in many different independent studies. In recent studies, both antibody and CTL responses were induced in non-human primates, although 1-2 mg of DNA was immunized on multiple occasions in these studies. However, the use of very high doses of DNA is less favorable from a process economics standpoint, therefore, there is a clear need to induce effective immunity in veterinary medicine with lower and fewer doses of DNA, as well as to increase the magnitude of the immune responses obtained.

A number of strategies available that have the potential to improve the potency of DNA vaccine include vector modifcation to enhance antigen expression, improvements in DNA delivery, or the inclusion of adjuvants. The monophoryl lipid A has been reported could enhance both humoral and cell-mediated immune responses to DNA vaccination against human immunodefciency virus type 1 (Shin et al., 1997). DNA vaccine formulated with QS-21 saponin adjuvant via intramuscular and intranasal routes could also induce systemic and mucosal immune responses. (Shin S, et al., 1998). Manmohan S had developed a delivery system for DNA vaccines, the cationic microparticles. Vitamin D3 also plays an important role in the immunization of DNA vaccine.

It is also reported that bacterial DNA-sequences called imunostimulatory sequences can be a potent adjuvant. Non-methylated, palindrome DNA-sequences containing CpG-oligodinucleotides (CpG-ODN) can activate an' innate ' immune response by activating monocytes, NK cells, dendrilic cells and B-cells in an antigen-independent manner (immunostimulatory DNA sequences, ISS). Methylation of the CpG-ODN reportedly abrogates the immunogenicity of the DNA vaccine. The use of large amounts ofplasmid for immunization might only overcome the low transfection effciency in vivo, as well as serve as an adjuvant, driving a Th1-type response.

The propagation of highly pathogenic avian influenza (HPAI) H5N1 virus remains a major concern globally. In addition to the many outbreaks reported annually in various bird populations all over the world, more than 385 human cases of H5N1 infection have also been reported [1]. Therefore it is of prominent importance to develop vaccines to help contain viral contagion in animals and deter further development into a major threat and health crisis for human beings.

There have been many approaches taken to make influenza viral vaccines. The most commonly used strategy is to administer heat inactivated whole viruses grown in embryonated eggs. But the production of egg-derived vaccines against the deadly H5N1 viruses has not proven very effective; moreover, a protective immune response has only been elicited upon the administration of large doses of inactivated whole viruses produced in this fashion. Seroconversion rates and the magnitude of immune response were suboptimum after administration of egg-derived vaccines [2-4]. Other more advanced approaches have included using recombinant subunit vaccines produced in a baculovirus expressing system [5], plasmid DNA vaccines [6-8], and replication-incompetent adenovirus vector vaccines [9-12]. Each showed promise in protecting mice against lethal viral challenges [6-7]. However, all such vaccines were designed to be administered intramuscularly, presenting practical difficulties in respect of administration to large populations of animals.

Therefore a practical and effective influenza vaccine that can be easily applied to humans and animals is more highly desirable if the vaccine can be administered with food. Towards this end, the present invention uses the *Lactococcus lactis* (*L. lactis*) vector system which can be safely administered orally. *Lactococcus lactis* is a Gram-positive lactic acid bacterium that is widely used for the production and preservation of fermented milk products, which is generally regarded as safe (GRAS). It can be engineered to express various proteins, including bacterial and viral antigens [13-17]. Mice given these vectors generated antigen specific mucosal as well as systemic immune responses [13, 14, 17]. However, antigen inoculation efficiency was still low because most of the organisms cannot survive the harsh acidic environment of the stomach and protease degradation in the GI track [20].

### Animal Diseases

Hog cholera (HC), also known as classical swine fever, is a severe systemic and hemorrhagic disease in swine caused by Hog Choler Virus (HCV). Classical swine fever or hog cholera represents an economically important disease of swine in many countries worldwide. Under natural conditions, the pig is the only animal known to be susceptible to HC. Hog cholera is a highly contagious disease that causes degeneration in the walls of capillaries, resulting in hemorrhages and necrosis of the internal organs, In the first instance hog cholera is characterized by fever, anorexia, vomiting and diarrhea which can be followed by a chronic course of the disease characterized by infertility, abortion and weak off-springs of sows. However, nearly all pigs die within 2 weeks after the first symptoms appear.

HC can be transmitted from the infected swine to the healthy one by direct contact. The disease can also be transmitted through contact with body secretions and excrement from infected animals. Flies, birds and human can act as vectors in transmitting the virus.

While hog cholera does not cause food-borne illness in people, it causes serious economic losses to the pig industry since it can result in widespread deaths in pigs. Traditionally, HC syndrome is an acute disease of high morbidity and mortality. From time to time, evolution of the virus has led to a higher incidence of subacute and chronic forms.

Virulent strains induce an acute disease that is characterized by persistent fevers that can raise body temperatures as high as 107°F. Other signs of the acute form include convulsions, anorexia, leukopenia, tonsillar necrosis and lack of appetite. At day 3 - 4 of post-infection, there is a generalized viremia with the virus replicating in epithelial cells, endothelial cells and cells of the mononuclear phagocyte system. Degeneration and necrosis of endothelial cells leads to vascular compromise, ischemia, and the induction of disseminated intravascular coagulation. These vascular changes result in petechial hemorrhage of the kidneys, urinary bladder and gastric mucosa, splenic infarction and lymph node hemorrhage. Death usually occurs within 5 to 14 days following the onset on illness.

The chronic form of hog cholera causes similar clinical signs in affected swine, but there is less hemorrhage associated, Discoloration of the abdominal skin and red splotches around the ears and extremities often occur. Pigs with chronic hog cholera can live for more than 100 days after the onset of the infection.

The mild or clinically unapparent form of hog cholera seldom results in noticeable clinical signs. Affected pigs suffer short periods of illness often followed by periods of recovery. The mild strain may cause small litter size, stillbirths and other reproductive failure. High morality during weaning may also indicate the presence of this mid strain of hog cholera,

When pregnant sows are infected with strains of lesser virulence, transplacental infection may occur. Depending on the stage of gestation, congenital infection can result in abortion, fetal mummification, stillborn and embryonic malformations. The most frequent outcome with low virulent strains is the birth of persistently infected piglets in a state of immunological tolerance and shed large quantities of virus.

Hog Cholera Virus (HCV) is a member of the Peslivirus genus of the Flaviviridae family (Francki, R.I.B. et al., 1991, Arch of Virol Supp 2:223-233; Horzinek, M., 1991, Arch of Virol Supp 3:1-5; Collett, M.S., 1992, Comparative Immunology, Microbiology and Infectious Diseases 15: 145-154). It replicates principally in lymphocytes and vascular endothelium. Replication of most of the HC strains is restricted to the cytoplasm of the cell and does not result in cytopathic effect.

Hog cholera virus has been shown to be structurally and serologically related to bovine viral diarrhea virus (BVDV) of cattle and to border disease virus (BDV) of sheep, which also belongs to the genus pestivirus within the family togaviridae. HCV is a small single positive-stranded RNA virus with a genome of approximately 12.3kb (Vanderhallen, H., et al., 1999, Arch Vii al 144: 1669-1677). It genome contains a single long open reading frame (ORF) that is flanked by a 5' and 3' nontranslated region (NTR). Meanwhile, it lacks both 5' cap and a significant 3' poly(A) sequences, if it possesses any polyadenylation. The HCV is believed to encode 3-5 structural proteins of which two are possibly glycosylated. The number of non-structural viral proteins is not known. Modified HCV vaccines (comprising attenuated or killed viruses) for combating hog cholera infection have been developed and are presently used. However, infection of tissue culture cells to obtain HCV material to be used in modified virus vaccines leads to low virus yields and the virions are very difficult to purify. Modified live virus vaccines always involve the risk of inoculating animals with partially attenuated pathogenic HCV which is still pathogenic and can cause disease in the inoculated animal or offspring and of contamination by other viruses in the vaccine. In addition the attenuated virus may revert to a virulent state. There are also several disadvantages using inactivated vaccines, e.g., the risk of only partial inactivation of viruses, the problem that only a low level of immunity is achieved requiring additional immunizations and the problem that antigenic determinants are altered by the inactivation treatment leaving the inactivated virus less immunogenic. The usage of modified HCV vaccines is not suited for eradication programs.

Vaccines containing only the necessary and relevant HCV immunogenic material that is capable of eliciting an immune response against the pathogen do not have the disadvantages of modified vaccines. Classically derived and administered recombinant HCV vaccines have been disclosed that contain only certain immunogenic portions of HCV. See, e.g., US Patent Nos. 5,935,582 (issued August 10, 1999), 5,925,360 (issued July 20, 1999), and 5,811,103 (issued September 22, 1998) to Meyers et al.

The cDNA sequence derived from the genomic RNA of HCV is a continuous sequence about 12,500 nucleotides in length. It contains one long open reading frame (ORF), starting with the ATG codon at position 364 to 366 and ending with a TGA codon as a translational stop codon at position 12058 to 12060. This ORF consists of 3898 codons capable of encoding 435 kDa of protein.

In vivo, during HCV replication in an infected cell, this protein is synthesized as a polyprotein precursor molecule that is subsequently processed to fragment polypeptides by (enzymatic) cleavage of the precursor molecule. These fragments form after possible post-translational modifcations the structural and non-structural proteins of the virus. It is possible to derive a sequence that contains the genetic information for such a fragment with immunizing properties against HCV or immunological properties characteristic for HCV or contains the genetic information for a portion of such a fragment that still has the immunizing properties or the immunological properties characteristic for HCV.

Fragment polypeptides are located within the amino acid position about 1-249, 263-487, 488-688 or 689-1067. The 1-249 region essentially represents the core protein whereas the 263-487, 488-688 and 689-1067 regions essentially represent glycoproteins of 44/48 kD, 33 IcD and 55 kD respectively.

HCV is 40-50 nm in diameter. It has a nueleocapsid of about 29 nm. There are fringelike projections of 6-8 nm on the surface of the virion. The buoyant density, depending on the gradient material and on the cells used to propagate the virus, has been reported between 1.12 g/ml and 1.17 g/ml.

HCV is stable at pH 5-10; but above and below these pH values, infectivity is rapidly destroyed. HCV is quickly made inactivate by lipid solvents, such as ether, chloroform and deoxycholate. Although its infectivity is lost in cell culture medium at 60°C after 10 minutes, the virus is still active in defibranted blood at 68°C after 30 minutes. Moreover, the virus can survives in frozen carcasses for long periods of time and it can remain infective in pork and pork product for months, so it is of great epizootiologic importance.

All HC strains discovered so far were clustered into two main groups and fve subgroups when genomic elements of the 5' nontranslated region (Hofmann, M.A. et at, 1994, Arch Viral 139: 217-229), E2 gene (Lowings, J.P. et at, 1994, J. Gen Viral 75: 3461-3468; Lowings, P. el al., 1996, J Gen Vral 77:1311-1321) or the NS5B gene (Vilcek, S. et at, 1996, Virus Res 43: 137-147) were compared.

The 5' to 3' genomic organization of HCV includes a nonstructural protein designated N, an encoded nucleocapsid protein designated C, a structural envelope associated glycoproteins (E) designated EO, E1 and E2, nonstructural proteins (NS), designated NS2, N83, NS4A, NS4B, NSSA and NSSB.

The resulting polyprotein of about 3900 amino acids is co-and post translationally processed by viral as well as host cellular proteases to yield four structural and seven to eight nonstructural viral proteins (Thiel, H-J. et at, 1996, Fundamental Virology, 3rd ed. Raven Press New York, 1059-1079).

Nucleocapsid protein C and the three envelope-associated glycoprotein EO (gp44/48), E1 (gp33) and E2 (gp55) are the structural components of HCV. They are located within the N-terminal third of the polyprotein (Stark, It, et al., 1990, Viral 174: 286-289). The pestiviral capsid protein is preceded by a nonstructural protein, p23, in the polyprotein.

This non-structural core protein is a putative protease exhibits autoproteolytic activity (Thiel et at, 1991, J. Virol 65: 4705-4712; Wiskerchen, M.A., et at, 1991, J Vrol 65: 4508-4514).

EO lacks a typical membrane anchor and is secreted in considerable amounts from the infected cells (Rumenapf, T. et al" 1993, J Vrol, 67:3288-3294). Although this protein exhibits RNase activity, its enzymatic action for the viral lifecycle is still unknown.

E2 and to a lesser extent, EO were found to be the targets for triggering neutralizing antibodies against the virus. On the other hand, E1 is believed to be buried in the viral envelope (Weiland, E. et al., 1990, J Virol 64: 3563-3569), and hardly any anti-El antibodies have been described. In the virions and in the infected cells, the glycoproteins form disulfde-linked complexes, such as, EO homodimer with a size of 100 kDa, E1-E2 heterodimer with a size of 75 kDa, and E2 homodimer with a size of 100 kDa (Thiel, H-J, et al., 1991, J Vrol 65:4705-4712).

Infectious bronchitis (IB) is an acute, highly contagious viral respiratory disease of chickens characterized by tracheal rates, coughing and sneezing. The poultry industry in Southern China experienced severe outbreak of IB every year, particular in recent years, other viral respiratory disease also had a high incidence in pearl river delta region of China, and consequently, accurate and rapid genotyping is an important factor in controlling infectious bronchitis. The causative agent of IB is infectious bronchitis virus (IBV)5 which is classifed in the coronaviridae family, genus coronaviras, with more than 20 serotypes identifed in the world. Although vaccination with Mass-typed vaccine is widely used in China, outbreaks were still reported each year, IBV usually damage respiratory tract, but strains of IBV replicate in the kidney, oviduct, intestine and glandular also had been reported in China.

The IBV encodes three major structural proteins: the nucleoeapsid protein (N), the membrane glycoprotein and S protein. The S protein can be cleaved post-translationally to release the N-terminal S-1 and C-terminal S-2 protein. The N-terminal subunit (S-I) is responsible for cell attachment, determine tissue tropism and virus-neutralizing antibody induction, whereas the C-terminal subunit (S-2) anchors S-I to the viral envelop. The N-terminal part of the S-1 protein is variable between different serotypes and between different strains of the same serotype. Keeler and Kingham had identifed two hypervariable regions (HVRs) and two conserved regions in the N-terminal part of the S-I gene in Mass sera-typed IBV. The HVRs that contain neutralization epitopes may be located in amino acid regions 56-69 and 117-137. The two conserved regions are located at 43-47 and 229-236. Previously HI and VN is often adopted in the diagnosis of IBV, but these methods had many drawbacks, such as time and labor consuming, and furthermore HI is not so reliable.

Since Jungher et al, first described antigenic differences between the Mass and Conn, the extensive antigenic diversity of IBV is well recognized. Due to immune selective pressures associated with intensive TBV vaccination and other poor bio-security practice, over 20 serotypes of the virus had been reported, and additional variant serotypes continue to emerge and cause disease.

Due to farm practice, mass-typed vaccine is widely used in China, In recent years, intensive farming of poultry industry in Southern China increased the immune selective pressure of vaccination, at the same time, other source of IBV vaccine are also brought into China due to open door policy. The recombination between field strain and vaccine strain will also cause outbreak of IBV.

Another commercially expensive disease is Infectious Bursal Disease (IBD), also known as Gumboro disease. IBD is. caused by Infectious Bursal Disease Virus (IBDV). Apart from direct contact with infected birds, it is possible that rats and mosquitoes can transmit the disease.

During the 63th General Session of the Ofce International des Epizooties (OlE. Paris, 15 to 19 May 1995), it was estimated that IBD has considerable socio-economic importance at the international level, as the disease is present in the more than 95% of the Member Countries (Eterradossi, N. el al., 1995; Paris OIE.).

Although IBDV does no infect human, it can cause severe economic loss. The economic importance of the disease can be categorized into two main aspects. First, some virus strains may cause up to 20% mortality in chicken three weeks of age or older. Second, prolonged immunosupression of chickens infect at early age.

Chickens from 3 and 6 weeks of age are most susceptible to IBDV infection and mortality may be high. The syndrome of IBD can be clinical or subclinical. The age susceptibility is broader in the case of very virulent IBDV strains (Van den Berg TP, at at., 1991. Avian Patho! 20:133-143; Nunoya T. et al" 1992. Avian Drs. 36:597-609). After a short period of time (within 24 hours after infection), bursa of Fabricius, shows lesions. There is gelatinous yellowish transudate covering the semsal surface of the bursa.

At day 3 of post-infection, due to edema and hyperemia, bursa increases in size and weight. At day 4 of post-infection, the size of the bursa has usually doubled. Later, the bursa recede in size and the transudate disappears in the subsequent days. At day 8 of post-infection, the bursa usually becomes one-third of the original weight. There are also necrotic foci and petcchial hemorrhages on the mucosal surface,

Due to impairment of clotting mechanism, pectoral muscle of the infected subject becomes dehydrated with darken dislocation. Lesions and hemorrhage of other major organs, for instance, kidney, spleen, thymus and harderian gland can also be observed.

Instead of exhibiting clinical signs, prolonged immunosuppression arises in chicken at early age (usually below three weeks of age) after catching IBD. As a result, the infected subject is more susceptible to other diseases. This predisposes the birds to other diseases such as colisepticaemia coccidiosis, infectious laryngotracheitis, infectious bronchitis and salmonellosis and colibacillosis. Moreover, lower antibody response to vaccination to other pathogen would be resulted. In addition to the dominant suppression of the humoral immunity of infected chicken, the cell-mediated immunity was also suppressed transiently.

IBDV is a member of the genus Birnaviurs of the Birnaviridae family. It primarily infects lymphoid cells, especially precursor B cells. The primary target organ of the virus, the bursa of Fabricus is the most severely affected.

IBDV is a single shelled, non-enveloped virion with icosahedral symmetry composed of 32 capsomers and it is 60 nm - 70 nm in diameter. The capsid symmetry is askew. Buoyant density of complete IBDV particle in cesium chloride gradient range from 1.31 g/ml to 1.34 g/ml.

1BDV resists treatment with ether and chloroform. It would be unaffected by pH 2. It is still viable exposing at 56°C for five hours. Moreover, the virus is unaffected by exposure to 0.5% phenol and 0.125% thimerosal at 30°C for one hour. On the other hand, it would be inactivated at pH 12. Its infectivity would be reduced considerable when exposed to 0.5% formalin for six hours. It would even be killed when incubating at 70°C for 30 minutes.

Two serotypes of IBDV wore recognized and there were several strains within each serotype. Although its presence will stimulate antibodies, type I1 virus does not cause clinical disease. Hence, only IBD vaccines have been made from type I IBDV nowadays. Type II antibodies do not confer protection against type I infection, neither do they interfere with the response to type I vaccine.

The high mutation rate of the RNA polymerase of IBDV leads to antigenic variation (that are, variant strains) and modification in virulence in vivo (for example, the very virulent strain). These may require special vaccines for maximum protection. Cross protection studies have shown that inactivated vaccines prepared from "classical" type I virus require a high antigenic content to provide good protection against some of these variants,

Moreover, very virulent strains of IBDV have also emerged and caused serious disease in many countries over the past decade.

IBDV consists of two segments, designated segment A and segment B, of double stranded RNA shown by polyacrylamide gel electrophoresis. The larger segment A is approximately 3.4 kb containing two open reading frames (ORF). The larger ORF is monocistronic and encodes a polyprotein which would later be auto-processed into structural protein VP2 (40 kDa-45 kDa), VP3 (30 kDa - 32 kDa) and protease VP4 (28 kDa) of IBDV (Muller & Becht, 1982 J. Virol. 1982 Ocr44(1):384-392; Azad, A.A. et at., 1985, Viral 143: 35-44; Azad A.A. at al., 1987, Virol 161: 145-152; Hudson, P.J., et al, 1986, Nucleic Acids Res 14: 5001-5012; Kibenge FS, et al., 1997. Arch Viral 142:2401-2419). Meanwhile, the shorter, partially VP2 overlapping ORF encodes VP5 protein (17 kDa). The smaller segment B (approximately 218 kb) encodes the 90 kDa functional protein VP1 (Muller H, Nitschke R. 1987. Virology 159:174-177; Speis et al., 1987, Virus Res 8: 127-140). The genome of IBDV contains two restriction sites of VP2, namely Accl and Spel. Overlapping occurs between VP5 and VP2. VPI functions as both RNA-dependent RNA polymerase and capping enzyme for in vivo replication of the virus. It presents as a free polypeptide and as a genome-linked protein (Muller H, Nitschke R. 1987, Virology 159:174-177; Kibenge FS, Dhama V. 1997. Arch Virol 142:1227-1236).

Sequences of VP2 in various strains are highly conserved, except the central Accl-SpeI restriction fragment which is designated as the hypervariable region (Bayliss et al., 1990, J. Gen Viral 71, 1303-1312). Sequence that is important for the virulence and attenuation of the virus were also identifed in this region (Yamaguchi T, et al., 1996. Virology 223:219-223), VP2 is the major host-protective inununogen of IBDV that contains the antigenic sites responsible for the induction of neutralizing antibodies (Azad et al., 1987, Vral 161: 145-152) while VP3 protein is recognized by non-neutralizing antibodies.

VP2 and VP3 form the capsid of virus. VP2 is likely to be exposed on the outer surface of the capsid while VP3 is laid inside, interacting with the viral RNA.

VP4 protease is a non-structural polypeptide, It is responsible for cleavage of the polyprotein on segment A, but it is not included in the mature virion. The presence of serine-lysine catalytic dyade accounts for its proteolytic activity (Birghan et at, 2000, EMBO Journal 4: 114-123).

VP5 is not essential for viral replication in cell culture but it has a regulatory function and could play a key role in virs release and dissemination (Mundt et al., 1997, J Viral 71:5647-51). Another disease that affects livestock is porcine reproductive and respiratory syndrome (PRRS) is caused by porcine reproductive and respiratory syndrome virus (PRRSV). Porcine Reproductive and Respiratory Syndrome (PARS) is considered to be the most economically important viral disease of intensive swine farms in Europe and North America. The syndrome frst began causing swine herd problems in the late 1980's in Unites States and prior to isolation of the causative agent, was often referred to as mystery swine disease. But in the time since the virus was identified in Europe (Leiystad Virus [LV]) in 1991 (Wensvoort G., et at, (1991). Veterinary Quarterly 13, 121-130) and in United States (VR-2332) in 1992 (Bonfield A.A., et at, (1992). J Veterinary Diagnostic Investment 4, 127-133), PRRSV has become a significant pathogen of swine herds worldwide, with new disease phenotypes continuing to emerge.

PRRS can result in losses in neonates and nursery from respiratory disease and reproductive losses in breeding stock. As a consequence, it causes dramatic financial consequences in swine industry. However, the inherent variability in clinical signs translates into highly variable economic losses. On a herd basis, most acute outbreaks are estimated to decrease annual production 5%-20%.

Due to the difference in prevailing health status of swine, virus strain and management strategies of different farm, clinical signs and production losses vary widely among heard. Moreover, many cases are often complicated with secondary infections that increase severity of this disease (De Jong, M.F. et at, 1991, European Comm Seminar on the New Pig Disease, 4:29-30 #4; Bonfeld, D.W. et al., 1992, J VetDiagn Invest. 4:127-133).

There are three phases in the acute form of PRRSV infection: the initial, climax and final phase (Raymakers, J.M.L., 1991, European Comm Seminar on PRRS 11:4-5, Brussels, #16). In the initial phase of the disease, clinical signs such as inappetance, lethargy, depression and pyrexia can be seen in the breeding/gestating, farrowing or grow/finish area of pig farm. During this phase, respiratory disorders, such as dyspnea and polypnea, may or may not be observed in adult pigs but these syndromes are usually prominent in younger animals. It typically lasts 1 - 3 weeks.

During the climax phase, premature farrowings, increased stillborn, mummifed, and weakbom pigs, and an increase in preweaning mortality can be observed. In the same time, most of the growth reduction and mortality is due to secondary infection. Increase secondary infections occur in growing pigs, especially in the nursery (De Jong, M.F. et al, 1991, European Comm Seminar on the New Pig Disease 4:29-30 #4; Benfeld, D.A., et al., 1992, Diseases of Swine 7i6 Ed, Ames, IA: Iowa State University Press: 756-762). It typically lasts 8 -12 weeks.

In the final phase, reproductive parameters return to near-normal pre-PRRS levels and there are variable respiratory diseases in nursery or grow-finish pigs. This phase may either be the prelude to chronic disease or to a return to normal pre-PRRS production level.

During chronic PRRS, a long term reduction in the number of pig bor alive can be seen (Dial, D., et at, 1990, MSD Corn Mtg. Denver: Livestock Conservation Institute, 3.6) and an extended duration of reduced farrowing rates has also been observed (Benfeld, D.A., at at, 1992, Diseases of Swine 7th Ed. Ames, IA: Iowa State University Press: 756-762; Benfield, D.W., et at, 1992, J Vet Diagn Invest. 4: 127-133). Pigs in chronic PRRS herds have higher feed gain ratios in addition to slower growth. Meanwhile, the increased numbers of secondary infections are probably responsible for the continued rhinitis and pneumonia observed in chronically affected herds.

Although the mechanism of reproductive failure is still unknown, transplacental infection of porcine fetuses is common in late gestation. Moreover, semen can transmit PRRSV though no virus can be isolated from the testicles or accessory sex glands of mature board (Oblinger, V., 1992, Pig Dis Info Centre).

Porcine reproductive and respiratory syndrome virus (PRRSV) is a member of the genus Anterivirus of Arteriviridae family (Cavanagh, D. 1997, Arch of Virol. 142: 629-633). Alveolar macrophages are the target cells of PRRSV in vivo.

PRRSV is a spherical, enveloped virus 45 run-70 nm in size (Benfeld, D.A., et at, 1992, Diseases of Swine 7'h Ed. Ames, IA: Iowa State University Press: 756-762; Benfeld, D.W., et at, 1992, J Vet Diagn Invest. 4: 127-133) and it contains a icosahedral nucelocapsid core of 20 rim-30 nm. The lipid bilayer that surrounds the nucleocapsid contains two major envelope components, GP5 and M, and two minor envelope components, GP2 and ON. Small surface projections are also apparent. The PRRSV has a buoyant density of 1.18 g/ml - 1.19 g/ml in CsCI and 1.13 g/ml - 1.14 g/ml in sucrose. Peak infectivities are greater in CsCI purifed preparations than in sucrose preparations (Benfeld, D.A., et al:, 1992, Diseases of Swine 7'h Ed. Ames, IA: Iowa State University Press: 756-762; Benfeld, D.W., et al., 1992, J Yet Diagn Invest. 4: 127-133).

Although the infectivity titer of the virus is stable for more than four months at-70°C, it is reduced 10 times when maintains at 56°C for 15 - 20 minutes or at 37°C for 10-24 hours (Bonfield, D.A., et at, 1992, Diseases of Swine 74 Ed. Ames, IA: Iowa State University Press: 756-762; Benfield, D.W., et at, 1992, J Vet Diagn Invest. 4: 127-133). Virus infectivity titers are reduced over 90% at pH levels less than 5 or greater than 7. In addition, virus replication is inactivated after treatment with chloroform or ether. PRRSV is made up of a polyadenylated, single-stranded positive sense RNA molecule of 15.1 kb (Meulenberg, J.7,M, at al., 1993, Viral 192: 62-72), which consists of eight open reading frames (ORFs), designated Is, Ib, 2, 3, 4, 5, 6 & 7 (Conzelmann, K.K. et al., 1993, Viral 193: 329-339; Meulenberg, et al., 1995. Virology 206:155-163), In PRRSV-infected cells, the ORFs of the virus are transcribed into 3' nested set of sex messenger RNAs. All six mRNAs have 3' polyA tail and a common leader sequence obtained from the 5' end of the genomic RNA.

ORP 1a and 1 b comprise 75% of the genome at the 5' end and code for protein with apparent replicase and RNA polymerase activities. ORFs 2 to 6 encode viral membrane-associated proteins. In addition, polypeptides encoded by ORFs 2-5 are glycosylated and have been designated GP2 (29kD), GP3 (431cD), GP4 (31 kD) and GPS (25kD).

GP2 is one of the minor components in the viral envelope. A portion of it is folded on itself via disulfide bonds, without forming homodimers, or heter-multimers with other viral protein (Mculenberg, J.J.M., et al., 1996, Viral 225: 44-51).

GP3 can provide protection for piglets against PRRSV infection in the absence of a noticeable neutralizing humoral response, as demonstrated in the North American and European strains. There are highly hydrophobic sequences at the Nand C-terminal regions of GP4. Although anti-GP5 antibodies can neutralize PRRSV infection, it is less effective than anti-GP5 antibodies (Weiland, E., at al., 1999, Vet Microbiol 66: 171-186).

GP5 that incorporated into the viral envelope contains N-linked oligosaccharides of the high mannose and complex type. GP5 is important for the infectivity of PRRSV as specific anti-GP5 antibodies can neutralize PRRSV infection of susceptible cells (Pirzadeh, B. et at, 1997, Viral, 78:1867-1873).

The polypeptide, designated as M protein, encoded by ORF6 (19 kD) is a non-glycosylated type III transmembrane protein. It forms disulfide-linked heterodimers with the GPS glycoprotein. In PRRSV infected cells, disulfide-linked M protein homodimer have also been observed but these were not incorporated into the virions (Mardassi, H. et at, 1996, Vrol221:98-112).

ORF 7 encodes a non-glycosylated polypeptide (151cD) that forms the nucleocapsid, designated N. N is a highly basic protein predominantly present as disufide-linked homodimer (Mardassi, H. et at, 1996, Viral 221: 98-112; Meulenberg , et at, 1995. Virology 206:155-163).

### Vaccines against HCV, IBDV and PRRSV

The vast economic importance of HC initiated various efforts to extinguish the disease. The live attenuated vaccine strains, for example, the Chinese strain, which is the most extensively used vaccine, can effectively protect pig against the disease.

Although vaccination with attenuated live virus is safe and effective, it interferes with scrodiagnosis and does not discriminate between vaccinated and infected animals. Therefore, current control to fight against large outbreaks of HC in Europe is based largely on quarantine restriction and slaughter policy. In order to avoid trade restrictions, the slaughter policy is mostly based on the destruction of infected and serologically positive (suspected) animals. The enormous cost of eradication programs stimulated the search for alternate methods to control the disease.

Due to the robustness of IBDV, hygienic measures alone are insufficient to control the disease. Hence, the currently favored practice to control IBD is to vaccinate the parent birds with an oil-emulsion vaccine just before laying in order to induce a high level of passive immunity in the offspring, which would protect them, The progeny are then vaccinated with a killed oil emulsion vaccine at seven days old to give a protection rate of around 85% to 90% (Wyeth PJ, Chettle NJ. 1990. Yet Rec 126:577-578). This way be followed by live vaccine in the drinking water at around 2.5, 3.5 and 4.5 weeks of age although researchers have shown that this offers no extra protection (Wyeth & Chettle, 1990 Yet Rec. Jun 9; 126(23):577-578; Goddard, et at, 1994 Pet Rec. Sep 17;135(12):273-274).

However, injected vaccine will be neutralized in the presence of high level of maternal antibodies if administered too early, Hence, serological monitoring is usually necessary to determine the optimal timing for vaccination (Van den Berg TP, et aL, 1991. Avian Patho120:133-143).

With the occurrence of variant strains (with different antigenic properties) and very virulent strains (can breakthrough even high levels of maternal antibodies), classical IBDV vaccine becomes ineffective in defeating IBD.

Current strategies for the control of PRRSV depend largely on immunization with modified-live vaccines that have inherent drawbacks. Firstly, the live vaccine has the intrinsic risk of reversion to a virulent phenotype. Secondly, it is not possible to discriminate between vaccinated and infected animals in a herd. It has been reported that a DNA vaccine against PRRSV trigger both cellular mediated immunity and humoral immunity (Kwang, I., et al., 1999, Res in Vet Sci, 67:199-201).

However, despite these advances, there still exists a need for effective and easy to produce and administer vaccines for HCV, IBDV and PRRSV and other animal diseases.

### SUMMARY OF THE INVENTION

In order to provide a clear and consistent understanding of the present invention, the following list of terms and their definitions are provided.

An animal is defined as any vertebrate or invertebrate, including, but not limited to humans, birds and fish.

An antigen is defined as a macromolecule that is capable of stimulating the production of antibodies upon introduction into a mammal or other animal including humans. As used in this application, antigen means an antigen per Sc, an antgenic determinant or the antigen, or a fusion protein containing the antigen or antigenic determinant sometimes referred to a native epitopes.

An antigenic determinant is defined as a small chemical complex that determines the specificity of an antigen-antibody reaction. Colonization and/or virulence antigens of a pathogen contain one or more antigenic determinants.

An amino acid domain is defined as an amino acid sequence within a protein that can be associated with a particular function or sequence homology.

A colonization or virulence antigen is defined as an antigen on the surface of a pathogenic microorganism that is associated with the ability of the microorganism to colonize or invade its host. Discussion and claims may refer to colonization or virulence antigens or antigenic determinants thereof. A pathogen may contain antigens of either colonization or vimlence or both and one or more DNA sequences for each or both may be transferred to a vector and used to transform a plant such that it expresses the antigen or antigens.

An immunogenic agent is defined as any antigen that is capable of causing an immune response in animals such as upon oral ingestion carrying vectors that express the antigen.

A chimeric sequence or gene is defined as a DNA sequence containing at least two heterologous parts, i.e., parts derived from, or having substantial sequence homology to pre-existing DNA sequences which are not associated in their pre-existing states. The pre-existing DNA sequences may be of natural or synthetic origin.

A coding DNA sequence is defined as a DNA sequence form which the information for making a peptide molecule, mRNA or tRNA are transcribed. A DNA sequence may be a gene, combination of genes, or a gene fragment.

A foreign DNA is defined as a DNA that is exogenous to or not naturally found in the microorganisms to be transformed. Such foreign DNA includes viral, prokaryotic, and eukaryotic DNA, and may be naturally occurring, chemically synthesized, cDNA, mutated, or any combination of such DNAs. The foreign DNA of this invention is derived from or has substantial sequence homology to DNA of pathogenic microorganisms and viruses, or is a synthetic gene that encodes a protein that is of similar amino acid sequence to prokaryotic genes.

A fusion protein is defined as a protein containing at least two different amino acid sequences linked in a polypeptide where the sequences were not natively expressed as a single protein. Fusion proteins are frequently the result of genetic engineering whereby DNA sequences from different genes are joined together to encode a single protein composed of amino acid sequences from the originally separate genes.

A gene is defined as a discrete chromosomal region that codes for a discrete cellular product.

A microorganism is defined as a member of one of the following classes: bacteria, fungi, protozoa, or viruses.

A pre-existing DNA sequence is defined as a DNA sequence that exits prior to its use, in tote or in part, in a product of method according to this invention. While such pre-existence typically reflects a natural origin, pre-existing sequences may be of synthetic or other origin.

An immune response involves the production of antibodies, which are proteins called imunoglobulins. The antibodies circulate in the bloodstream and permeate the other body fluids, where they bind specifically to the type of foreign antigen that induced them. Binding by antibody inactivates viruses and bacterial toxins (such as tetanus or botulinum toxin) frequently by blocking their ability to bind to receptors on target cells. Antibody binding also malts invading microorganisms for destruction, either by making it easier for a phagocytic cell to ingest them or by activating a system of blood proteins, collectively called complement, which kills the invaders. Cell-mediated immune responses, the second class of immune responses, involve the production of specialized cells that react with foreign antigens on the surface of other host cells. The reacting cell can kill a virus-infected host cell that has viral proteins on its surface, thereby eliminating the infected cell before the virus has replicated. In other cases the reacting cell secretes chemical signals that activate macrophages to destroy invading microorganisms.

A secretory immune response (SIR) is defined as a specific type of immune response. It involves the formation and production of secretory IgA antibodies in secretions that bathe the mucosal surfaces of human and other animals and in secretions form secretory glands. An agent that causes the formation and production of such antibodies is considered to stimulate secretory immunity or to elicit a SIR, Secretory immunity is also sometimes referred to as mucosal immunity.

A substantial sequence homology is defined as a functional and/or structural equivalence between sequences of nucleotides or amino acids. Functional and/or structural differences between sequences having substantial sequence homology are frequently de minimus.

The invention provides an orally acceptable immunogenic composition comprising unpurified or partially purified recombinant immunogen expressed in bacteria, wherein the immunogen is expressed at a level such that upon oral administration of the composition to an animal, an immunogenic response is observed. Examples of the recombinant immunogens include, but are not limited to, HCV, IBDV, IBV, ILTV and PRRSV. In one embodiment, the recombinant immunogen is a chimeric protein. In certain embodiments, the HCV, IBDV, IBV, ILTV or PRRSV immunogen is capable of generating an immunogenic response to HCV, IBDV, IBV, ILTV or PRRSV when the immunogen interacts with a mucosal membrane. In particular aspects, the HCV, IBDV, IBV, ILTV or PRRSV immunogen is capable of binding a glycosylated molecule on the surface of a membrane of a mucosal cell. In further aspects, the HCV, IBDV, IBV, ILTV or PRRSV immunogen is a chimeric protein.

The present invention also provides an orally acceptable immunogenic composition comprising unpurified or partially purified recombinant immunogen expressed in bacteria for use as a medicament. Examples of the recombinant immunogens include, but are not limited to, HCV, IBDV, IBV, ILTV and PRRSV.

The present invention also provides uses of an orally acceptable immunogenic composition comprising unpurified or partially purified recombinant immunogen expressed bacteria for the manufacture of a medicament for the treatment of a disease caused by the immunogen. Examples of the recombinant immunogens include, but are not limited to, HCV, IBDV, IBV, ILTV and PRRSV.

The invention additionally provides an immunogenic composition comprising unpurified or partially purified recombinant immunogen expressed in a bacteria, wherein the inununogen is expressed in the bacteria at a level such that upon administration of the composition to an animal, an immunogenic response is observed. Examples of the recombinant immunogens include, but are not limited to, immunogens derived from HCV, IBDV, IBV, ILTV or PRRSV. In one embodiment, the immunogen is a chimeric protein. In certain aspects, the HCV, IBDV, IBV, ILTV or PRRSV inununogen is unpurified from the bacteria. In other aspects the HCV, IBDV, IBV, ILTV or PRRSV immunogen is partly purified from the bacteria. In some embodiments, the HCV, IBDV, IBV, ILTV or PRRSV immunogen is a chimeric protein. In other embodiments, the administration comprises injection. In further aspects, the administration comprises oral ingestion.

The invention also provides a vaccine comprising an immunogen of hog cholera virus, porcine reproductive and respiratory syndrome virus or infectious bursal disease virus expressed in bacteria. In certain embodiments, the immunogen is a hog cholera virusimmunogen. In other aspects, the imunogen is a porcine reproductive and respiratory syndrome virus imunogen, In additional aspects, the immunogen is an infectious bursa] disease virus.

The invention further provides a method for producing an imunogenic response to an inununogen in an animal, comprising the steps of expressing a recombinant immunogen in a bacteria, wherein the immunogen is expressed in the bacteria at a level such that upon administration of the bacteria to an animal, an immunogenic response to the inummogen is observed. In certain aspects, the imunogen is derived from HCV, IBDV, IBV, ILTV or PRRSV. In other aspects, the immunogen is partly-purified from the bacteria.

The development of safe and efficient avian influenza vaccines for human and animal use is essential for preventing virulent outbreaks and pandemics worldwide. In the present invention, it is found that genetically modified lactic acid bacteria such as *Lactococcus lactis* strains expressing the avian influenza HA gene can be used as an oral vaccine for the protection of H5N1 virus infection.

In one embodiment, oral administration of genetically modified *Lactococcus lactis* strains disclosed herein induced strong HA-specific humoral and mucosal immune responses in subjects which were able to withstand lethal dose of H5N1 virus infection.

The foregoing has outlined rather broadly the features and advantages of the present invention in order that the detailed description of the invention that follows may be better understood. Additional features and advantages of the invention will be described hereinafter which form the subject of the claims of the invention.

### BRIEF DESCRIPTION OF THE FIGURES

**Figure 1** shows construction and characterization of expression vector NZ9700 (HA). A 1704 bp HA gene fragment was fused into the secretion expression vector pNZ8110 (A). (B) Lane 1, lane 2 and lane 3 represented DNA marker DL15,000, pNZ8110 before being cut by double restriction endo-nuclease, and pNZ8110 after double restriction endonuclease cutting, respectively. (C) Lane 1 and lane 2 represented DNA marker DL2,000, and PCR detection of recombinant *L.lastic* NZ9700 after electroporation.
**Figure 2** shows HA protein expression of NZ (HA). Specific expression of HA protein was examined by SDS-PAGE (A) and Western blotting (B).
**Figure 3** shows microcapsules for oral administration.

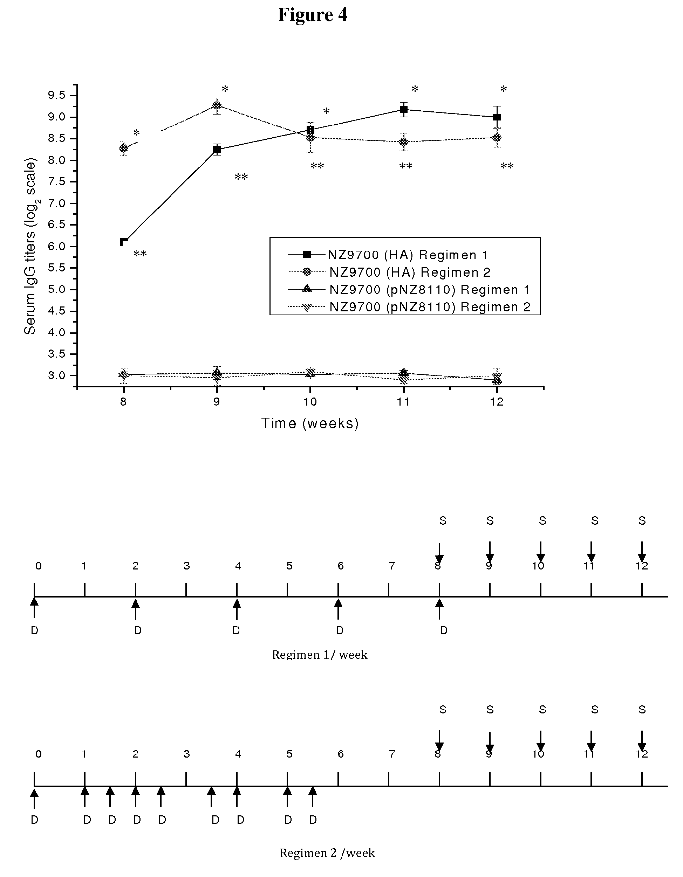

**Figure 4** shows HA-specific immune responses after oral administration. Each group of four mice was orally administered five (regimen 1) or eight (regimen 2) times over 8 weeks with 10⁹ CFU of NZ9700 (HA); NZ9700 (pNZ8110) was used as a negative control. Antigen-specific antibody (serum IgG) titers were determined by ELISA. Antibody titers were calculated as the inverse of the dilution (2ⁿ) of serum that corresponds to two times the background absorbance. Each data point represents the mean serum IgG titer of at least four individual animals, and is representative of two experiments; error bars, standard deviation; **p <* 0.0001 compared to control group; ***p* < 0.05 compared to control group. D(t) denotes for dosing; S (↓) for sampling. Results are expressed as the mean value (log2)±S.D. (n = 4).

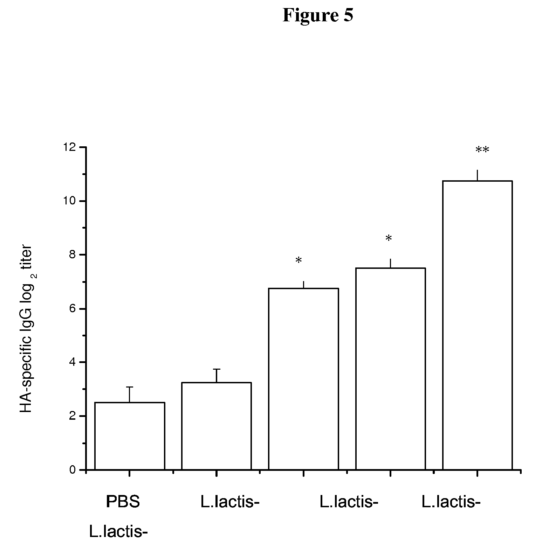

**Figure 5** shows HA-specific serum IgG was determined by ELISA. Mice were orally immunized with 150ul 10¹⁰ CFUs of L.lactis-pEmpt, L.lactis-pHA(HA protein expressed in cytoplasm), L.lactis- pSHA(HA protein was secreted), L.lactis- pgsA-HA(HA protein was displayed on the surface of cell wall), on days 0~3, 7~10, and 21~24. Immune sera were taken at day 34. * and ** represent statistically significant differences relative to the PBS control (*p<0.05, ** p<0.01). Data are expressed as the mean value (log2) ± S.D. of duplicate experiments.
**Figure 6** shows fecal IgA detected by ELISA. IgA antibody titers were monitored at 10^{th} week after the first immunization. * Mean values significantly different between the groups. (p<.05).
**Figure 7** shows survival rate after challenge with H5N1 virus. BALB/c mice (six per group) were administered orally with NZ9700 (HA) or NZ9700(pNZ8100) and challenged with H5N1virus. The percentages of survival rate post-challenge are shown.

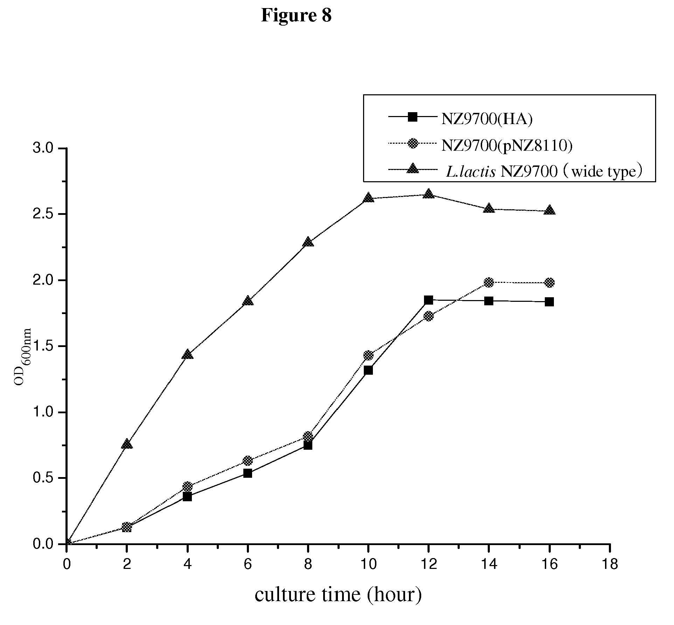

**Figure 8** shows growth curves of NZ9700 (HA), NZ9700 (pNZ8110) and *L. lactis* NZ9700. Culture samples were taken during the growth phase from 0 to 16 h. Growth of *L. lactis* was measured based on the optical density (OD) at 600 nm. Analysis of variance (ANOVA) indicated Significance was defined as a *P* value less than 0.05.

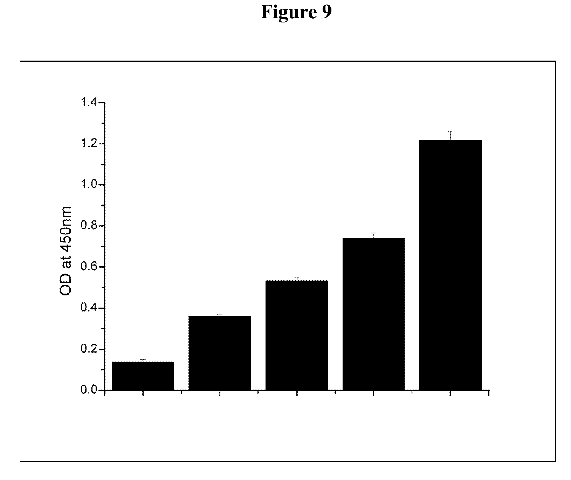

**Figure 9** shows HA-specific mucosal IgA was detected by ELISA. Fecal pellets were collected on day 34. * and ** represent statistically significant differences relative to the PBS control (*p<0.05, ** p<0.01). Data are given as mean ±SD of duplicate experiments.

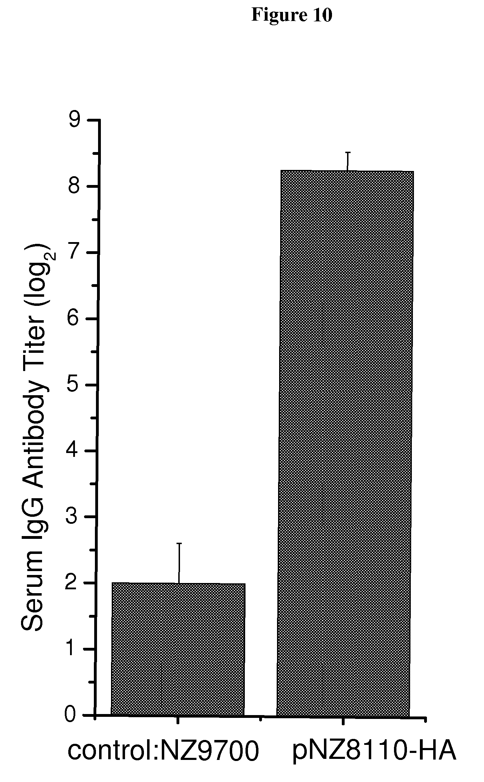

**Figure 10** shows HA-specific sera IgG antibody detected by ELISA.

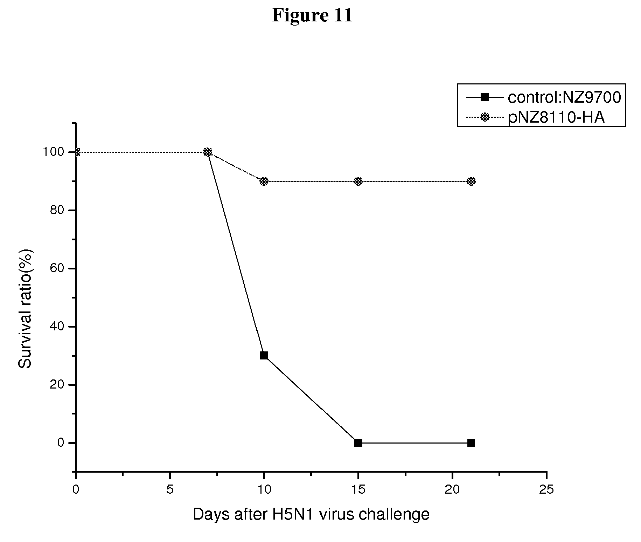

**Figure 11** shows the survival ratio of immunized mice against lethal H5N1 virus challenge.

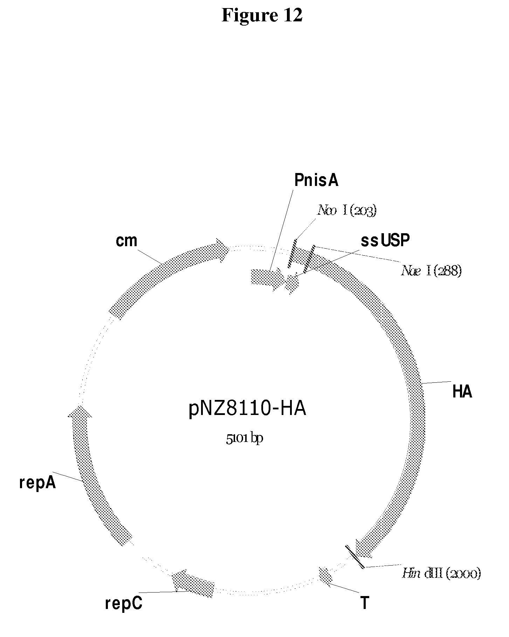

**Figure 12** shows the map of vector pNZ8110-HA.

### DETAILED DESCRIPTION OF THE INVENTION

This invention provides an inununogenic composition comprising unpurified or partially purified recombinant HCV, IBDV, IBV, ILTV or PRRSV immunogen expressed in a bacteria, wherein said immunogen is expressed in the bacteria at a level such that upon administration of said composition to an animal, an immunogenic response is observed. In an embodiment, the said HCV, IBDV, IBV, ILTV or PRRSV immunogen is unpurifed from said bacteria.

In a separate embodiment, the HCV, IBDV, IBV, ILTV or PRRSV immunogen is partly purifed from said bacteria. In another embodiment, the said HCV, IBDV, IBV, ILTV or PRRSV immunogen is a chimeric protein. In another separate embodiment, the said administration comprises injection. In still another embodiment, the said administration comprises oral ingestion.

This invention provides for a vaccine comprising an immunogen of hog cholera virus, porcine reproductive and respiratory syndrome virus or infectious bursal disease virus expressed in a bacteria. In an embodiment, the said immunogen is a hog cholera virus immunogen. In a separate embodiment, the said immunogen is a porcine reproductive and respiratory syndrome virus immunogen. In another embodiment, the said immunogen is an infectious bursal disease virus.

This invention provide a method for producing an immunogenic response to an immunogen in an animal, comprising the steps of expressing a recombinant immunogen in a bacteria, wherein said immunogen is expressed in the bacteria at a level such that upon administration of said bacteria to an animal, an immunogenic response to said immunogen is observed. In an embodiment, the said immunogen is derived from HCV, IBDV, IBV, ILTV or PRRSV. In another embodiment, the said immunogen is partly-purified from said bacteria.

The present invention provides novel DNA vaccines and edible vaccines for veterinary infectious diseases. Traditionally, vaccines comprise attenuated and/or killed virus, sub-unit vaccine (protein based), and/or DNA vaccine (DNA based) compositions. In preferred embodiments, the present invention discloses DNA and sub-unit vaccine compositions, methods of preparation and administration.

Specifically disclosed herein are DNA based vaccines and methods of preparing and administering such vaccines that combine efficacy, safety, and the opportunity for serological discrimination between vaccinated and infected animals.

Moreover, the vaccines and methods of using vaccines disclosed herein can elicit neutralizing antibodies that are regarded as an important specific defense against diseases including, but not limited to, HC, IBDV and/or PRRSV. The DNA vaccine disclosed herein further provides the advantages of chemical stability, as well as the ability to elicit both humoral and cell-mediate immunity. It is contemplated that certain embodiments of the present vaccine invention may provide better protection value than other vaccines against the same or like diseases. It has been demonstrated that using pcDNA3.1 inserted with VP5 - 5.2 and VP2 - 3.4 of IBDV HK46 was effective in fighting against IBDV.

As would be known to one of ordinary skill in the art, foreign antigenic agents for a vaccine may be produced using standard molecular biological techniques. For instance, in a well know use of this type of methodology, foreign DNA for human interferon was spliced into a plasmid vector, introduced into a bacterial cell, and then cloned. The gene for human interferon was be excised from a human chromosome, and a bacterial plasmid linearized, through the use of the same restriction enzyme. The interferon was gene joined with the plasmid by sticky end ligation, and the plasmid containing the interferon gene taken up by a bacterium. Each daughter bacterium inherited the interferon gene, the interferon-producing bacterial population was grown and the interferon isolated from the bacteria for administration to an animal, specifically a human patient.

Often, such methods are clinically applied to produce bacterial antigens for components of vaccines. Note that these antigens are generally located at the surface of the bacteria or are secreted molecules. Such antigens include, but are not limited to, one or more virulence molecules, secreted proteins, processed surface proteins, outer membrane proteins, capsular antigens, toxins, *pili*, and/or *flagella* antigens.

Traditional DNA vaccines or "naked DNA" requires purification to separate the recombinant plasmid form the host cells (i.e., bacterial cells) to obtain the clinical grade quality of the DNA vaccine. This type of vaccine is generally administered by injection. The utility of naked DNA for mucosal vaccination may be limited by the liability of DNA in tissue fluids. Hence, it is likely that a barrier must be placed between DNA vaccines and extracellular digestive enzymes if effective mucosal delivery is to be achieved. In the presence of cationic lipids, transfection of various cell types (e.g., cells of stomach and colon) with DNA vaccine was facilitated via a non-specific mechanism or a physiologic pathway present (Schmid, R.M., et al., 1994, Gastroenterol, 32:665-670).

Disclosed herein, however, a DNA vaccine was complexed to E. coli. In a preferred aspect of a DNA vaccine, bacteria that are expressing a foreign immunogen expressed from a DNA vector is administered as a vaccine. Such a vaccine composition is known herein as a "DNA-Bac' vaccine. The DNA-Bac embodiment of the present invention may provide an advantage in simplicity of creation and utilization, relative to other specific types of vaccines (e.g, plant vaccines, recombinant DNA vaccines, purified protein based vaccines, live or attenuated antigen vaccines, etc). Such a DNA-Bac vaccine of the present invention may provide the advantage of a reduced cost of vaccine production by lacking part or all of traditional purification processes, and thus be more cost-effective for use in the prevention or treatment of veterinary infectious diseases. Specifically demonstrated herein in a non-limiting embodiment, transformed bacterial host cells comprising a recombinant plasmid expressing an immunogen against a pig infectious virus (i.e., HCV) was used as a vaccine without a further purification process. The vaccine induced both humoral and cellular immunity responses. In certain embodiments, the DNA vaccine (e.g., DNA-Bac vaccine) targeted mucosal inductive sites.

In one aspect of the present invention, cultured bacteria may be prepared containing and expressing one or more genes for one or more foreign antigen(s) on one or more commercially available expression vectors, including but not limited to, plasmid, cosmid, BAC, PAC and/or P1 DNA vectors. The vector may be separated from the bacteria using methods and materials commonly utilized by those of skill in the art, including but not limited to, the use of pilot-scale plasmid preps, ultrapure 100 columns, contract CAN manufacture, large-scale plasmid preparations, EndoFree Plasmid Kits, QlAflter Plasmid Kits, QIAGEN Plasmid Kits, Large-scale BAC/PAC/PI/cosmid preparations, QIAGEN Large-Constmet Kit, High-throughput plasmid minipreps, QIAwell Plasmid Kits and the like. For example, bacteria containing the expression vector may be pelleted through centrifugation, undergo alklaline lysis and endotoxin removal steps, the vector further purified using, for example, a commercially available QIAGEN anion-exchange chromatography apparatus, as well as isopropanol precipitation to produce ultrapure plasmid DNA. Such methods can produce, for example, up to 100 mg high-copy plasmid DNA from 20 liters of LB culture ('60 g bacterial pellet).

An example of one type of DNA vaccine used beta-Gal as reporter construct. The DNA-Bae is active both in intro and in vivo. A DNA-Bac vaccine against the hog cholera virus induced significant enhancement in serum antibody responses and cytotoxie T lymphocyte responses as compared with naked DNA. Specifically, neutralization titers against the hog cholera virus were compared using DNA-Bac low dose, DNA-Bac high dose, a DNA vaccine and commercial attenuated vaccine, and demonstrated the effectiveness of DNA-Bac vaccine preparations at both low and high dose administrations. A rabbit fever responses assay was also conducted comparing the fever reductive ability of DNA-Bac low dose, DNA-Bac high dose, a DNA vaccine, a commercial vaccine and a control, and the efficacy of the DNA-Bac composition administered at low dose and high dose were demonstrated.

Another aspect of the present invention is a novel formulation of DNA vaccine for veterinary infection diseases. Advantages for a DNA vaccine composition, method of preparation or method of administration disclosed herein include relative low cost and flexible routes of delivery. This is of particular usefulness in the developing world. For example, Hong Kong and China veterinary vaccine market for chicken and pig farms are regionally focused, as most of the animal infectious virus are geographically localized, and vaccine manufactured by foreign firms made based on foreign strains do not give full protection most of the time.

For example, since HCV and PRRSV are two devastating viral disease of swine, they have already caused enormous financial losses worldwide. HC has already caused significant mortality and morbidity in commercial piggeries in many countries in Europe and Asia. PRRSV is now recognized throughout North America and Europe. To protect pigs against these two diseases, every individual pig has to be subjected to two sets of vaccination schemes. Such vaccination schemes suffer from the disadvantages of being time-consuming and/or money-consuming. In order to help overcome these disadvantages, and provide maximum protection against the disease, alternative vaccination methods and compositions are disclosed herein.

Additionally, the vaccines of the present invention may be used in protection for birds. For example, infectious bursal disease (IBD) is a worldwide economically important disease to poultry industry. Infectious bursal disease virus (IBDV) has been shown to be the causative agent and it is an avian lymphotropic virus that causes immunosuppression.

Thus, in certain aspects of the present invention, a DNA-Bac vaccine is contemplated for use in animal (including but not limited to pigs and chickens), for diseases including but not limited to IBD, HC and/or PRRS. It is contemplated that in certain embodiments, the invention comprises a DNA vaccine against PRRS, and thereby having use in the pig industry in protecting pigs against that disease.

Since the site and method of delivery will affect the nature of immune response (Feltquate, D.M., et al., 1997; J. Immunol, 158:2278.2284; Tones, C.A.T., et al., 1997, J Virol, 158:4529-4532), an appropriate procedure must be chosen that balances the practical aspects of vaccine delivery to large animals, with the desire to generate the most protective response possible. Intramuscular injection, although popular in many animal models, may be undesirable for livestock because of the potential effects on meat quality of food producing animals. Moreover, it is undesirable to have needle tracts or vaccine residues in the relevant tissue. Delivery of plasmid into the epithelium (skin or mucosal surfaces) is considered to be the most promising site of plasmid delivery because of the immune competence of these tissues. These tissues have highly developed immune surveillance function. Finally, because these tissues are the sites of entry by most pathogens, immunization at these sites is expected to be more effective in fighting against the disease.

Intramuscular injection was thought to lead to the rapid movement of DNA or DNA-transfected cells out of the injected muscle, so that the immune stimulatory events leading to antibodies production and cytotoxic T lymphocytes reactivity took place primarily in distal tissue.

Bone marrow derived antigen presenting cells, probably dendritic cells, has been demonstrated to be required for process antigen expressed from DNA plasmids (Ulmer, J.B., et at, 1996, Immunol, 89: 59-67; Ulmer, J.B., el at., 1996 Cur Opin Immunol, 8:531-536), though it is not clear whether the antigen presenting cells are themselves transfected with the DNA or pick up antigen from other cells.

Although muscle contains relatively few resident dendritic cells, macrophages or lymphocytes, the recent discovery at IL-15 and its high levels of expression in skeletal muscle cells (Grabstein, K.H., el al., 1994, Science, 264:961-965) indicated that muscle cells may not be as immunologically inert as once thought. Therefore, it was also a suitable site for immunization with DNA vaccine.

Currently, DNA vaccines are delivered by intramuscular or subcutaneous injection, which can induce systematic response, but generally no mucosal immunity. In the same time, the mucosal surface of the gastrointestinal tracts is the frequent site of transmission of numerous diseases. Hence, the mucosal immune response plays an important role in the protection against viral infection. In other embodiments, it is contemplated that a DNA vaccine of the present invention (e.g., a DNA-Bac vaccine) may be orally administered.

Specifically disclosed herein are DNA vaccines, including but not limited to, peDNA3.1-VP5-5.2 & VP2-3.4 and pHCV2.5, for use in vaccination against, but not limited to, IBDV HK45 strain and HCV Alford strain, respectively. The efficiency of these two vaccines delivered to the mucosal surface of gastrointestinal tracts was demonstrated.

There are gut-associated lymphoid tissues (GALT), which consist of the organized lymphoid tissues along the gastrointestinal tract. It includes isolated lymphoid follicles, Peyer's patches, appendix, tonsils, and mesenteric lymph nodes. In addition, many small lymphoid follicles populate the mucosa. From the mouth to the anus, these follicles are covered by specialized surface epithelial cells called M cells. They play a key role in deliver samples of foreign antigens by trans-epithelial transport from the lumen to organized lymphoid tissues. It is contemplated that GALT sites on mucosal surfaces may be a desirable location for contact with the vaccines of the present invention during or after administration.

Disclosed herein, the viral coat proteins were inserted into a mammalian expression vector, pcDNA3.1 and used as the DNA vaccine. The DNA vaccine, pcDNA3.I-VP5-5 2&VP2-3.4, pHCV2.5 and pcDNA3.1-ORFS have been reported to be effective in fighting against IBD, HC and PRRS respectively when they were administered through intramuscular injection. Since intramuscular injection was undesirable for livestock because of the potential effects on meat quality of food producing animals, the effectiveness of these three DNA vaccines delivered to the mucosal surface of gastrointestinal tracts of the animals was determined.

It has been reported that combined DNA immunization could induce double-specific protective immunity and non-specific response in Rainbow Trout (Pierre et at, 1998, Virol 249: 297-306). However, the inventors know of no report of combined DNA vaccine against pig diseases. The efficacy of vaccine compositions and methods of delivery against HCV, IBDV and/or PRRSV, along or in combination, is disclosed herein.

DNA vaccines using pHCV2.5 and pcDNA3.1-ORFS construct have been shown to be effective in protecting pigs against HC and PRRS respectively. However, subjecting to two separated vaccination schemes was very time-consuming and money-consuming. Disclosed herein, the efficacy of combined DNA vaccine against HC and PRRS was demonstrated.

After ELISA and Western blot analysis, it was observed that IBD and HC DNA vaccines were both effective in triggering specific antibodies against the respective virus when they were delivered orally.

It could also be seen that combined HC-PRRS DNA vaccine was effective in triggering specific antibodies against HCV and PRRSV simultaneously. The antibody reaction was rather similar in quality and intensity to that obtained with separated immunization.

The present invention provides for a method of using genetically modified lactic acid bacteria such as *Lactococcus lactis* strains expressing the avian influenza HA gene as an oral vaccine for protection against H5N1 virus infection. In one embodiment, the oral administration of recombinant *L. lactis* NZ9700 (HA) microcapsules can induce significant HA-specific humoral and mucosal immune responses, and most importantly, provide protection against H5N1 virus challenge.

In one embodiment, the method comprises an oral dosing regimen which can be easily administered to both human and animal populations. In another embodiment, the method has the ability to generate a mucosal immune response.

The present invention provides a method of inducing immune responses to an antigen, comprising the step of administering to an animal or human genetically modified lactic acid bacteria expressing the antigen. Examples of lactic acid bacteria include, but are not limited to, *Lactococcus, Streptococcus, Lactobacillus, Leuconostoc*, *Pediococcus, Brevibacterium* and *Propionibaterium.* In one embodiment, the lactic acid bacteria are of the genus *Lactococcus* as described in U.S. Patent No. 5,580,787, 6,333,188, and 7,553,956. In another embodiment, the lactic acid bacteria are of the species *Lactococcus lactis.*

The genetically modified lactic acid bacteria of the present invention are capable of inducing immune responses when administered to a subject. Immune responses induced by the bacteria of the present invention include, but are not limited to, humoral immune responses and mucosal immune responses. For example, the bacteria of the present invention are capable of inducing systemic IgG responses and mucosal IgA responses.

In one embodiment, the genetically modified lactic acid bacteria of the present invention are capable of inducing protective immune responses, i.e. immune responses that can protect immunized subjects from lethal challenges by pathogens (such as viruses or bacteria).

In general, the lactic acid bacteria of the present invention are genetically modified to express one or more antigens. In an embodiment, said antigens are heterologous. Examples of heterologous antigens include, but are not limited to, bacterial, protozoan, fungal, and viral antigens. Sources of heterologous antigens include, but are not limited to, influenza virus, helicobacter pylori, Salmonella, rotavirus, respiratory coronavirus, etc. as described in U.S. Patent No. 6,551,830, 7,432,354, and 7,339,461.

In one embodiment, a viral antigen such as hemagglutinin of avian influenza virus H5N1 can be expressed in genetically modified lactic acid bacteria.

The genetically modified lactic acid bacteria of the present invention can be administered in amounts and by using methods that can readily be determined by persons of ordinary skill in this art. The vaccines of the present invention can be administered and formulated, for example, for oral administration, either as liquid solutions or suspensions, or solid forms suitable for solution in, or suspension in, liquid prior to administration. The preparation may also be emulsified, or the ingredients mixed with excipients such as, for example, pharmaceutical grade mannitol, lactose, starch, magnesium stearate, sodium saccharine, cellulose, magnesium carbonate, and the like. These compositions take the form of solutions, suspensions, tablets, pills, capsules, sustained release formulations, nose drops or powders.

The vaccines of the present invention can also be in the form of injectables. Suitable excipients would include, for example, saline or buffered saline (pH about 7 to about 8), or other physiologic, isotonic solutions which may also contain dextrose, glycerol or the like and combinations thereof. However, agents which disrupt or dissolve lipid membranes such as strong detergents, alcohols, and other organic solvents should be avoided. In addition, if desired, the vaccine may contain minor amounts of auxiliary substances such as wetting or emulsifying agents, pH buffering agents, and/or adjuvants well-known in the art which enhance the effectiveness of the vaccine.

Generally, the vaccine of the present invention may be administered orally, subcutaneously, intradermally, or intramuscularly in a dose effective for the production of the desired immune response. The vaccines are administered in a manner compatible with the dosage formulation, and in such amount as will be prophylactically and/or therapeutically effective. The quantity to be administered depends on the subject to be treated, the capacity of the subject's immune system to develop the desired immune response, and the degree of protection desired. Precise amounts of the vaccine to be administered in view of the subject and antigen used would be readily determined by one of skill in the art.

In one embodiment, the genetically modified lactic acid bacteria of the present invention can be administered to a subject in a number of ways, such as orally, or by intranasal administration, intramuscular injection, subcutaneous injection, and vaginal application as described in U.S. Patent No. 7,541,044, and 7,476,686.

The genetically modified lactic acid bacteria of the present invention can be formulated in a number of ways, such as encapsulated inside acid labile microcapsules, enteric coated microcapsules and capsules, polymer hydrogels, or adhesive polymer patches.

The present invention also provides genetically modified lactic acid bacteria expressing a heterologous antigen. Examples of lactic acid bacteria and heterologous antigens have been described above. In one embodiment, these lactic acid bacteria can be used as oral vaccines.

The present invention also provides a composition comprising the genetically modified lactic acid bacteria described herein. In one embodiment, the composition further comprises a pharmaceutically acceptable carrier.

The present invention also provides uses of the genetically modified lactic acid bacteria described herein as a medicament for inducing immune responses in a subject.

In certain aspects, it is contemplated that the vaccine inventions disclosed herein may be used alone or in combination with one or more other pharmacological or therapeutic agents. In certain aspects, such an agent may comprise a biopharmaceutical, including but not limited to, one or more cytokines including but not limited to one or more interferons, interleukins, colony stimulating factors, and/or tumor necrosis factors; one or more antisense nucleic acid compositions and the like; one or more cytokines; one or more gene therapy agents or methodologies; one or monoclonal antibodies; one or more clotting factors; one or more additional vaccines or vaccine related compositions or methods of administration; and/or one or more hormones.

### A. Construction and Generation of a DNA Vaccine

The strategy of constructing and generating a DNA IBD and HC vaccine is described herein as a merely as an exemplary aspect of the present invention. However, the present invention is not limited to the specific constructs disclosed herein. As would be understood by those of ordinary skill in the art, other constructs may be created and other genes from various pathogens may be expressed using the techniques described herein to produce a DNA vaccine. In certain preferred aspects, the DNA constructs are expressed in a microorganism (e.g.bacteria). In certain aspects, a DNA/bacterial vaccine ("DNA-Bad') is partly purified. As used herein, "partly purified" means removal of about 10%, about 20%, about 30%, about 40%, about 50%, about 60%, about 70%, about 80%, about 90%, or greater, and any range derivable therein, of bacterial component(s) from the DNA construct and/or expressed immunogen(s). In additional aspects, a DNA vaccine is administered orally, to a mucus membrane, or a combination of both. However, it is contemplated that such a DNA and/or bacteria vaccine may be administered using any technique known to those of ordinary skill in the art, and it is further contemplated that the DNA and/or bacteria vaccine may be administered in combination with one or more immunogenic or pharmacological agents, such as, for example, an adjuvant.

### 1. Design of DNA construct

For IBD vaccine, VP5-5.2 and VP2-3.4 were cloned into pcDNA (+) vector under the control of a CMV promoter.

For HC vaccine, three consecutive genes (E0, E1 and E2) that encode viral structural glycoprotein were cloned into expression vector pcDNA3,1 with CMV promoter. The recombinant plasmid was called pHCV2.5 since the size of the insert was about 2.5 kb. For PRRS vaccine, ORFS of PRRSV that encodes the major envelope protein GP5 was cloned into expression vector peDNA3.1 with upstream CMV promoter. This vector was designed specifically for eukaryotic expression. These three constructs have been prepared and their identities have been confirmed previously.

### 2. Transformation of E. coli cell

501 thawed competent E, coli cell and 5 1 plasmid DNA were added to a cuvette that has been chilled on ice for 5 minutes. It was then placed into the sample chamber of a bacteria electroporator (Bio Rad E. coil Pulser Transformation Apparatus), which has been set to 1.8 kV. I ml SOC medium (Gibco BRL) was added immediately to the euvette after applying the pulse. After 1 hour of inoculation at 37°C, 100 1 of transformed cell was spread on a LB agar plate containing ampicillin and it was incubated overnight.

### 3. Large-scale plasmid extraction for injection

A single transformed colony on the agar plate was picked and inoculated in 2 ml LB supplemented with ampicillin. It was grown at 37°C overnight with shaking. 1.5 ml culture was then added to 1.5 L LB supplemented with ampicillin and grown at 37°C overnight with shaking. The cells were spun down at 8000X g (Beckman JA-14 Rotor) for 10 minutes and the supernatant was discarded. The cells were resuspended in 75 ml P1 solution (50 mM glucose, 25 mM Tris, pH 8, 10 mM EDTA). 102.5 ml P2 solution (0.2 M NaOH, 1% sodium dodecyl sulfate) was added and it was allowed to stand at room temperature for 5 minutes. 150 ml P3 solution (5 M potassium acetate solution, pH 4.8) was then added. After standing on ice for 30 minutes, the mixture was centrifuged at 8000 rpm for 10 minutes (Beckman JA-14 Rotor). 375 ml isopropanol was added to the supernatant and allowed to stand at 4°C overnight. It was then centrifuged at 12000 rpm for 10 minutes (Beckman JA-14 Rotor). The pellet was resuspended in 112.5 ml water containing RNase A. 7.5 ml P3 solution and 112.5 ml 100% ethanol was then added and it was kept at -20°C for 1 hour. After centrifuging at 12000 rpm for 10 minutes (Beckman JA-14 Rotor), the pellet was washed with 150 ml 70% ethanol. It was centrifuged again at 12000 rpm for 10 minutes (Beckman JA-14 Rotor). The pellet obtained was then resuspended in 2 ml 1X PBS and its concentration measured by fluorometer.

### Large-scale bacteria preparation for feeding

A single transformed colony containing the desired plasmid on the agar plate was inoculated in 2 ml LB supplemented with ampicillin. It was grown at 37°C overnight with shaking. 1.5 ml culture was then added to 1.5L LB supplemented with ampicillin and grown at 37°C overnight with shaking.

The cells were spun down at 8000X g for 10 minutes (Beckman JA- 14 Roster) and the supernatant was discarded. The E ccli cells were dried at 37°C overnight and were resuspended in IX PBS. It was then sonicated (Branson Sonifer 250) for 10 minutes. I% v/v antibiotics (Penicillin-Streptomycin, Gibco BRC) were finally added to it.

In one embodiment, the antigen expressed by the genetically modified bacterium is a bacterial antigen or viral antigen. For example, the antigen is hemagglutinin of avian influenza virus H5N1. In another embodiment, the antigen is capable of binding a glycosylated molecule on the surface of a mucosal cell membrane. In yet another embodiment, the antigen is a chimeric protein.

The present invention also provides a method of inducing an immune response to an antigen in a subject, comprising the step of administering to said subject the composition described herein. In one embodiment, the immune response is humoral immune response, mucosal immune response, or protective immune response. In another embodiment, the composition is administered orally.

The present invention also provides a composition as described herein for use as a medicament for inducing an immune response in a subject.

The present invention also provides uses of the composition described herein for the preparation of a medicament for inducing an immune response in a subject.

The invention will be better understood by reference to the Experimental Details which follow, but those skilled in the art will readily appreciate that the specific experiments detailed are only illustrative, and are not meant to limit the invention as described herein, which is defined by the claims which follow thereafter.

Throughout this application, various references or publications are cited. Disclosures of these references or publications in their entireties are hereby incorporated by reference into this application in order to more fully describe the state of the art to which this invention pertains.

It is to be noted that the transitional term "comprising", which is synonymous with "including", "containing" or "characterized by", is inclusive or open-ended and, as used herein, does not exclude additional, un-recited elements or method steps.

### EXAMPLE 1

### Materials and Methods

In the present invention, a recombinant *L. lactis* vector encoding the hemagglutinin (HA) gene of avian influenza virus H5N1 was constructed. The live vectors were encapsulated inside alginate microcapsules or enteric coating capsules to protect them from acid destruction and maintain antigen expression for an extended time period. Mice that were immunized orally mounted an effective immune response against H5N1 virus.

### Plasmid constructs and transformation

A 1704 bp fragment containing the HA gene from pGEM-HA (kindly supplied by Prof. Ze Chen, Wuhan, China) was amplified by polymerase chain reaction(PCR) using the following primer pairs with *Nae*I or *H*indIII sites underlined: 5'-tctgccggcgagaaaatagtgcttctt- 3' (SEQ ID NO. 53), and 5'-cccaagctttaaatgcaaattctgcattgtaacg- 3'(SEQ ID NO. 54). The PCR product was sequenced. The resulting *Nae*I/HindIII fragment was cloned into various vectors including L. lactis-pHA (HA protein expressed in cytoplasm), L. lactis-pSHA(HA proteins secreted), and L. lactis- pgsA-HA(HA protein was displayed on the surface of cell wall).

*L. lactis* was cultured in M17 broth medium (Difco, Sparks, MD, USA) containing 0.5% (W/V) glucose (GM17) at 30°C overnight without agitation. *L. lactis* NZ9700 was purchased from NIZO (the Netherlands) and transfected with the pNZ8110-HA vector by electroporation using a Gene Pulser (Bio-Rad) at 25 uF, 1000V with 0.1-cm electrode cuvette (Bio-Rad). The highest HA expressing *L. lactis* NZ9700 clone was selected and named NZ9700(HA). As a negative control, *L. lactis* NZ9700 was transformed with an empty vector pNZ8110 to generate NZ9700 (pNZ8110). Plasmid DNA was isolated from *L. lactis* NZ9700 for PCR detection and sequencing of the target gene.

### HA antigen expression in vitro

To confirm the expression of the HA gene insert, *L. lactis* were cultured in GM17 medium with 10µg/ml chloramphenicol at 30°C overnight without agitation. The cultures were centrifuged at 5000xg for 8 minutes at 4°C. Pellets were washed twice with wash buffer in phosphate-buffered saline [PBS], and bacteria were suspended in equal volumes of 2×sodium dodecyl sulfate (SDS) buffer (125 mM Tris[tris(hydroxymethyl) aminomethane]-HCl, pH 6.8, 4% SDS, 20% glycerol, 0.01% bromophenol blue, and 10% β-mercaptoethanol). After boiling for 10 minutes, the cell lysates were electrophoresed on 4% concentration gel and 10% separated polyacrylamide gel. Another gel was transferred to a nitrocellulose membrane. Protein was detected using mouse anti-HA antibody followed by affinity-purified horseradish peroxidase (HRP)-conjugated goat anti-mouse IgG. The membrane was radiographed on X-film using the ECL Western Blotting Detection System. The NZ9700 (pNZ8110) was used as a control.

### Alginate microcapsule preparation

Concentrated sodium alginate solution was added to the *L. Lactis* culture medium and mixed well. Soybean oil containing 0.2% of Tween-80 was then mixed with the solution to form an emulsion with constant stirring (540 rpm) for 10 min. Calcium chloride solution (1% w/v) was gradually infused into the emulsion until the emulsion collapsed. The suspension was then subjected to 3000g centrifugation to collect the microcapsules. The pellet was resuspended, washed with deionized water, and then lyophilized.

### Enteric coated capsule preparation

L. Lactis culture medium was mixed with dextran and lyophilized. The lyophilized powder was filled into enteric coated capsules.

### Oral immunizations in mice

Eight-week-old BALB/c female mice were purchased from SLC, Inc. (Shanghai, China) and housed in the Animal Center of the school of pharmacy of the Shanghai Jiao Tong University. The mice were kept under standard pathogen-free (SPF) conditions and provided with free access to food and water during the experiments.

After overnight fasting, groups of ten mice received 200 µl 10¹⁰ CFU (colony-formation units) of *L. lactis* using an oral zonde needle. Mice were immunized at days 0~3, 7~10, and 21~24. Sera were taken at day 34.

### Antibody titers analysis

Samples of sera (collected by retro-orbital puncture) and feces were collected 2 weeks after final immunization. Sera were stored at -20°C until use. Fecal pellets (100 mg) were suspended in 0.5 mL sterile PBS. After centrifugation at 12 000× *g* for 5 minutes, the supernatants were collected and tested for IgG or IgA by ELISA.

The Enzyme-linked immunosorbent assay (ELISA) assay was performed as described elsewhere[21]. Briefly, 96-well microtiter plates were coated with mouse anti-HA antibody overnight at 4°C. The wells were blocked with PBS-1% bovine serum albumin (BSA) and incubated for 2 hours at room temperature. Serially diluted serum or fecal suspension (100µl) was added for 1 hour at 37°C. Bound Ab was detected using HRP-conjugated goat anti-mouse IgG or HRP-conjugated goat anti-mouse IgA. The ELISA end-point titers were expressed as the highest dilution that yielded an optical density (OD) greater than the two times mean OD₄₅₀ₙₘ plus S.D. of similarly diluted negative control samples.

### Hemagglutinin inhibition assay

Sera were treated with receptor destroying enzyme (RDE) to inactivate non-specific inhibitors of agglutination, prior to being tested. Briefly, three parts RDE were added to one part sera and incubated overnight at 37 °C. Samples were then heat-inactivated at 56°C for 30 min. Following inactivation, PBS was added to the sample for a final serum dilution of 1:10. The diluted samples were then stored at 4°C prior to testing (up to 6 days) or stored at -20 °C.

Chicken Red Blood Cells (CRBC) were adjusted with PBS to obtain a 1% (v/v) suspension and kept at 4°C until use within one week of preparation. Mice serum samples were diluted serially in two-fold dilutions using v-bottom microtiter plates. An equal volume of H5 subtype standard antigen, adjusted to approximately 8 HAU/50µl, was added to each well. The plates were covered and incubated at room temperature for 30 min followed by the addition of 1% CRBC. The plates were mixed by agitation, covered, and the CRBC were allowed to settle for 30 min at room temperature. The HAI titer was determined by the reciprocal dilution of the last row that contained non-agglutinated CRBC. Positive and negative ferret serum controls were included for each plate. HI titers of 2³ or higher were counted as positive.

### H5N1 virus challenge

Eight-week-old female BALB/c mice were used in all experiments. Influenza A virus (A/chicken/Henan/12/2004(H5N1)) was used for the virus challenge. Fifty percent mouse infectious dose (MID50) and 50% lethal dose (LD50) titers were determined as previously described [22]. To evaluate the degree of protection from lethal challenges, vaccinated mice were infected intranasally (i.n.) with 10 LD50 of Influenza A virus (A/chicken/Henan/12/2004(H5N1)) virus(lethal challenge dose). Six mice from each group were examined daily for survival for 21 days.

All values are expressed as meansstandard error (SE). Statistical analysis of the experimental and control data were determined by using Student's *t*-test or analysis of variance (ANOVA). Significance was defined as a *P* value less than .05. For survival, the probability was calculated by using Fisher's exact test, comparing the rate of survival in mice immunized with the recombinant *L. lactis* to that of the control groups.

### Results

### Construction of recombinant L. lactis NZ9700 (HA)

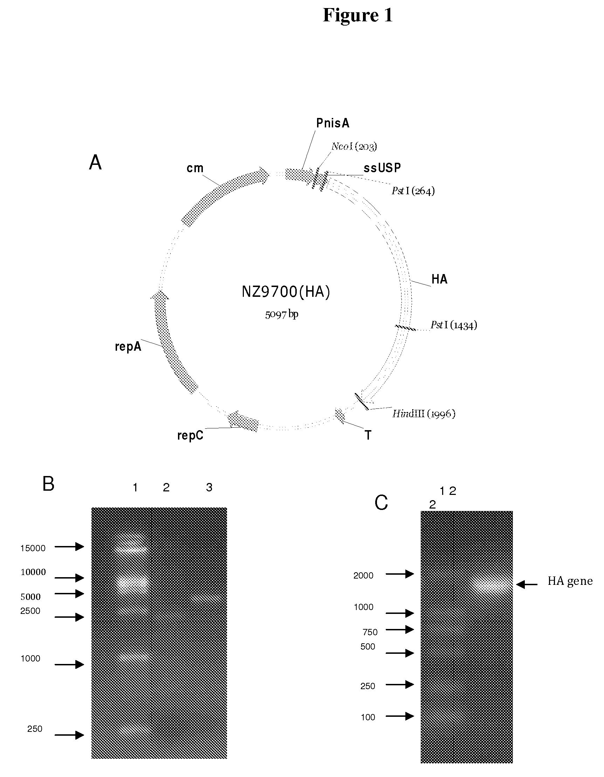

The avian influenza HA gene was cloned into pNZ8110 (Figure 1A) and transformed into the *L. lactis NZ9700 strain.* Three vectors including L. lactis-pHA (HA protein expressed in cytoplasm), L. lactis-pSHA (HA protein was secreted), and L. lactis- pgsA-HA (HA protein was displayed on the surface of cell wall) were constructed. Electrophoresis analysis of the expression vectors and PCR detection of the HA sequence in the clones were shown in FigurelB and Figure 1C respectively. The full length HA gene (1704 bp) was sequenced after PCR amplification and proved to be correct.

### HA protein expression by NZ9700 (HA) in vitro

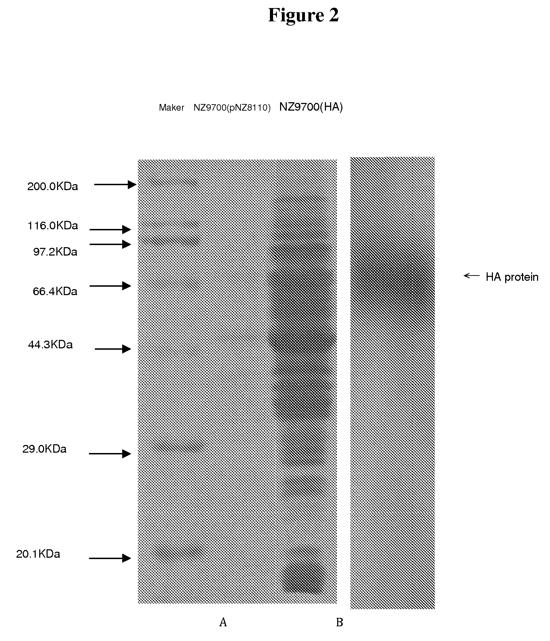

To evaluate whether the encoded protein was actively produced by the NZ9700 (HA) bacteria, SDS-PAGE and Western blotting analysis were done as shown in Figure 2. The expression of the HA protein (66.2 kDa) was detected by SDS-PAGE in L. lactis-pHA lysates but not NZ9700 (pNZ8110) lysates (Figure 2A). The culture supernatant was also analyzed by Western blot and the HA band was clearly visible (Figure 2B), indicating HA proteins were expressed and secreted outside of the *L. lactis* due to the presence of the secreting signal Usp45.

### Microcapsules for oral administration

Alginate microcapsules were prepared to encapsulate NZ9700(HA) to facilitate oral administration and to protect the live vectors from the acidic environment in the stomach.

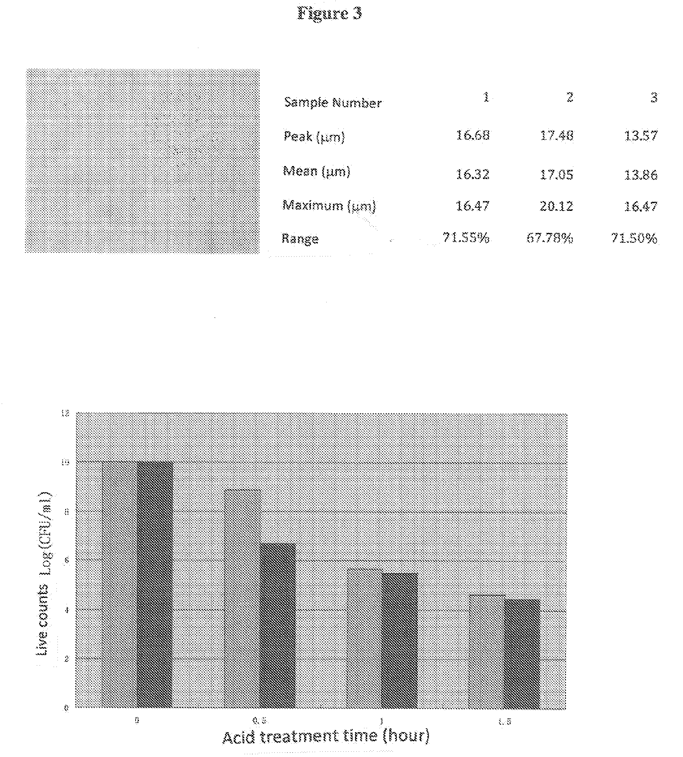

Figure 3A showed a microscopic picture of an alginate capsule containing L. Lactis. The size distribution of these capsules were around 15um. The protective effect on L. Lactis by these microcapsules against acidic environment was demonstrated in Figure 3C.

### Immune responses induced by oral vaccination with NZ9700 (HA) in mice

10¹⁰ colony-formation units (CFUs) of L. lactis were administered orally to BALB/c mice (SPF grade) 5 or 9 times over a 8-week period. The effect of vaccination on the production of HA-specific serum IgG and fecal IgA antibodies was examined 5 weeks after the final immunization. Two vaccination regimens were investigated (Figure 4). Both IgG and IgA responses were detected in both groups immunized with L. lactis-pHA, while those treated withNZ9700 (pNZ8110) had no HA specific antibody at all (Figure 4).

As shown in Figure 4, serum IgG titers were significant after 4 biweekly oral doses and remained high for at least several months afterwards. An increase in the number and frequency of the oral doses as in regimen 2 did result in faster IgG generation, but the final titers were similar. The maxima dilution of serum IgG was 2^{9.4} (regimen 1).

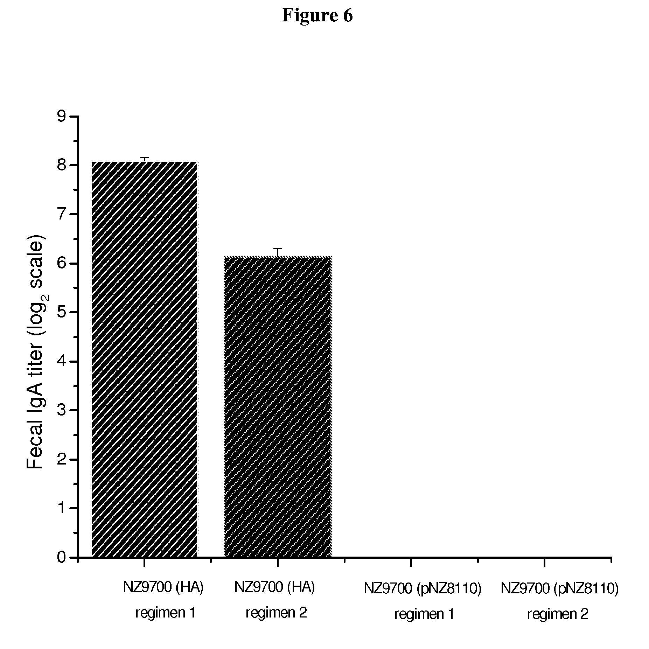

Fecal IgA antibodies were also examined at tenth week after the initiation of immunization. Both L. lactis dosing regimens resulted in HA specific mucosal IgA production, while NZ9700 (pNZ8110) dosing did not (Figure 6). Interestingly, dosing regimen 1 seemed to be more effective at IgA induction than dosing regimen 2 (Figure 4).

### Hemagglutinin inhibition (HAI) assay

The HA neutralization ability of IgG antibodies generated after oral vaccination was examined using the classic hemagglutinin inhibition (HAI) assay. Mouse sera taken at 10 weeks after the first dosing in regimen 1 were tested and the data were listed in table 1. The geometric mean HI titer was 2⁷ in NZ9700(HA) treated group, compared to the background level (< 2³) in the NZ9700(pNZ8110) group.

### Protection against lethal H5N1 virus challenge

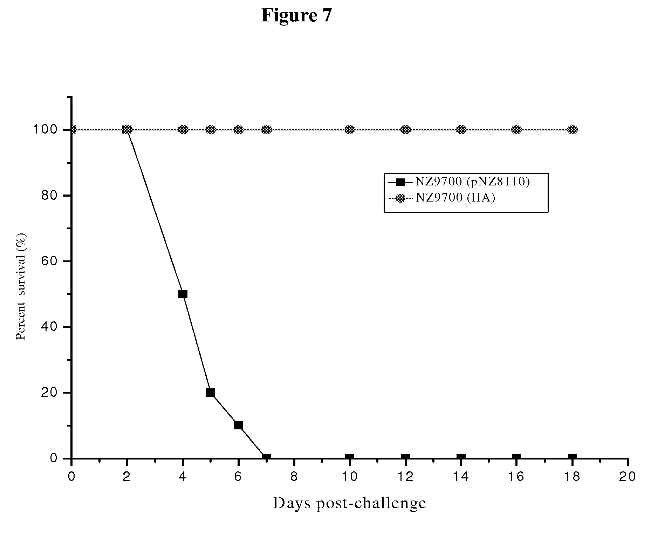

To test whether NZ9700 (HA) could stand against H5N1 virus challenge, we performed lethal challenge experiments at the tenth week after five biweekly oral dosing. The results indicated that mice immunized with NZ9700 (HA) were protected completely (6/6) after being challenged with a lethal dose (10 LD50) of H5N1 virus, and the percent survival rate was 100%. The control group [treated with NZ9700 (pNZ8110)] however was killed completely within seven days (Figure 7).

**Table 1**

| **Hemagglutinin inhibition titers of vaccinated mouse sera against H5 subtype standard antigen** | |
|---|---|
| Group | HI titer before challenge^{a}(GMT) |
| NZ9700 (HA) | 2⁷ |
| NZ9700 (pNZ8110) | <2³ |

| | |
|---|---|
| GMT: geometric mean titer. ^{a} HI titer of 2³ was recorded as positive. | |

### EXAMPLE 2

### Demonstration of DNA Constructs in Animal Models

BALB/c mice (male) of 7 to 8 weeks of age were provided by the Laboratory Animal Unit of The University of Hong Kong. The mice were kept and fed by the animal technicians in the animal house of Department of Zoology, The University of Hong Kong.

### A. Design of Animal Inoculations

35 BALB/c mice were randomly divided into 7 groups, each group consisted of 5 mice. The approach was separately replicated in twice (demonstration 1 and demonstration 2).

Details of the embodiment of the invention are shown in Table 3:

| **Table 3** | | |
|---|---|---|
| **Details of demonstration setup** | | |
| | **Group #** | **Treatment** |
| IBD vaccine | Group 1 | Injected with 100 g pcDNA3.1-VP5-5.2 & VP2-3,4 intramuscularly. |
| IBD vaccine | Group 2 | Fed with *E. coli* cells containing 100 g pcDNA3.1-VP5-5.2 & VP2-3.4. |
| HC vaccine | Group 3 | Injected with 100 g pHCV2.5 intramuscularly. |
| HC vaccine | Group 4 | Fed with *E. coli* cells containing 100 g pHCV2.5. |
| PRRS vaccine | Group 5 | Injected with 100 g pcDNA3.1-ORF5 intramuscularly. |
| HC-PRRS combined vaccine | Group 6 | Injected with 100 g (pHCV2.5 and pcDNA3.1-ORF5) intramuscularly. |
| Control | Control Group | Without any treatment |

In Groups 1, 3, 5 and 6, the mice were injected intramuscularly at the tibialis using 27-gauge needles. DNA vaccine was injected at a single site each time. For Groups 2 and 4, the mice were fed by using feeding needle (18060-20, Fine Science Tools).

The vaccination scheme was the same for all the mice, and it was shown in the following tables.

| **Table 4** | |
|---|---|
| **Vaccination scheme of mice in demonstration 1** | |
| **Day** | **Treatment** |
| 0 | Bleeding & 1^{st} vaccination |
| 7 | Bleeding |
| 8 | 2^{nd} vaccination |
| 15 | Bleeding |
| 18 | 3^{rd} vaccination |
| 28 | Bleeding |

| **Table 5** | |
|---|---|
| **Vaccination scheme of mice in demonstration 2** | |
| **Day** | **Treatment** |
| 0 | Bleeding & 1^{st} vaccination |
| 8 | Bleeding |
| 10 | 2^{nd} vaccination |
| 17 | Bleeding |
| 19 | 3^{rd} vaccination |
| 28 | Bleeding |

### B. Blood Sampling and Serum Preparation

For the first three times, mice were bled by cutting small portion of their tails (-2 mm) and 200 gl of blood was collected, For the last time, mice were bled by cardiac puncture under ether anesthetic, and 700 gl of blood was bled using 27-gauge needle. Blood samples were allowed to clot by incubation at room temperature for 4 hours. The clotted blood was centrifuged at 5000 rpm for 10 minutes and the serum was collected.

### C. Growing CEF Cells

I ml CEF cells (Chicken Embryo Fibroblast) were thawed from the liquid nitrogen. The cells were then resuspended in 10 ml Dulbecco's Modified Minimal Essential Medium (DMEM) with 10% heat-inactivated fetal calf serum (FCS, Gibco BRL) and 1% antibiotics (Penicillin-Streptomycin, Gibco BRC) and seeded in a T-75 flask (Falcon). The cells were incubated at 37°C with 5% CO2 overnight.

The 100% confluent CEF cells monolayer was washed with 1X PBS twice and detached by 0.05% trypsin EDTA (Gigco BRL) for 5 minutes. Ttpsin was neutralized by 10% FCS in MEM and centrifuged at 1000 rpm for 5 minutes. The cell pellet was resuspended in DMEM with 10% FCS and 1% antibiotics (Penicillin-Streptomycine, Gibco BRC). The cells were seeded to two T-75 flasks and if needed, one T- 175 flask in the split ratio of I to 3 for subculturing.

### D. Growing PIC 15 Cells/MARC-145 Cells

1 ml PK-15 / MARC-145 cells was thawed from the liquid nitrogen. The cells were then resuspended in IOral Minimum Essential Medium (MEM) with 10% heat-inactivated fetal calf serum (FCS, Gibco BRL) and 1% antibiotics (Penicillin-Streptomycin, Gibco BRC) and seeded to at T-75 flask (Falcon). The cells were incubated at 37°C with 5% CO 2 overnight.

The 100% confluent cells monolayer was washed with IX PBS twice and detached by 0.05% trypsin EDTA (Gibco BRL) for 5 minutes. Trypsin was neutralized by 10% FCS in MEM and centrifuged at 1000 rpm for 5 minutes. The cell pellet was resuspended in MEM with 10% FCS and 1% antibiotics (Penicillin-Streptomycin, Gibco BRC). The cells were seeded to two T-75 fasks and if needed, one T-175 flask in the split ratio of 1 to 3 for subculturing,

### E. Virus Purification from Tissue Culture

CEF, PK-15 and MARC-145 cell were used for the amplification of IBDV, HCV and PRRSV respectively. The cell lines were infected with the respective virus in DMEM (for CEF) or MEM (for PK-15 and MARC-145) supplemented with 10% heat-inactivated PBS for 5 days. Virus was released from the cells by freezing and thawing for 3 times. Any cells attached on the culturing flasks were scraped off. Detached cells and cell debris were removed by centrifugation at 2000 rpm for 10 minutes. The supernatent containing the partially purified virus was then centrifuged at 30000 rpm for 2 hours with a Beckman 40-Ti rotor. The pellet containing the purified virus was finally resuspended in TNE for uses in ELISA and Western blotting.

### F. Immunological Techniques

IBDV, HCV, PRRSV and the prestained protein markers (board range, Bio-Rad) were mixed with 6X loading buffer (30 mM Tris-Cl, pH 6.8, 30% glycerol, 10% SDS, 600 mM Dithiothreitol, 0.0 12% bromophenol blue) and denatured in boiling water bath for 10 minutes. The denatured viral proteins were then resolved by sodium dodecyl sulfate-polyacrylamide gel electrophoresis (SDS-PAGE) with a vertical electrophoresis unit (Hoefer Scientifc Instruments). The sample were first concentrated in 5% stacking gel and then resolved in 12% separating gel. Electrophoresis was performed in protein running buffer at 80 V for 2.5 hours. The gel was stained with coomassie blue or used for the Western Blot.

### 1. Western blotting

The resolved protein bands on the SDS-PAGE gel, the bands were transferred to absolute methanol-soaked protein membrane (Immun-Blot PVDF membrane, Bio-Rad) in the vertical unit with transfer buffer at a current of 40 mA overnight.

The membrane was then rinsed with PBS and blocked in blocking agent (5% non-fat milk and 1% Tween-20 in IX PBS) at room temperature (RT) for 15 minutes with shaking.

The blocking agent was removed and the mice anti-sera, diluted in blocking reagent, was added and incubated at RT for 2 hours with shaking. The membrane was then washed with PBS five times at 5 minutes each. After then, alkaline phosphatase (AP) conjugated goat anti-mouse IgG (Zymed, 1:50), which acted as secondary antibodies, was added to the membrane and incubated at RT for 1.5 hours. The membrane was rinsed with PBS as above. 10% 5-Bromo-4-chloro-3-indo[y] phosphate (BCIP) and 10% Nitroblue tetrazolium salt (NBT) were finally added as a substrate. Colour was allowed to be developed in the dark overnight.

### 2. Enzyme-linked immunosorbent assay (ELISA)

The ELISA assay was modified to measure IBCV-, HCV-and PRRSV-specific antibody in vaccinated mice. Purified IBDV, HCV or PRRSV was firstly diluted at 1:100 using coating buffer, PBSN (15 mM Na2CO3, 35 mM NaHCO3 and 0.05% NaN3, pH 9.6*in 1X PBS). 100 iii of diluted virus was added to each well of a 96-wells ELISA plate at 4°C overnight. The plate was washed with PBST (0.05% Tween-20 IN 1X PBS) three times at 5 minutes each. The non-specific binding sites were then block by PBS with N Bovine Serum Albumin (BSA, USB Inc.) at 37°C for 2 hours. 100 µl diluted mouse serum (1:50) was added to the antigen-coated wells in duplication as primary antibody and incubated at 37°C for 1 hour. The plate was then washed with PBS as above. After then, HRP-conjugated goat anti-mouse IgG (Zymed, 1:5000) was added as secondary antibody and incubated at 37°C for 1 hour. The plate was then washed with PBST as above. 100 µl substrate TMB (Zymed) was added to each well and allowed to react in dark for 15 minutes. 100 p.1 stop solution (IN HCI) was finally added to stop the reaction. OD reading of ELISA plate was measured at 490 nm with microplate reader (Bio-Rad Model 550).

### EXAMPLE 3

### ELISA and Western blotting performance

During the whole immunization scheme (Example 2), three of the mice died after vaccination or blood collection. This included one mouse from group 4 and one mouse from group 7 in demonstration 1 and one mouse from group 2 in demonstration 2.

### A. ELISA

In order to assess the ability of the DNA construct to elicit specific antibodies in animal model (i.e., BALE/c mice), ELISA and Western blotting were performed.

ELISA was performed to investigate if there was any humoral immune response in the vaccinated mice. Specific anti-IBDV, anti-HCV or anti-PRRSV antibodies in the mice sera were tested by the modified ELISE in duplicate.

The results are shown from Table 6 to Table 26 on subsequent pages. They were summarized in the following table:

| **Table 6** | | | | |
|---|---|---|---|---|
| **The humoral immune response of mice from groups 3 and 4 after vaccination.** | | | | |
| | | | | |

| | **Demonstration 1** | | **Demonstration 2** | |
|---|---|---|---|---|
| | **# of mice producing specific antibodies** | **Total number of mice** | **# of mice producing specific antibodies** | **Total number of mice** |
| Group 1 (IBD vaccine) | 1 | 5 | 3 | 5 |
| Group 2 (IBD vaccine) | 3 | 5 | 4 | 4 |
| Group 3 (HC vaccine) | 3 | 5 | 2 | 5 |
| Group 4 (HC vaccine) | 3 | 4 | 3 | 5 |
| Group 5 (PRRS vaccine) | 3 | 5 | 3 | 5 |
| Group 6 (HC vaccine) | 5 | 5 | 2 | 5 |
| Group 6 (PRRS vaccine) | 3 | 5 | 2 | 5 |

Although most of the mice sera showed a slight decrease in ELISA reading in the last time when compared to that in the third time, the reading was still higher than the initial value. Hence, the DNA vaccine applied on them was proven to be effective.

Transient increase occurred when the ELISA reading of a particular mouse increased after the first and/or the second vaccination. However, the reading dropped to a level similar to that in Day 0 (pre-treatment) afterwards.

Among the 10 mice injected with pcDNA3.1-VP5-5.2 &VP2-3.4 (group 1), mouse 4 from demonstration I (Table 7) and mice 1, 3 and 5 from demonstration 2 (Table 8) showed an increase in IBDV-specific antibody.

| **Table 7** | | | | | |
|---|---|---|---|---|---|
| **IBDV specific ELISA reading of group 1 vaccinated mice** | | | | | |
| **Group number** | **Code of mice** | **ELISA reading** | | | |
| | | Day 0 | Day 7 | Day 15 | Day 28 |
| 1 | 1 | 0.189 | 0.149 | 0.209 | 0.236 |
| | 2 | 0.261 | 0.171 | 0.189 | 0.281 |
| | 3 | 0.252 | 0.245 | 0.176 | 0.186 |
| | 4 | 0.137 | 0.248 | 0.358 | 0.590 |
| | 5 | 0.158 | 0.235 | 0.205 | 0.252 |

| **Table 8** | | | | | |
|---|---|---|---|---|---|
| **IBDV specific ELISA reading of group 1 vaccinated mice** | | | | | |
| **Group number** | **Code of mice** | **ELISA reading** | | | |
| | | Day 0 | Day 7 | Day 15 | Day 28 |
| | | | | | |
| 1 | 1 | 0.416 | 0.416 | 0.574 | 0.654 |
| | 2 | 0.535 | 0.505 | 0.638 | 0.549 |
| | 3 | 0.166 | 0.256 | 0.319 | 0.314 |
| | 4 | 0.251 | 0.209 | 0.337 | 0.291 |
| | 5 | 0.224 | 0.335 | 0.379 | 0.368 |

Among the 9 mice fed with *E*. *coli* cells containing 100 jg pcDNA3.1-VP5-5.2 & VP2-3.4 (group 2), mice 1, 2 and 3 from demonstration 1(Table 9) and all mice from demonstration 2 (Table 10) showed an increase of IBDV-specific antibody after vaccinating orally.

| **Table 9** | | | | | |
|---|---|---|---|---|---|
| **IBDV specific ELISA reading of group 2 vaccinated mice** | | | | | |
| **Group number** | **Code of mice** | **ELISA reading** | | | |
| | | Day 0 | Day 7 | Day 15 | Day 28 |
| 2 | 1 | 0.420 | 0.626 | 0.654 | 0.638 |
| | 2 | 0.389 | 0.530 | 0.660 | 0.714 |
| | 3 | 0.378 | 0.526 | 0.659 | 0.579 |
| | 4 | 0.614 | 0.495 | 0.547 | 0.601 |
| | 5 | 0.638 | 0.593 | 0.628 | 0.600 |

| **Table 10** | | | | | |
|---|---|---|---|---|---|
| **IBDV specific ELISA reading of group 2 vaccinated mice** | | | | | |
| **Group number** | **Code of mice** | **ELISA reading** | | | |
| | | Day 0 | Day 7 | Day 15 | Day 28 |
| | | | | | |
| 2 | 1 | 0.379 | 0.492 | 0.294 | 0.836 |
| | 2 | 0.434 | 0.640 | 0.569 | 0.885 |
| | 3 | 0.472 | 0.277 | 0.527 | 0.626 |
| | 4 | 0.406 | 0.382 | 0.410 | 0.747 |
| | 5* | 0.440 | 0.464 | / | / |

| | | | | | |
|---|---|---|---|---|---|
| *Mouse number 5 was dead after collecting blood at day 7 | | | | | |

Among the 10 mice injected with pHCV2.5 (group 3), mice 2,3 and 4 from demonstration I (Table 11) showed an increase in ELISA reading, Meanwhile, mice 4 and 5 from demonstration 2 (Table 12) showed transient increase of HCV-specific antibody.

| **Table 11** | | | | | |
|---|---|---|---|---|---|
| **HCV specific ELISA reading of group 3 vaccinated mice** | | | | | |
| **Group number** | **Code of mice** | **ELISA reading** | | | |
| | | Day 0 | Day 7 | Day 15 | Day 28 |
| 3 | 1 | 0.837 | 0.740 | 0.916 | 0.792 |
| | 2 | 0.326 | 0.555 | 0.858 | 0.661 |
| | 3 | 0.404 | 0.993 | 1.102 | 0.934 |
| | 4 | 0.727 | 0.712 | 0.956 | 0.749 |
| | 5 | 0.684 | 0.846 | 0.758 | 0.518 |

| **Table 12** | | | | | |
|---|---|---|---|---|---|
| **HCV specific ELISA reading of group 3 vaccinated mice** | | | | | |
| **Group number** | **Code of mice** | **ELISA reading** | | | |
| | | Day 0 | Day 7 | Day 15 | Day 28 |
| 3 | 1 | 0.772 | 0.621 | 0.484 | 0.579 |
| | 2 | 0.690 | 0.527 | 0.482 | 0.741 |
| | 3 | 0.659 | 0.513 | 0.549 | 0.673 |
| | 4 | 0.746 | 0.543 | 0.827 | 0.627 |
| | 5 | 0.406 | 0.598 | 0.480 | 0.529 |

Among the 9 mice fed with E. coli cells containing 100 gg pHCV2.5 (group 4), mice 1, 2 and 5 from demonstration 1 (Table 13) and mice 1, 3 and 5 from demonstration 2 (Table 14) showed an increase of HCV-specific antibody, though mouse 5 from the demonstration 2 only showed a transient increase.

| **Table 13** | | | | | |
|---|---|---|---|---|---|
| **IBDV specific ELISA reading of group 4 vaccinated mice** | | | | | |
| **Group number** | **Code of mice** | **ELISA reading** | | | |
| | | Day 0 | Day 7 | Day 15 | Day 28 |
| 4 | 1 | 0.743 | 0.899 | 0.989 | 0.948 |
| | 2 | 0.809 | 0.936 | 0.965 | 1.097 |
| | 3* | / | / | / | / |
| | 4 | 1.002 | 1.135 | 1.102 | 1.115 |
| | 5 | 0.785 | 0.853 | 0.991 | 1.009 |

| | | | | | |
|---|---|---|---|---|---|
| *Mouse number 3 was dead before the start of the vaccination scheme. | | | | | |

| **Table 14** | | | | | |
|---|---|---|---|---|---|
| **HCV specific ELISA reading of group 4 vaccinated mice** | | | | | |
| **Group number** | **Code of mice** | **ELISA reading** | | | |
| | | Day 0 | Day 7 | Day 15 | Day 28 |
| 5 | 1 | 0.660 | 0.802 | 0.644 | 0.869 |
| | 2 | 0.924 | 0.887 | 0.801 | 0.711 |
| | 3 | 0.523 | 1.088 | 0.910 | 0.790 |
| | 4 | 0.849 | 0.870 | 0.735 | 0.880 |
| | 5 | 0.648 | 0.981 | 0.855 | 0.713 |

Among the 10 mice injected with pcDNA3. I-ORF5 (group 5), mice 3, 4•and 5 from demonstration 1 (Table 15) and mice 1, 2 and 5 from demonstration 2 (Table. 16) showed an increase in PRRSV-specific antibody level after vaccination.

| **Table 15** | | | | | |
|---|---|---|---|---|---|
| **PRRSV specific ELISA reading of group 5 vaccinated mice** | | | | | |
| **Group number** | **Code of mice** | **ELISA reading** | | | |
| | | Day 0 | Day 7 | Day 15 | Day 28 |
| 5 | 1 | 0.513 | 0.424 | 0.390 | 0.419 |
| | 2 | 0.483 | 0.358 | 0.681 | 0.500 |
| | 3 | 0.278 | 0.425 | 0.572 | 0.514 |
| | 4 | 0.220 | 0.451 | 0.525 | 0.433 |
| | 5 | 0.396 | 0.473 | 0.749 | 0.551 |

| **Table 16** | | | | | |
|---|---|---|---|---|---|
| **PRRSV specific ELISA reading of group 5 vaccinated mice** | | | | | |
| **Group number** | **Code of mice** | **ELISA reading** | | | |
| | | Day 0 | Day 7 | Day 15 | Day 28 |
| 5 | 1 | 0.431 | 0.354 | 0.326 | 0.525 |
| | 2 | 0.300 | 0.331 | 0.347 | 0.466 |
| | 3 | 0.371 | 0.245 | 0.425 | 04.62 |
| | 4 | 0.253 | 0.353 | 0.319 | 0.282 |
| | 5 | 0.285 | 0.316 | 0.404 | 0.701 |

Among the 10 mice injected with combined vaccine of pHCV2.5 and pcDNA3.1-ORFS (group 6), all mice from demonstration 1 (Table 17) and mice 4 and 5 from demonstration 2 (Table 18) showed an increase of HCV-specific antibody. In the meantime, mice 3, 4 and 5 from demonstration 1 (Table 19) and mice 2 and 4 from demonstration 2 (Table 20) showed an increase of PRRSV-specific antibody.

| **Table 17** | | | | | |
|---|---|---|---|---|---|
| **HCV specific ELISA reading of group 6 vaccinated mice** | | | | | |
| **Group number** | **Code of mice** | **ELISA reading** | | | |
| | | Day 0 | Day 7 | Day 15 | Day 28 |
| 6 | 1 | 0.304 | 0.915 | 0.755 | 1.000 |
| | 2 | 0.340 | 0.939 | 0.851 | 0.611 |
| | 3 | 0.267 | 0.814 | 0.922 | 0.737 |
| | 4 | 0.197 | 0.601 | 0.764 | 0.688 |
| | 5 | 0.424 | 0.951 | 0.932 | 0.848 |

| **Table 18** | | | | | |
|---|---|---|---|---|---|
| **HCV specific ELISA reading of group 6 vaccinated mice** | | | | | |
| **Group number** | **Code of mice** | **ELISA reading** | | | |
| | | Day 0 | Day 7 | Day 15 | Day 28 |
| 6 | 1 | 0.711 | 0.621 | 0.659 | 0.741 |
| | 2 | 0.733 | 0.553 | 0.716 | 0.737 |
| | 3 | 0.897 | 0.453 | 0.834 | 0.831 |
| | 4 | 0.862 | 0.556 | 0.729 | 0.999 |
| | 5 | 0.626 | 0.441 | 0.819 | 1.082 |

| **Table 19** | | | | | |
|---|---|---|---|---|---|
| **PRRSV specific ELISA reading of group 6 vaccinated mice** | | | | | |
| **Group number** | **Code of mice** | **ELISA reading** | | | |
| | | Day 0 | Day 7 | Day 15 | Day 28 |
| 6 | 1 | 0.380 | 0.420 | 0.395 | 0.380 |
| | 2 | 0.355 | 0.390 | 0.525 | 0.405 |
| | 3 | 0.395 | 0.365 | 0.470 | 0.760 |
| | 4 | 0.455 | 0.365 | 0.370 | 0.720 |
| | 5 | 0.510 | 0.350 | 0.365 | 0.685 |

| **Table 20** | | | | | |
|---|---|---|---|---|---|
| **PRRSV specific ELISA reading of group 6 vaccinated mice** | | | | | |
| **Group number** | **Code of mice** | **ELISA reading** | | | |
| | | Day 0 | Day 7 | Day 15 | Day 28 |
| 6 | 1 | 0.460 | 0.595 | 0.385 | 0.425 |
| | 2 | 0.390 | 0.430 | 0.375 | 0.855 |
| | 3 | 0.450 | 0.430 | 0.385 | 0.380 |
| | 4 | 0.415 | 0.440 | 0.565 | 0.400 |
| | 5 | 0.435 | 0.370 | 0.365 | 0.385 |

None of the mice in the negative control groups (Table 21 to Table 26) showed any increase in the anti-HCV or anti-PRRSV antibodies levels.

| **Table 21** | | | | | |
|---|---|---|---|---|---|
| **IBDV specific ELISA reading of mice in the control group** | | | | | |
| **Group number** | **Code of mice** | **ELISA reading** | | | |
| | | Day 0 | Day 7 | Day 15 | Day 28 |
| Control group* | 1 | 0.434 | 0.445 | 0.319 | 0.254 |
| | 2 | 0.389 | 0.422 | 0.308 | 0.260 |
| | 3 | 0.284 | 0.320 | 0.239 | 0.222 |
| | 4 | 0.252 | 0.312 | 0.302 | 0.202 |

| | | | | | |
|---|---|---|---|---|---|
| *Mouse number 5 was dead before the start of the vaccination scheme | | | | | |

| **Table 22** | | | | | |
|---|---|---|---|---|---|
| **IBDV specific ELISA reading of mice in the control group** | | | | | |
| **Group number** | **Code of mice** | **ELISA reading** | | | |
| Control number | Code of mice | Day 0 | Day 7 | Day 15 | Day 28 |
| | 2 | 0.417 | 0.417 | 0.316 | 0.284 |
| | 3 | 0.266 | 0.282 | 0.353 | 0.205 |
| | 4 | 0.333 | 0.254 | 0.206 | 0.222 |
| | 5 | 0.242 | 0.328 | 0.247 | 0.212 |

| **Table 23** | | | | | |
|---|---|---|---|---|---|
| **HCV specific ELISA reading of mice in the control group** | | | | | |
| **Group number** | **Code of mice** | **ELISA reading** | | | |
| | | Day 0 | Day 7 | Day 15 | Day 28 |
| **Control group*** | 1 | 0.686 | 0.626 | 0.549 | 0.515 |
| | 2 | 0.582 | 0.593 | 0.604 | 0.568 |
| | 3 | 0.533 | 0.530 | 0.453 | 0.489 |
| | 4 | 0.500 | 0.593 | 0.586 | 0.507 |

| | | | | | |
|---|---|---|---|---|---|
| *Mouse number 5 was dead before the start of the vaccination scheme | | | | | |

| **Table 24** | | | | | |
|---|---|---|---|---|---|
| **HCV specific ELISA reading of mice in the control group** | | | | | |
| **Group number** | | **ELISA reading** | | | |
| | | Day 0 | Day 7 | Day 15 | Day 28 |
| | 1 | 0.647 | 0.480 | 0.650 | 0.599 |
| | 2 | 0.844 | 0.904 | 0.609 | 0.543 |
| | 3 | 0.572 | 0.519 | 0.500 | 0.435 |
| | 4 | 0.772 | 0.710 | 0.782 | 0.560 |
| | 5 | 0.508 | 0.625 | 0.587 | 0.461 |

| **Table 25** | | | | | |
|---|---|---|---|---|---|
| **PRRSV specific ELISA reading of mice in the control group** | | | | | |
| **Group number** | **Code of mice** | **ELISA reading** | | | |
| | | Day 0 | Day 7 | Day 15 | Day 28 |
| Control group* | 1 | 0.243 | 0.242 | 0.180 | 0.203 |
| | 2 | 0.152 | 0.235 | 0.203 | 0.230 |
| | 3 | 0.185 | 0.201 | 0.169 | 0.204 |
| | 4 | 0.188 | 0.253 | 0.205 | 0.202 |

| | | | | | |
|---|---|---|---|---|---|
| *Mouse number 5 was dead before the start of the vaccination scheme | | | | | |

| **Table 26** | | | | | |
|---|---|---|---|---|---|
| **PRRSV specific ELISA reading of mice in the control group** | | | | | |
| **Group number** | **Code of mice** | **ELISA reading** | | | |
| | | Day 0 | Day 7 | Day 15 | Day 28 |
| Control Group | 1 | 0.218 | 0.197 | 0.233 | 0.235 |
| | 2 | 0.278 | 0.358 | 0.196 | 0.159 |
| | 3 | 0.218 | 0.249 | 0.289 | 0.169 |
| | 4 | 0.271 | 0.171 | 0.193 | 0.178 |
| | 5 | 0.217 | 0.234 | 0.241 | 0.209 |

### B. Western Blotting

Western blot analysis showed whether the viral envelope protein has been expressed. Moreover, it also demonstrated the specificity of mice-antisera to the viral protein.

Since the viral proteins were heat-treated at 100°C for 10 minutes and resolved in the SDS-PAGE, so the viral protein were denatured. Therefore, only linear epitopes, but not conformation dependent epitopes, could be detected on the blotting membrane.

All the mice sera (collected on day 28) in a group were pooled together and diluted with blocking agent for the Western Blot analysis.

All mice sera in demonstrations 1 and 2 showed positive result in the Western blot analysis, although there was variation in intensity of the bands.

There was no banding pattern for control mice sera pooled from demonstration 1 or pooled from demonstration 2 against IBDV, HCV and PRRSV protein. In the other words, there were no specific anti-IBDV, ant-HCV or anti-PRRSV antibodies produced.

In Western blot of group 1 and 2 mice sera, wherein pooled sera from demonstration 1 and pooled sera from demonstration 2 were tested, there was a band between 36.4 kD and 486 kD. It corresponded to VP 2 of IBDV. The viral proteins were run in duplicate for each test.

In Western blot of group 3 mice sera, there were two bands between 48.6 kD and 96 kD. The viral proteins were run in duplicate for each test. One band was closer to 48.6 kD in the pooled mice anti-sera from demonstration 1 and represented the E2 protein of HCV (55 kD). There was an additional band between 29.8 kD and 36.4 kD. The band corresponded to E 1 of HCV (33 kD). Meanwhile, one band was closer to 94 kD in the pooled mice anti-sera from demonstration 2 which corresponded to E1-E2 heterodimer of HCV (75 kD). Glycoprotein E0 has been described as a 44 kD to 48 kD protein and was observed slightly lower than 48.6 kD on the blotting membrane.

The result of Western blot of group 4 mice sera was similar to that in group 3.

In case of group 5 mice sera, there was a band just below 29.8 kD. It corresponded to GP5 of PRRSV. In the demonstration 1 test, the strong band was between 29.8 kD and 36.4 kD. Although typical GP5 was 24.5 kD - 26 kD, this strong band should represent GP5 since pcDNA3.1-ORF5 was injected. The difference may due to incomplete denaturation of the protein and resolving ability'of the polyacrylamide gel.

The Western blot of group 6 mice sera showed, on the lane with HCV glycoprotein in the test of the demonstration I anti-sera, a band just higher than 94 kD that represented E2-E2 homodimer of HCV (100 kD). Glycoprotein EO (44 kD to 48 kD) was observed slightly lower than 48.6 kD on the blotting membrane. Moreover, there was a band between 29.8 kD and 36.4 kD. The band corresponded to E1 of HCV (33 kD). In the test of the demonstration 2 antisera, on the lane with HCV glycoprotein, there was a band close to 94 kD. It corresponded to E1-E2 heterodimer of HCV (75 kD).

On the lane with PRRSV glycoprotein, there was a band just below 29.8 kD. It corresponded to GP5 of PRRSV in both the demonstration I and demonstration 2 anti-sera tests. Above the GP5 band, there were several bands. They may be the incomplete denatured GP5 or the disulfde-linked M & GP5 heterodimer.

### C. Results

In the modified ELISA analysis, although ELISA plates have been blocked by blocking agent to avoid non-specific binding, non-specific background staining was still in some samples wells. For example, in group 3 mice in demonstration 1, initial reading of number 1, 4 and 5 showed a deviated value from that of number 2 and 3 which was run on another plate. Hence, the tread of ELISA reading over time has also been taken into consideration for the evaluation of specific antibodies production.

In ELISA analysis, 5 out of 10 mice injected with pcDNA3.I-VP5-5.2&VP2-3.4 have shown to produce specific antibodies against IBDV. In the same time, 7 out of 9 mice fed with *E. coli* containing pcDNA3. 1-VP5-5.2&VP2-3.4 have shown to produce specific antibodies against IBDV.

Five out of ten mice injected with pHCV2.5 have shown to produce specific antibodies against HCV. In the same time, 6 out of 9 mice fed with *E. coli* containing pHCV2.5 have shown to produce specific antibodies against HCV.

The above results indicate that the DNA vaccine administered through the two routes (intramuscular injection and feeding) was effective in triggering specific antibodies against the virus. The result was further confirmed by Western blot analysis.

In ELISA analysis, 5 out of 10 mice injected with pHCV2.5 have shown to produce specific antibodies against HCV. In the same time, 6 out of 10 mice injected with pcDNA3.1-ORF5 have shown to produce specific antibodies against PRRSV.

In group 6, the mice were injected with combined DNA vaccine (pHCV2.5 and pcDNA3,1-ORF5), 7 out of 10 mice have shown to produce specific antibodies against HCV. In the same time, 5 out of 10 mice have shown to produce specific antibodies against PRRSV. In addition, 4 mice in this group have shown to produce specific antibodies against HCV and PRRSV simultaneously.

The above results indicate that the combined DNA vaccine administered through intramuscular injection was effective in triggering specific antibodies against both viruses (HCV and PRRSV). The result was further confirmed by Western blot analysis.

The antibody reaction against each virus using a combined immunization was rather equivalent in quality and intensity to that obtained with a single immunization, considering the mouse to mouse variability. This indicates that the mouse immune system could accommodate multiple antigens and open the way for multipotent DNA vaccine preparations for pigs.

There are at least three conformation dependent and one linear (conformation independent) neutralizing epitopes in IBDV. Two conformation dependent neutralizing epitopes located in the central region of VP2 while the linear neutralizing epitope was located in the C-terminal of VP3 (Yamagucki, T., et at., 1996, Virol 223:219-223). Although Western blot can only detect linear antigen, VP2 was detected on the membrane. It may due to the incomplete denaturation of VP2 that made it detectable.

In the Western Blot analysis, homodimer and heterodimer of HCV has been detected. Dimerization of HCV protein maybe important for authentic antigen presentation to the host's immune system and the induction of a stable, long-term immunity (Konig, M et al., 1995, J Virol 69:5479-86). In addition, since the viral proteins were separated in non-denaturing condition, E1-E2 heterodimer of HCV that was linked by intermolecular disulfide bridges could be detected. Intramolecular disulfide bonds coated in the N-terminal half of E2 have been shown to be important for antigen recognition by specific antibodies. In order to demonstrate monomeric E1 and E2, the proteins should be separated in reducing condition (e.g. in the presence of 2-mecaptoethanol).

Three different forms of glycoproteins E2 were found in HCV, they were E2 monomers, E2 homodimers and E1-E2 heterodimers. E1-E2 heterodimer was preferentially formed after introducing pHCV2.5 that expressed both E1 and R2. The appearance of doublets or triplet of these forms may be due to different E2 protein backbones and to different glycosylation. Moreover, alternative processing at the carboxy terminus generated E2 molecules with different apparent molecular weights.

In Western blot analysis of PR RSV-specific antibodies, there were a number of bands. They may due to the following reasons. Firstly, the PRRSV protein was beat-treated at 100°C for 10 minutes before loading onto the SDS-PAGE. Hence, three types of GPS may appear on the gel, i. Totally denatured, ii. Partially denatured, iii. Partially degraded.

Secondly, at around 26.4 kD and 48.6 lcD, there was a clear band that may correspond to the disulfide-linked M-GP5 heterodimer. Cysteine residues located at the N-terminal ectodomains of both envelope proteins were probably involved in the formation of an intermolecular disulfide bridge. Hence, the presence of anti-GP5 antibody could detect its presence.

There were at least two types of neutralizing antigenic determinants associated with the GP5 of PRRSV. Among these determinants, some were conformation dependence and some were linear. Therefore, Western blot analysis could still detect the presence of anti-GP5. In addition, it appeared that glycosylation was not necessarily associated with the neutralizing epitopes.

### EXAMPLE 4

### Modifications to Vaccine Compositions and Methods

It is contemplated that certain modifications to the vaccine compositions and methods demonstrated herein may provide additional aspects of the present invention.

Firstly, it is contemplated that a counterpart to each group should be included for comparison. For example, besides feeding mice with E. coil containing the plasmid (potential DNA vaccine), an additional group of mice should be fed with naked plasmid DNA. Besides injecting mice with naked plasmid, an additional group of mice should be included and injected with E. coli containing the plasmid. Moreover, instead of injecting or feeding nothing to the mice in the control group, they could be injected with PBS or vector (pcDNA3.1) alongside the vaccination scheme.

Secondly, it is contemplated that the production of IgA and cytokine could be confirmed by analyzing frozen small intestinal tissue sections of the vaccinated mice containing the most Peryer's patches. It has been said that oral immunization can elicit the production of both systemic and mucosal antibodies (Gallichan WS, Rosenthal KL. 1995. Vaccine. 13:1589-1595).

Thirdly, although the DNA vaccine (pcDNA3.1-VP505.2&VP2-3.4, pHCV2.5 and pcDNA3.1-ORF5) can elicit the production of specific antibodies, as previously demonstrated, it is contemplated that a DNA vaccine may be able to protect the animal against the disease. To further demonstrate the efficacy of the DNA vaccine, the neutralizing ability of the specific antibodies can also be tested by virus neutralization assay.

Fourthly, it has been said that immunization with DNA vaccine can elicit both humoral and cellular immunity (Ulmer, J.B., et al., 1996, Immunol 89: 59-67) Ulmer, J.B., et al. Cur Opin Immunol 8:531-536). However, only the humoral immune response was measured after each vaccination. It is contemplated that T lymphocytes, which play an important role in fighting against the virus, may be stimulated by the vaccine and compositions of the present invention to produce an immune response. It is further contemplated that the quantity and the role of antigen specific cytototic and helper T lymphocytes in fighting against a disease, after vaccination using the compositions and methods of the present invention, may be demonstrated by flow cytometric analysis.

Fifthly, it is contemplated that an anesthetized animal may be easier to vaccinate using the compositions and methods of the present invention. For example, during injecting mice with DNA vaccine, a vaccinated animal may contract their muscles and squeeze the vaccine solution (e.g., a DNA vaccine) out when they were awake.

Sixthly, it is contemplated that in order to enhance or boost the immune response of an animal (e.g. mice), one or more additional agents may be used in combination with any of the vaccine methods and compositions described herein, Such agents may include, but are not limited to, one or more chemical (e.g., complete Freund's adjuvant) or genetic (e.g., vector expressing cytokines) adjuvants. In an non-limiting example, co-inoculation of both an immunogenic DNA vaccine (e,g. a plasmid) and a genetic adjuvant (e.g. another plasmid) may result in an augmentation of an antigen specific humoral and/or cell mediated immune response.

### EXAMPLE 5

### Demonstration of Isolation and Characterization of a Porcine Pathogenic Virus

Described herein this example is methods for identifying, isolating and characterization of a pathogen, specifically Guangzhou porcine reproductive and respiratory virus. Specifically described is a method to detect the presence of porcine reproductive and respiratory syndrome virus (PRRSV) in tissue samples of infected pigs, based on reverse transcription of the viral RNA coupled to DNA amplification by polymerase chain reaction (RT-PCR). Tissue samples (lung, muscles) were collected from infected pigs from different pig farms in Guangzhou. AV Guangzhou field isolates described herein have a close similarity with virions of North American strain through the RT-PCR amplification, sequencing analysis, and Western immunoblotting analysis. As would be understood by one of skill in the art, such methods may be applied in identifying, isolating and characterizing other pathogens in the practice of the present invention.

### Identification of PRRSV as a New Pathogen

In the late 1980's, a mysterious disease broke out in pig farms throughout the world, characterized by severe reproductive failure in sows of any parity and respiratory problems for pigs of all ages (Mardassi et al., Can J Yet Res 58, 55-64; Mardassi et al., 1994. J Clinical Mcrobiology. Sept. 2197-2203). The disease was first reported in North American in 1987, and the etiological agent was first isolated in 1990 in Europe and then in 1992 in the USA (Suarez et al., 1994 Arch Virol. 135, 89-99). Both strains were structurally related but antigenically distinct. The disease was finally given the name Porcine Reproductive and Respiratory Syndrome disease, caused by a fastidious virus-Porcine Reproductive and Respiratory Syndrome Virus (PRRSV).

### Characterization of PRRSV

Molecular characterizations have shown that the viral genome consists of a positive single-stranded RNA molecule with an approximate genome size of 15 kb. The genome contains 8 open reading frames (ORFs) with ORFIa and ORFIb (at the 5' end) representing nearly 75% of the viral genome. It encodes for the proteins with the function of polymerase and replicase activities. The other six structural proteins, ORFs2 to 7, are located at the 3' end of the genome (Conzelmann, K.K. et al., 1993, Viral 193: 329-339). The spherical, envelope PRRSV has been described with morphological and morphogenetical similarity to members of the arteriviius group, including *equine arteritis virus* (EAV) and *lactafe dehydrogenase-elevating virus* of mice (LDV). Virus replication has failed in many different kinds of primary and established cell lines, but it does replicate in Porcine Alveolar Macrophages (PAM) and MARC-145 derived from MA-104 monkey cell (Dea et al., Ultrastructural characteristics and morphogenesis of Porcine Reproductive and Respiratory Syndrome Vrus propagated in the highly permissive Marc-145 cell clone. Plenum Press, New York 1995).

### Isolation and Characterization of the Sub-Strains of PRRSV

In brief, multiplex PCR utilizing different sets of oligonucleotide primers was used to differentiate between North American and European isolates among the field isolated samples collected from Guangzhou. Using purifed PRRS virion from the infected cell cultures as the starting material, molecular cDNA cloning and sequencing was also performed. The morphological and physicochemical properties of the field isolate of PRRSV were characterized as well.

### 1. Cells and virus isolation

A total of 3 Guangzhou field isolates of PRRSV were recovered from clarifed lung tissues and muscles of dyspneic pigs and were propagated on MARC-145, a PRRSV permissive cell line (Department of Veterinary Medicine, South China Agricultural University, Guangzhou, 510642, China). The MARC-145 cells were cultivated in Minimum Essential Medium (MEM) supplemented with 10% gamma-irradiated fetal bovine serum (FBS) and 10% Tryptose Phosphate Broth (TPB) and 1% antibiotic-Antimycotic (Life Technologies, GIBCOBRL®). The representative US isolate of PRRSV, NVSL, was isolated from commercially available modified-live vaccine (Animal and Plant Health Inspection Services, National Veterinary Services Laboratories. Ames, IA). Aliquots of 1.5 ml of clarifed tissue samples were inoculated into the cell monolayer of the 75 cm2 culture fask with culture medium containing no fetal bovine serum (FBS). Infected cell culture was monitored daily for the appearances of cytopathic effect (CPE).

For all tissue sample homogenates, no CPE was observed during the first six passages in the MARC-145 cell culture. CPE was observed after the seventh passage beginning with the characteristics of rounding off; cell enlargement and rounded cells with many vacuoles appearing after 24 hours postinoculation with the AV field isolates of PRRSV; syncytia of the infected cells was observed; the infected cells started to aggregate after 3-4 days postinoculation. Finally the infected cell sloughed off from the flask. These CPE was also seen on positive control NVSL infected cells throughout the experiment.

### 2. Virus purification

Stock viruses were produced by at least seven successive passages in MARC- 145 cells. The viruses were harvested by freezing and thawing the infected cells three times at-80°C and 37°C respectively. The cellular debris was removed by centrifagation at 4000 x g for 20 min at 4°C. The extracellular virus in the clarified supernatant fluids was first pelleted for 3 hours at 75,000 x g (21,000 rpm) in a Beckman SW40Ti rotor. The pellet was resuspended in 11100 original volume of THE buffer. Concentrated virus was then purified through a 30%-50% (w/v) sucrose gradient in a Beckman SW55Ti rotor at 110,000 x g (35,000 rpm) for 16 hours.

### 3. RNA preparation

Viral RNA was extracted directly from 500 µl of the supernatant from virus infected MARC-145 cells by using 500 pi TRIZOL reagent (Life Technologies INC.,) and following manufacturer's protocol,

### 4. cDNA synthesis

cDNA synthesis was done according to the Superscript™ Preamplification System for First Strand cDNA Synthesis (Life Technologies, GIBCO BRL).

### 5. Primer Design

Several sets of primers were designed based on both North American (VR-2332) and European (LV) genome sequences within ORFIb encoding the polymerase protein in order to perform a rapid multiplex PCR assay. PCR primers were designed on the basis of ORFIb due to their conservation among arteriviruses. One set of internal primers was designed from Leiystad virus (LV) genome sequences, while the other set were type-specific internal primers and type-common primers for multiplex or nested multiplex PCR. Primers were designed after sequencing a portion of ORFIb from two North American strains of PRRS virus: Minnesota MNIb and Quebec LHVA-93.3 isolates (Gilbert el al., 1997, J. Clinical Mcrobiology Jan 35(1), 264-267). Details of the oligonucleotide primers for nested multiplex PCR were shown in Table 27.

| **Table 27** | | |
|---|---|---|
| **Oligonucleotide primers for PCR amplification and typing of PRRSV** | | |
| **Primer Sequence (5' to 3')** | **Size of PCR product (bp)** | **Type Detected** |
| **Nested multiplex primers** | | |
| U1 GTATGAACTTGCAGGATG (SEQ ID NO:17) | 186 | European |
| D1 GCCGACAATACCATGTGCTG (SEQ ID NO:18) | | |
| U2 GGCGCAGTGACTAAGAGA (SEQ ID NO:19) | 107 | North American |
| D2 GTAACTGAACACCATATGCTG (SEQ ID NO:20) | | |

| **External primers** | | |
|---|---|---|
| EU CCTCCTGTATGAACTTGC (SEQ ID NO:21) | 255 | Common |
| ED AGGTCCTCGAACTTGAGCTG (SEQ ID NO:22) | | |

### 6. Nested multiplex PCR for PRRSV typing of samples D2, D3 and AV

For the first round of PCR, 2.5 µl of cDNA was added into the mixture containing 10 x PCR buffer, 25 mM MgC12; 10 mM dNTPs mix, .12.5 µM primers EU and ED and 5 units of Taq DNA polymerase (Life Technologies, GiBCRO, BRL) in a total volume of 25 pl. After denaturation at 94°C for 3 min, the reactions were cycled 4 times at 94 °C for 20 sec, 42°C for 1 min, and 72°C for 1 min; and then 40 times at 94°C for 20 sec, 47°C for 1 min, and 72°C for 1 min, with a final extension step of 72°C for 5 min.

For nested PCR, 2.5 pl of the template (PCR product in first round PCR) was added into a reaction mixture containing 12.5 pM primers U1 and D1, and 12.5 pM primers U2 And D2. After denaturation at 94C for 3 min, the reactions were cycled 35 times at 94°C for 20 sec, 47C for 30 sec and 72°C for 30 sec with a final extension step of 72C for 15 min. The amplified products were detected by electrophoresing 5 pl aliquots through 1.5% agarose gel and stained with ethidium bromide, the gels were photographed under UV illumination.

No amplification was observed for the European strains, thus confirming the specificity of the internal primer pair U2 and D2 for the North American isolates and its use for the differentiation between North American and European isolates of PRRSV.

### 7. Sequencing determination/analysis

The RT-PCR amplified products were purified from a 1.5% agarose gel with Geneelean II Nucleic acid Purification Kit (BiolOl). The genomic region was sequenced on both strands using U2 and D2 primers in an Automated Laser Fluorescent DNA sequencer (PERKIN ELMER, ABI Prism™ 310 Genetic Analyzer), Computer analysis of the nucleotide and sequences was performed using the sequencing analysis 3.4 program as well as MAC DNAsis Version.2.4 software. Nucleotide homologies were also calculated using the MAC DNAsis Version 3.6 program and Geneworks Version 2.2 program (Intelligenetics, Inc., Mountain View, California, USA).

The obtained nucleotide sequences of the ORFIb of the 3 feld PRRSV isolates were compared to the published sequences of a reference US strain (ATCC VR-2332) and a reference European strain (LV) as well as the reference NVSL. Table 28 shows no nucleotide substitutions, deletion or insertion among the 3 field PRRSV isolates.

| **Table 28** | |
|---|---|
| **Comparison of the nucleotide sequences of the polymerase (ORFIb) gene of the field isolates PRRSV with the sequences of the reference NVSL, European (LV) as well as the US (VR-2332)** | |
| AV-ORFIb | |
| D2-ORFIb | |
| D3-ORFIb | |
| LV-ORFIb | |
| NVSL-ORFIb | |
| VR2332-ORFIb | |

Phylogenetic tree analysis based on the nucleotide sequences on the polymerase gene of the filed isolates as well as the reference NVSL, European (LV) and US (VR 2332) isolates was performed. A 17.5% nucleotide homology was observed between the LV-ORF1b strain and the D2-ORFIb, VR2332-ORFIb, NVSL-ORFIb, D3-ORFIb and AV-ORFI b strains; a 95.1% nucleotide homology was observed between the D2-ORFIb strain and the VR2332-ORFIb, NVSL-ORFIb, D3-ORFIb and AV-ORFIb strains; a 95.5% nucleotide homologies of those field isolates (i.e., NVSL-ORFIb, D3-ORFIb and AV-ORFIb strains) was observed with the reference US strain (the VR2332-ORFIb strain, ATCC VR-2332); a 100% nucleotide homology was observed between the NVSL-ORFIb strain and the D3-ORF1b and AV-ORFIb strains; and a 100% nucleotide homology was observed between the D3-ORFIb and AV-ORF 1b strains.

The sequencing data of the PRRSV feld isolates were used to perform phylogenic analysis by Unweighted Pair Group Method with Arithmetic Mean (Geneworks Version 2.2). Accordingly, the field PRRSV isolates were grouped in the North American genotype (the NVSL-lb, Av-Lb, D3-1b, VR-2332 1b and D2-OREIb strains) distinct from the reference European LV strain (LV-ORF1b).

Thus shown herein, RT-PCR can be used for the detection of several RNA viruses. This technique was applied in the present example in order to characterize the strains of the field isolates collected from Guangzhou, so as to confirm the transmission of the new porcine virus from either North America or Europe to Mainland China, It has been indicated that it is a suitable diagnostic procedure not only for detection but also for differentiation between North American and European strains of PRRSV Mardassi et al., Can J Vet Res 58, 55-64; Mardassi et al., 1994. J Clinical Microbiology. Sept, 2197-2203 (1994). Meulenberg et al., (1993, Virology 192, 62-74) showed that most of the PRRSV isolates were able to cultivate in PAM (Porcine Alveolar Macrophages) cells from lung homogenates that supported virus replication. Until recently, propagation of North American PRRSV isolates was achieved in the MARC-145 cells highly permissive cell clones of PRRSV derived from the MA-104 cell line (Kim et al., 1993, Arch Virol 133, 477-483). In the present example, virus isolation in MARC-145 was successful, it was found to be permissive to the Guangzhou PRRSV field isolates.

### Determination of ultra-structural characteristics

Ultracentrifugation of concentrated extracellular virus yielded an opalescent band at the bottom of the tube corresponding to 50% sucrose density gradient. Viruses in the fractions from density gradients were spotted on forrnav-carbon-coated grids by floating the grid on 30 µl aliquots of fraction from density gradients for 30 sec and then negatively stained with 3% aqueous phosphotungstic acid (PTA) pH 6.3 for 30 sec. The grids were examined on a Philips EM 208s electron microscope at different potentials after irradiating the grid with UV light for 15 min. Negative stain electron micrograph of the AV and NVSL reference viral particles observed in sucrose gradient fraction of 1.34 g/ml under different potentials (90 kV and 31.5 kV). Through electron microscopic examination, this gradient fraction contained numerous spherical, enveloped viral particles 60 nm in diameter with cellular debris.

The morphological characteristics of the AV field isolates of PRRSV were also in agreement with previously described tissue culture adapted from ATCC-VR2332 American isolate of the PRRSV (Murphy et al., 1992, Vet. Mcrobiol. 32, 101-115; Pirzadeh, B., Gagnon, C, A. and Dea, S., 1998, Can J Vet. Res. 62, 170-177).

### 9. Viral polypeptide identification

Sucrose gradient purified preparations (i.e., fractions from 50% to 30%) of the feld PRRSV isolates AV and the reference NVSL PRRSV were analysed by SDS-PAGE under non-reducing conditions, and viral polypeptides were deduced from Western immunoblotting experiments using commercially available homologous hyperimmune sera of pigs as the source of specific virus antibodies. Both the SDS-PAGE and Western immunoblotting assay were done according to the Fritsch, Maniatis and Sambrook, 1989, Molecular Cloning, A Laboratory Manual. (Second Edition. pp 18.47-18.75). In

Western immunoblotting, reference hyperimmune serum (Animal and Plant Health Inspection Service, National Veterinary Services Laboratories, Ames, IA), Alkaline Phosphatase (AP) labeled rabbit anti-porcine IgG (Zymed) and enzyme-substrate solution consisting of NBT and BCIP bufer solution. (Zymed, S. San Francisco, California, USA) were used. The relative migration distances of the bands observed in the gels correspond to approximate MW of 15 kDa, 19 kDa, 26 kDa, 45 kDa and 80 kDa. Only the 15 kDa, 19 kDa, 26 kDa and 45 kDa viral polypeptides were immunoprecipitated using the positive control antisera.

From the result of the Western immunoblotting experiments, it appears that from the bands obtained by SDS-PAGE, only those corresponding to estimated MW of 15 kDa, 19 kDa, 26 kDa and 45 kDa may in fact represent viral proteins. Other bands detected by SDS-PAGE could correspond to cellular proteins co-purified with the extracellular virions. The polypeptide patterns identified for the Guangzhou field isolate AV appear identical to those reported for the American reference isolate ATCC-VR2332 propagated in the continuous CL2621 cell line (Francki et al., 1992, Arch Virol. 2, 220-222) as well as the reference NVSL. As mentioned by the others (Plageman et al., 1992, Adv. Virus Res. 41, 91-192; Mardassi et al, 1994), these polypeptide patterns are compatible with those determined for EAV and LDV. By analogy to EAV and LDV, the 15 kDa, 19 kDa and 26 kDa polypeptides identifed for PRRSV represent the major nucleocapsid protein N, the matrix protein M and the envelope protein E respectively, while the 30 kDa, 31 kDa and 45 kDa polypeptides represent minor structural protein of the viruses.

### EXAMPLE 6

### Demonstration of Isolation and Characterization of a Chicken Pathogenic Virus

The example herein demonstrates the isolation and characterization of different sub-strains of chicken Infectious Bronchitis Virus (IBV) based on Si gene diversity. Five regional I13V isolates collected from different geographical region in Southern China were characterized with PCR sequencing. A pair of primer flanking the whole S-1 gene of the IBV was designed according to the published sequence data, and the expected PCR products size is 1760-base pair. The resulting PCR product was further sub-cloned into pGEM-T easy vector and subjected to sequencing. The analysis of the genetic relationship among the isolates indicates the diversity of S-1 gene of the 5 isolates in Southern China was very high. The nucleotide variation among these 5 isolates was ranging from 8% to 48%, and the 5 isolates could be classified into 3 groups according to the phylogenetic tree analysis. Two conserved regions and two hypervariable regions were also identified among these isolates. This example indicates chicken farms at different regions of China should vaccinate their chicken with their respective genotype matched vaccine strains of the particular region so as to prevent failure of vaccination. Additionally, other pathogen substrains can be identified for more effective vaccine preparation and administration in accordance with the present invention.

### Viruses Collection

Field IBV isolates VI, V2, V3, V4 and V5 were obtained from Yunnan, Hunan, Hubei, Guangxi, and Guangdong provincial veterinary service station of China.

### Virus Isolation

Each isolate was propagated and titrated in 10-day-old specific-pathogen-free (SPF) embryonated chicken eggs at 37°C for 48 hr 500 microliters of allantoic fluid was collected from inoculated embryos and centrifugated at 2500 x g for 5 min at 4°C. After centrifugation, the supernatant was collected; IRV genomic RNA was isolated according to manufacturer's instructions (Gibco BRL, Grand Island, NY).

### RT-PCR

Viral RNA was used as a template to reverse transcribe the first strand cDNA. The superscriptase RT kit was used per manufacturer's instructions (Gibco BRL, Grand Island, NY). cDNA was synthesized from 1 pl (200 ng) viral RNA primed with random hexamers. Amplification of cDNA was performed in a volume of 25 µl that included 2.5 pl 10 x PCR reaction buffer, 0.5 µl 10 mm dNTP mix, 0.5 25 mm MgCl₂, 1 µl cDNA, 1 µl Taq DNA polymerise and 50 pmole of each primer. Adjust volume to 25 µl with distilled water. PCR amplification was performed for 35 cycles (44°C 1 min, 52°C 2 min, 72°C 2 min), with a final elongation step of 10 min at 72°C. Using a robocycler PCR apparatus. S1 gene was amplified by using a 1760-base pair primer. All the isolates yielded a 1760-base pair fragment with 1760-base pair primer. A Lambda DNA/*Hind*III marker was used to size the clone fragments. This result confirmed all isolates were IBV. The isolated were designated V1, V2, V3, V4 and V5 respectively.

### Cloning and Sequencing S-1 Gene of the 5 Isolates

The S-1 gene PCR products were purified by using a Geneclean II kit (101 Bio, Co), and cloned into the plasmid pGEM-T easy vector (Promega) according to the manual, Plasmid PCR and EcoRI digestion was used to confirm the right clone (pT-S). A Lambda DNA/*Hind*III marker was used to size the clone fragments. The clone was sequenced by primer walking strategy, primer and its position was listed in Table 30 and the resulting sequence of the isolates were compared by MacDNAsis and PAUP (Hitachi software engineer Co. Sun brew, CA).

| **Table 30** | | | | |
|---|---|---|---|---|
| **Primers used for PCR and sequencing** | | | | |
| **Primer** | **Sequence** | **Strain** | **PCR Product** | **Position** |
| Primer-1 | 5' CCGAATTCGCTATGAAAACTGAACAAAA 3' (SEQ ID NO:29) | + | 1760 bp | -124 |
| Primer-2 | 5' GGGTCGACATCCATAACTAACACAAGGG 3' (SEQ ID NO:30) | - | | 1636 |
| IBV-F | 5' TCAAAGCTTCANGGNGCNTA 3' (SEQ ID NO:31) | + | 573-601 bp | 127 |
| IBV-R | 5' CTCGAATTCCNGTRTAYTGRCA 3' (SEQ ID NO:32) | - | | 708 |
| IBV-FOR | 5' GTATTCTGCTTTAAAAAG 3' (SEQ ID NO:33) | + | 788-800 bp | 395 |
| IBV-REV | 5' AGCTCACCACTATAAACA 3' (SEQ ID NO:34) | - | | 1183 |

The whole sequence of Si gene was obtained by sequencing with three primer pairs (primer-1 and primer-2; IBV-F and IBV-R; IBV-FOR and IBV-REV). In order to get the whole sequence of Si gene, a 800-base pair fragment of S-1 gene for the VI, V2, V3, V4 and V5 isolates was RT-PCR amplified; and three positive clones subcloned into pGEM-T easy vector.

Nucleotide sequences in the N-terminal region of the S-I gene (Table 31) revealed two hypervariable regions and two conserved regions identified by Keeler et at., in the five isolates.

| **Table 31** | | | | |
|---|---|---|---|---|
| **S-1 gene sequence differences** | | | | |
| | **CK4 Positions 43 to 47** | **HV1 Positions 54 to 68** | **HV2 Positions 116 to 141** | **CK2 Positions 229 to 236** |
| D41 | HGGAY (SEQ ID NO:35) | SENNAGTAPSCTAG (SEQ ID NO:36) | | ..CQYNTG (SEQ ID NO:38) |
| V5 | HGGAY | .TISYNACTA..CTAG (SEQ ID NO:39) | .KAGSN..GLIPKGQIRISAMR-SVNSRLHL (SEQ ID NO:40) | ..CQYNTG |
| Beaudette | HGGAY | .SENNAGTAPSCTAG (SEQ ID NO:41) | | ..CQYNTG |
| V3 | HGGAY | .SENNAG-APSCTAG (SEQ ID NO:43) | .KSGSNSCPLTGLIPKGQIRIRMASVNSRLTI (SEQ ID NO:44) | ..CQYNTG |
| Holte | HGGAY | .SENNAGTAPSCTAG (SEQ ID NO:45) | | ..CQYNTG |
| V2 | HGGAY | .SENNAPTQY.SCTAG (SEQ ID NO:47) | | ..CQYNTG |
| V4 | HGGAY | .TISY..TAPSCT----AG (SEQ ID NO:49) | ..ISGSNSCPLTGLIP..RISAMR-NVNSRLLI (SEQ ID NO:54) | ..CQYNTG |
| V1 | HGGAY | .SEINAG.......SCTAG (SEQ ID NO:51) | .KAGSNSC..LIPKGQIRISAMR-SVNSRLHA (SEQ ID NO:52) | ..CQYNTG |

### E. Genotype Analysis

Phylogenectic tree analysis based on the comparison of S-1 nucleotide sequences of 8 IBV strains was conducted. The five IBV field isolates collected from Southern China were highly heterogeneous on the basis of nucleotide sequences (range from 48%-8%). V4 and V5 were categorized as belonging to the same group, and are closer genetically due to a high homology (92%) with the Holte strain than the mass-2 (Beaudette strain). V1, V2, and V3 were classified into different groups. VI belongs to a new type. V2 and V3 also showed some homologous with Holte, but there was also a big variation between them. The D41 was highly homologous with mass-2 (Beaudette strain).

Two hypervariable regions 56-69 and 117-133 and two conserved regions 43-47 and 229-236 in the N-terminal amino acid residues of the S-1 gene of Mass-typed IBV had been recognized previously. Both hypervariable regions and conserved regions were identified among the 5 isolates. The results indicate these isolates were mass-typed. However, the phylogentic tree analysis showed the isolates had some difference with mass-typed strain. Therefore, it is contemplated that the mutation of mass-typed vaccine had lead to the emerging of field virulent IBV in Southern China.

The demonstrated high diversity among these isolates indicates that chicken farms at different regions of China may vaccinate their chicken with their respective genotype matched vaccine strains of the particular region. It is contemplated that such genotyping of pathogens (e.g., IBV) may allow genotype specific the production and utilization of the vaccines and methods of the present invention.

### EXAMPLE 7

### Demonstration of an Immunization of a DNA Vaccine against Hog Cholera Virus

The example herein demonstrates the immunization of DNA vaccine with improved efficacy. To demonstrate an improved efficacy of a DNA vaccine against hog cholera virus, a formulated crude DNA vaccine was prepared. After preparation and characterization, the crude bacteria DNA vaccines were administrated to experimental animals and immune responses induced were compared with immunization with naked DNA. A significant improvement in immunogenicity over naked DNA was achieved for both antibody and CTL induction. Specifically, a bacteria DNA vaccine against hog cholera virus induced significantly enhanced serum antibody responses (humoral) and cytotoxie T lymphocyte (cell-mediated) responses in comparison to naked DNA after i.m. immunization in rabbit.

### A. Plasmid DNA

Immungenic UNAs pHCV2.5 and pHCV1.25, constructed with the cytomegalovirus promoters that drive expression of the glycoprotein E2 gene of HCV, have been described herein.

### i. Immunization and Vaccine Formulation.

To induce immune responses against HCV, animals were immunized with the following protocol. Female rabbit (1 kg --2 kg) were purchased from animal center of Hong Kong University. Rabbit were fundamentally immunized with a single intramuscular injection into the right biceps femurs muscle of 0.1 ml and 0.5 ml crude bacteria preparations, 0,1 ml formulated vaccine is equal to 100 g naked plasmid DNA and so on. Booster occurred twice at 7 days interval. The formulated vaccine is prepared as follows:

Inoculate 1 ml bacteria seeds that are propagated from a purified colony to 1.51 LB broth added with I mg/ml ampicillin. Incubate at 37°C and shaked with 200 rpm overnight. Spin down and get the bacteria pellet. Weighing and reconstitute to a given concentration, sonication for 10 min, and then add-1 mg/1 ampicillin overnight for administration.

### ii. Anti-DNA Antibody Responses (ELISA)

An enzyme-linked inumunosorbent assay (ELISA) kit was used to determine antibody responses against HCV according to the manufacturer's manual (IDEXX-Co). The rabbit inoculated with a formulated DNA vaccine had a highest ELISA titer among all the rabbits two weeks later; it is even higher than commercial vaccine. Compared with the NA parameters, the data matched.

### iii. Flow Cytonietry

Preparation of single cell suspensions (Peripheral blood PBLs), Whole-blood samples were collected by cardiac puncture into heparinized syringes and placed on ice. The blood was diluted in Han's balanced salt solution (HBSS) without phenol red plus 1.5% fetal bovine serum (Sigma Chemical Co. St. Louis, Missouri) and sieved through a 15 ml rayon wool column primed with HBSS. Five ml of the filtered blood was layered over 5 ml Ficoli Hypaque (Sigma) and centrifuged at 2000 rpm for 10 min. The interface buffy coat was removed with a Pasteur pipette and rinsed three times with 5 ml HBSS. After the final rinse, total lymphocyte from 104 cells per sample was determined by analysis on a flow cytometric apparatus.

Virus neutralization test for HCV CSFV neutralizing antibodies (VNAb) were titrated by the rapid fluorescent focus inhibition test (RFFIT) with modification, PK.15 was used for the detecting of VNAb against CSFV. Anti CSFV VNAb titers are expressed in serum dilution, Using CSFV C strain as the reference or as the reciprocal serum dilution (rd) that inhibited 50% of the fluorescent focus.

### iv. Fever Response in Rabbit after a Challenge Test

Based on the principle of virulent HCV cannot cause fever of rabbit under normal circumstances, while laprinized HCV vaccine can cause fever in the rabbit. Therefore, it was contemplated that should formulated DNA vaccine could induce immune response in rabbit, it will neutralize the vaccine strain inoculated thereafter, and there will be no fever response.

Five rabbits (2 kg-2.5 kg) were used in this demonstrated. Rabbit No.1 and No. 2 were injected with high dose and low dose formulated DNA vaccine, No. 3 and No. 4 were injected with naked plasmid DNA and conventional vaccine respectively, No. 5 were set as control.

Firstly, each rabbit was primed with above materials on day 0. After rabbits were intravenous inoculated with a laprinized vaccine, formulated vaccine and naked plasmid DNA respectively and the last two was bolstered after 2 weeks. Then, each rabbit was bolstered twice at 1 week intervals. Then the rabbits were challenged with the laprinized vaccine strain two weeks later, and the body temperature determined for each rabbit every day for I week.

From the temperature curve, control rabbit had a progressive fever, rabbit No. 3 immunized with the naked plasmid DNA had a fever 4 days later; rabbit inoculated with formulated vaccine both low dose and high dose had no fever response. The results indicated formulated DNA vaccine had the same efficiency as the HCV laprinized vaccine, while naked plasmid DNA could delay fever 4 days, compared with a formulated vaccine; it only had partially protective capability.

### v. Histopathological Examination of the DNA Vaccine-Injected Muscle

7 days after challenge, muscles of rabbit were resected and fixed with 10% buffered formalin and were embedded in paraffin. Sections were directly used for light microscopic observation. Rabbit injected with naked DNA and conventional commercial vaccine was set as control.

A histopathological section injected with formulated vaccine showed massive accumulation of mononuclear cells, indicating that strong inflammation was caused by the injection, furthermore, destructive changes of the muscle fibers associated with severe inflammatory cell recruitment were noted, While in the muscle injected with naked DNA and conventional commercial vaccine, the degree of mononuclear cell infiltration and muscle fiber change were distinguishably mild.

### vi. Formulated DNA Vaccine in Humoral Immunity (VN)

When rabbit was inoculated with a bacteria preparation of IICV DNA vaccine (pHCV2.5), Both of the rabbit inoculated with low dose or high dose had a humoral response two weeks later, after booster, The VN titer continued to increase, it still remained at a high level I week after challenge. Compared with the naked plasmid DNA and conventional commercial vaccine, the response of neutralization antibody in the rabbit inoculated, with the formulated DNA vaccine was even better over them.

### vii. Formulated DNA Vaccine in Cell-Mediated Immune Response

The un-clotted blood of the rabbit was analyzed by using a flow cytometery, mphocyte, monocyte and granular cell of the peripheral blood was counted respectively. The results showed rabbit immunized with the formulated DNA vaccine had a highest concentration of inflammatory cells, especially the granular cell. Rabbit No. 2 immunized with a high dose of formulated DNA vaccine had a higher concentration of granular cells; it reflects the non-specific immune response of the rabbit had been greatly enhanced. The rabbit injected with a low dose of formulated DNA vaccine had a relatively low concentration of these cells.

### viii Bacterial DNA Vaccine Properties

This example demonstrated that a bacterial formulated DNA vaccine had a better effect over naked DNA vaccine; Superior parameters of Ab titer (ELISA), neutralization activities, cellular immune response and fever response were demonstrated for the bacterial DNA vaccine.

Histopathological examination of inflammatory cells and formulated DNA vaccine injected muscle revealed massive accumulation of inflammatory cells and segmental degeneration and necrosis of myocytes. These characteristics of the DNA bacteria vaccine indicate development strategies for effective DNA vaccine construction, preparation and administration. For example, in immunized rabbits with formulated DNA vaccine will cause a cellular immune response. The higher the inoculation dose, the higher immune response it will cause: demonstrating superior properties than the commercial vaccine. It is contemplated that these characteristics may be this is due to the bacteria protein or bacteria sequence. In contrast, the effect caused by naked DNA was not as significant as formulated DNA vaccine. Moreover, it is contemplated that the rabbits had some endurance to the endo-toxin of bacteria, and/or the long term treatment to the bacteria vaccine preparation by ultrasonic techniques produced reduced toxicity, since the bacteria vaccine preparations didn't cause fever or death and clinical symptom to the rabbit. Additionally, there was no gross lesion in the injection site, and the rabbit injected with a low dose had been well absorbed, only the rabbit injected with a high dose had some tyromatosis, indicating it had a strong inflammation response once before.

### EXAMPLE 8

### Demonstration of a DNA Vaccine against a Regional strain of PRRSV

This example demonstrates a DNA vaccine in the generation of both humoral and cellular immune responses and the ability of a crude bacteria formulation to act as an adjuvant. Specifically, this example demonstrates the immunogenicity of the DNA plasmid constructs in pigs and the use of the ORF 5 polypeptide of PRRSV for specific recognition by T cells to elicit cell-mediated immunity.

An understanding of the humoral immune responses to structural proteins of PRRSV following vaccination is useful for development of an effective vaccine strategy. Furthermore, the production of recombinant vaccines depends on selection of a viral protein that will induce the proper antibody response to protect the animal from disease. The development of humoral immune responses described herein was monitored by ELISA, viral neutralization (VN) and immunoblotting assays (Nelson E.A., et al., (1994). J. Veterinary Diagnostic Investigation 6, 410-415). The role of cell-mediates immunity (CMI) in protection against viral diseases had been widely documented. Most notably, it has been shown that cytotoxic T cells are critical for viral clearance of a number of viruses. The identification of epitopes recognized by T cells has proven to be useful in the design of subunit vaccines for induction of effective immune responses to various microbial pathogens. Previous studies have demonstrated that infected pigs develop CMI responses to PRRSV (Bautista E.M., et al., (1996). Archives of Virology 14, 1357-1365). However, it was unknown whether PRRSV polypeptides differ in their ability to elicit T cell immune responses in swine.

PRRSV ORF 5 envelope protein and ORF 6 matrix protein regions were chosen for the production of recombinant construct as a DNA vaccine. As mentioned (Pirzadeh B. and Dea S. (1997). J General Virology 78, 1867-1873), PRRSV GP has a role in virus infectivity and may function in attachment to cell receptors and/or in virus penetration into the cytoplasm of target cells. It is indicated that at least one neutralizing antigenic determinant is associated with PRRSV GP. Since PRRSV GP, is rather abundantly present in the virion and is partially exposed in association with the lipidic envelope (Mardassi H., et al., (1996). Virology 221, 98-112; Meulenberg J.J.M, et al., (1995). Virology 206,155-163), it shows that GP, is the major viral envelope glycoprotein being recognized by most convalescent pig sera (Meulenberg JJ.M, et al., (1995). Vrology 206,155-163; Nelson E.A., et al., (1993). J Clinical Mcrobiology 31, 3184-3189). Then in the case of ORF 6 matrix protein, Bautista (Bautista E.M., et al. (1999). Archives of Virology 144, 117-134) indicates that the-greater T cell proliferation response induced by in vitro stimulation with the product of ORF 6, as compared to the other polypeptides of PRRSV. It indicates that the matrix protein may have a major role in cell mediated immunity to PRRSV. The matrix polypeptide gene is the most conserved gene among all the PRRSV isolates tested (Kapur V., et al., (1996). J General Virology 77,1271-1276; Meng X.J., et al., (1995). Archives of Vrology 140, 745-755; Meng X.J et al., (1995).J General Virology 76, 3181-3188; Meng X.J., et al., (1996). J General Virology 77, 1265-1270; Meng X.J., et al., (1994). J General Virology 75, 795-801; Murtaugh M.P., et al., (1995). archives of Vrology 140, 1451-1460), indicating that the structure of this polypeptide may be essential for the assembly of PRRSV. The matrix protein of PRRSV was then selected for a vaccine of the present invention to demonstrated immunization.

The genomic regions encoding ORFs 5 and 6 of the regional strain PRRSV were selected and cloned into the mammalian expression vector pcDNA3.1(+) to construct a DNA vaccine. DNA immunization with plasmid encoding GPS or ORF 6 of PRRSV, under the control of a human cytomegalovirus promoter, induced anti-GP5 neutralizing antibodies or ORF 6 specific antibodies in both Balbfc mice and its natural host, pigs. The GP5 protein specificity of neutralizing sera was confirmed by immunoblotting, ELISA as well as neutralization assay. This result indicates that neutralizing epitopes for this regional strain PRRSV is present on the viral envelope glycoprotein only.

Inoculation of crude bacteria conjugated with ORF 5-plasmid construct resulted in a stronger antibody level than with plasmid construct alone as shown in the ELISA test. In addition, cellular immune response was detected in immunized mice and pigs by flow cytometrically counting the increase in number of T cells (CD4+ and CD8+ populations. It was demonstrated that ORF 5 plasmid construct with crude bacteria as adjuvant may be a used as a vaccine against this regional PRRSV.

### A. Animals

Seven-week-old male Balb/c mice were purchased from the Laboratory Animal Unit of the University of Hong Kong and separated in groups of five mice per cage. Mice were randomly divided into seven experimental groups. Eight piglets weaned at 5 weeks of age were obtained from a breeding farm in the Department of Veterinary Medicine, South China Agricultural University, Guangzhou, 510642. The breeding piglets were tested and proven to be seronegative for PRRSV. The piglets used in this example were randomly divided into four groups.

### B. Virus

The regional strain AV Guangzhou feld isolate PRRSV was initially isolated from tissue samples of PRRSV infected pigs in Guangzhou and propagated in MARC-145 cells, a clone of MA-104 cells highly permissive to PRRSV (Kim H.S., et al., (1993). Archives of Virology 133,477-483). Virus titers were expressed as tissue culture infective dose 50 (TCID50) per ml, as previously described (Dea S., et al., (1992). Canadian Veterinary Journal 33, 801-808).

### C. Recombinant Constructs

Viral RNA was extracted from AV strain PRRSV-infected MARC-145 cells as previously described (Mardassi H., et at, (1995). Archives of Virology 140,1405-1418). Oligonucleotide primers enabling the amplification of PRRSV ORFs 5 and 6 were designed based on the-nucleotide sequence of the genome of the American strain VR-2332. HindIll or Xbal restrictions (RE) sites for ORF 5 region and BamHI or EcoPJ for ORF 6 region were incorporated at the 5' end of the primers to facilitate cloning. The ORF 5 and ORF 6 regions were first cloned into the TA-cloning vector (pGEM®-T and pGEM®-T Easy Vector Systems Promega) and then directly inserted into specific RE sites of the pcDNA3.1(+) expression vector (Invitrogen) and resulted in the production of pcDNA3.1-ORFs 5 and 6 constructs. Recombinant constructs were verified by sequencing and then transformed into E. Coli strain, Top-10. Bacterial culture pellet was dried and sonicated in preparation for injecting the animals.

### D. Transient Expression of the Recombinant Proteins

In vitro expression of the pcDNA3.1(+)-ORF 5 or 6 constructs were tested in transient expression experiments in HEIR-293 cells maintained as confluent monolayers. Cells in 6-well tissue culture plates were transfected with 5 pg plasmid DNA by liposome. For immunoflourescent staining, cells were incubated at 37°C and fixed with 37% formalin for 15 minutes at room temperature at various times (18 hours-72 hours) post-transfection. The monolayers were then reacted for 1 hour with anti-PRRSV polyclonal antibody (National Veterinary Services Laboratories, Ames, IA) and the immune reaction was determined following incubation with FITC-conjugated secondary antibody (Zymed).

Transient expression of the cloned ORF 5 and ORF 6 gene were detected by immunofluorescent staining. Expression of the protein product was demonstrated in HEIR-293 cells at 72 hours post-transfeetion. The identification of the protein product was confirmed by using porcine anti-PRRSV serum. Secondary antibody with FITC-conjugated was added to give signals to the transfected cells. Green color fluorescent signals were detected under fuorescence microscopy. The transfected cells showed bright homogenous cytoplasmic reaction by using polyclonal antibody and the expressed proteins tended to accumulate near the perinuclear region. In order to confirm the expressed proteins represented the PRRSV envelope protein and the matrix protein, Western immunoblotting was carried out using the porcine anti-PRRSV serum, Specific protein bands with size 26 kDa and 19 kDa were observed in either lane, which corresponded to envelope and matrix protein respectively.

### E. Mice Immunization Schedule

hi vivo expression ofpcDNA3.I-ORF 5 or 6 was verified by immunizing groups of five Balb/c mice with 500 µg of either ORF 5 or ORF 6 construct or 100 jig of ORF 5 construct; a mixture of 100 µg of each construct or crude bacteria with 50 µg or 10 µg of ORF 5 construct plasmid equivalent diluted in 250 µl PBS. DNA was injected into the tibialis cranialis muscle with a 27-gauge needle. The mice were boosted twice with the same quantities of DNA at 2-week intervals. Control mice received 100 leg of pcDNA3.1(+) vector via an identical route.

Following DNA inoculation, the protein specificity of mouse sera to GP5 or ORF 6 was established by ELISA and by immunoblotting with expressed proteins. Antibodies against PBRSV antigen in the ELISA were tested positive at various time post-immunizations, indicating a humoral immune response had been generated. In general, antibodies specific to PRRSV were increased in all mice after the first booster of the constructs (day 0). The second booster occurred on day 14. Antibody titer rose to maximum value by 21 days post-immunization (PI) for groups injecting with DNA plasmid constructs and then began to decline. But for groups injecting with crude bacteria with plasmid equivalent, antibody titer rose to maximum value by days 14 PI and remained at a steady high level. Serum neutralization assay was performed using day 35 mice sera. Sera from three of the four mice inoculated with pcDNA3,1-ORF 5 and one of the three mice inoculated with mixture of pcDNA3,1-ORES and pcDNA3.1-ORF6, together with four of the five mice inoculated with crude bacteria pcDNA3.1-ORF 5 plasmid equivalent demonstrated in vitro neutralizing activity at day 35 of PI, Neutralization was not observed in mice injected with pcDNA3,1-ORF 6, or sera obtained from the vector-injected control mice and pre-immune sera, These results indicate that PRRSV-neutralizing antibodies were specifically targeted to the epitopes of proteins encoded by ORF 5 of PRRSV.

### F. Pig Immunization Schedule

Two piglets in each group were injected three times at 2-week intervals with 100 µg ORF 5 plasmid construct or crude bacteria with 100 µg or 500 µg of ORF 5 plasmid equivalent diluted in 5 ml PBS. PIG 1 and PIG 2 were administered 100 jg ORF 5 plasmid construct; PIG 3 and PIG 4 were administered 100 µg control vector peDNA3.1; PIG 5 and PIG 6 were administered crude bacteria with 100 µg ORF 5 plasmid equivalent; and PIG 7 and PIG 8 were administered crude bacteria with 500 µg ORF 5 plasmid equivalent. Control piglets received 100 µg of pcDNA3.1 (+) vector via an identical route. Two-thirds of the volume was injected, using 22-gauge needle, into the tibialis cranialis muscle of the leg and one-third was intradermally administered into the dorsal surface of the ear.The first booster was administered day 0, the second booster was administered day 14 and the third booster administered day 28. Preimmune and hyperimmune sera and blood lymphocytes were collected from all mice and pigs prior to each plasmid inoculation to evaluate their immune responses.

Seroconversion was also demonstrated in pigs by ELISA whereas the protein specificity of pig sera to GP5 was established by immunoblotting with samples taken 35 days post-innoculation. All pigs developed antibody after immunization, but pigs injected with crude bacteria with ORF S plasmid equivalent (PIG 5-8) showed a stronger antibody response than pigs injected with ORF 5 plasmid construct only (PIG 1-2). Each pig may show variation in response towards vaccination, only PIG 6 and 7 developed a faster antibody response after the third booster, but only PIG 7 was able to maintain a high-level antibody level for about two weeks, then started to decline, Neutralizing antibodies were detected in sera of the DNA-immunized pigs only 2 weeks after the second booster injection and in vitro neutralizing activities still maintained until the end of the experiment. Thus, the ability of the DNA vaccine to produce a humoral immune response in another animal model (i.e., pigs) was demonstrated.

### G. Virus Neutralization and Serological Tests

Under some circumstances, as in vaccination, it may be desirable to enhance the normal immune response by administering an adjuvant with the antigen. Adjuvants enhance the body's immune responses to the antigen. A large variety of compounds have been employed as adjuvants. In the type of bacterial fractions, endotoxins enhance antibody formation if given about the same time as the antigen. They have no effect on delayed hypersensitivity, but they can break tolerance, and they have a general stimulatory activity. Endotoxins act by stimulating macrophage production of interleukin-1. Thus the use of crude bacteria as adjuvant shows substantial facilitating efects on the antigen-specific serum and antibody responses. To determine if the anti-ORF 5 and anti-ORF 6 protein monoclonal antibodies could neutralize virus infectivity, virus neutralization assay testis done on MARC-145 cells in the presence of 106.5 TCID50 of the virus.

Following DNA inoculation, humoral immune responses in each animal are monitored by ELISA and immunoblotting prior to the detection of antibody by the virus neutralization assay test. The virus neutralization assay test is less sensitive than the other tests; it could be affected by circulating immune complexes, or neutralizing antibody may actually not appear until later after initial infection. Also, the antigenic epitope(s) associated with vims neutralization may represent only one of many antigenic epitopes on a viral protein. Therefore, the immunoblotting assay detects antibody prior to the appearance of neutralizing antibody.

Mouse and pig sera were tested for the presence of specific anti-GP5 or ORF 6 antibodies by virus neutralization (VN), ELISA and Western immunoblotting tests. The VN tests was performed in duplicate with 100 1 serial dilutions of virus (10⁶⁴ TCID₅₀) in the presence of 50 fold dilution of heat-inactivated test sera, incubated for 60 minutes at 37°C. The mixtures were put in contact with confluent monolayers of MARC-145 cells seeded in 96-well plates 48 hours earlier. Cell monolayers were incubated at 37°C and observed daily for the appearance of cytopathic effect (CPE). Neutralizing index was expressed by subtracting the reciprocal of log10 highest virus dilution liter showing no CPE with test serum from log 10 liter with non-immune control serum. Humoral immune responses in each animal were monitored by ELISA using the PRRSV antibody test (IDEXX HERDCHEK_PRRS, Westbrook, ME) and Western blot assays was also performed (Pirzadeh B, and Dea S. (1997). J General Vrology 78, 1867-1873) using ORF 5 expressed protein as antigen.

*In vivo* expression of foreign proteins via simple injection of plasmid DNA with or without crude bacteria conjugate into mice and its natural host was demonstrated. It was found that circulating antibodies to PRRSV are detected on 14 days PI after the first dose booster by the ELISA test, whereas antibodies could still be detected by ELISA for the entire sampling period. Interestingly, it was found that those groups of animal injected with crude bacteria. as adjuvant showed a higher antibody level and maintained at a steady high level after the second booster rather than declining as in other groups with only plasmid injection. The lower liters of antibodies in DNA-immunized mice and pigs as compared to the crude bacteria-ORF 5 immunized animals could be explained by the fact that the injected antigens are available to the B cells and other antigen-presenting cells which can potentially stimulate a strong antibody response.

These results demonstrate the presence of neutralization epitopes on proteins encoded by ORF 5 but not on ORF 6 encoded proteins. ORF 5 of PRRSV is has been described to elicit neutralizing antibody in pigs and Balb/e mice (Pirzadeh B. and Dea S. (1998). J General Virology 79, 989-999). It is important to note that neutralizing antibodies can only be detected with the association of the conformational neutralizing epitopes on PRRSV protein. DNA constructs, poDNA3.I-ORF5 and ORF6, has an advantage in that expression in mammalian cells which mimic proper viral protein conformations. These constructs thus have the ability to drive antigen production in host animals resulting in immune responses that recognize native PRRSV antigens in a relevant and protective fashion (Kwang J., et at, (1999). Research in Veterinary Science 67, 199-201).

### H. Flow Cytometric Analysis of Peripheral Blood Lymphocytes (PBL)

Peripheral blood lymphocytes were prepared from heparinized blood. 0.2 ml of fresh whole blood was added into 5 ml of freshly prepared red blood cell lysing buffer (0.8% ammonium chloride, 0.083% sodium hydrogencarbonate and 0.003% EDTA-fee acid, pH 7.3) using PYREX Borosilicate glass disposable culture tube (CORNING, NEW YORK) until complete lysis of red blood cell. Lymphocytes were collected by centrifugation at 2000 rpm for 2 minutes. Lymphocytes were washed with 1 ml of 1 x phosphate buffered saline (PBS) by vortexing the tube and spun down at 2000 rpm for 2 minutes. Lymphocytes were then stained with appropriate FITC conjugated CD4 or R-PE conjugated CD8a (BD PharMingen) at the recommended concentration (1 µg per 1 x 106 cells) in darkness for 1 hour with constantly shaking. Before running the analysis in flow cytometer (EPICS ELITE ESP COULTER), 0.5 ml of wash buffer (0.1 % sodium azide, 1% Bovine Serum Albumin BSA in I x phosphate buffered saline PBS) was added into each tube to lop up the volume. At least 5000 cells were analyzed for each sample.

The ability of the DNA vaccine constructs to induce a cell-mediated immune (CMI) response was determined by estimating the increase in CD4+ and CD8+ T cells population at various times of post-immunizations in mice. Boosters for tests involving measurement of CD4+ were given on days 0 and 14. A negligible amount of lymphoproliferation in response to mice immunized with control DNA plasmid. 83% of the immunized mice demonstrated cell mediated immune responses. Most prominent was the change in the percentages of CD4+ T cells, which peaked on day 14 and 25 of PI at roughly 50% and 42% respectively above the level of day 0. Whereas in the percentages of CD8+ T cells, which peaked on day 14 and 28 of PI at roughly 52% and 37.5% respectively above the level of day 0.

On day 14 of PI, mice of group 5 inoculated with mixture of both constructs induced the strongest increase in percentages of both T cells. High percentages of T cells also showed in mice of group 4 inoculated with ORF 6 construct. These results indicated the ability of ORF 6 protein to induce higher T cell proliferation response.

In pigs, boosters for tests involving measurement of CD4+ were given on days 0, 14 and 28. Peripheral T cell populations of growing pigs showed then-numbers to drop transiently at day 14 post-immunization (PI) except CD8+ T cell populations in PIG 1. Further analysis on the overall trend of the T cell populations, it found an increase in CD4+ T cell populations, with a decrease in CD8+ T cell populations or vice versa. This observation was shown most significantly in PIG I and PIG 2. The decline in CD8+ T cell populations (a marker for cytotoxic T cells, which recognize vims-infected cells) continued for at least 3 weeks, whilst CD4+ cells (which include T-helper cells, involved in immunological memory) increased, peaking range from days 28-42 in different pigs. This result may confirm the ability of the PRRSV to suppress the immune system of its host as this result was not shown in mice.

Another characteristics of porcine T cells included a reversal of the CD4/GD8 ratio of T cell subsets, i.e., the population of CD8+ T cells is higher than CD4+ T cells in porcine, which was vice versa in human and mice. Moreover, the porcine T cell population was unique in that there was a large percentage of CD4+CD8+ dual expressing peripheral T cells, the percent of this dual expressing cells also increased with the age of the pigs. The result was evaluated in two-color flow cytometric analysis.

DNA constructs induced in vivo CMI responses by estimating the number of the individual T cells population i.e., CD4+ and CD8+ T cells. It demonstrated that during DNA constructs immunization in mice a strong influx of both CD4+ T cells and CD8+ cytotoxic T lymphocytes in the peripheral blood. This fnding strongly indicates that the presence of cytolytic cells in the peripheral blood during primary immunization is protective. Cytotoxic T cells are potent at lysis of infected cells and may thus prevent spread of the virus (Samsom J-N., et at., (2000). J General Virology 497-505).

Furthermore, this type of cells has been shown to regulate cellular immunity via the production of interferon-y whereas CD4+ T cells implicated in eliciting an antibody response.

On the contrary, peripheral T cell populations of growing pigs show their numbers to drop transiently at 14 days post-immunization. During the sampling period, it shows an increase in CD4+ cells and a decrease in CD8+ cells in young pigs or vice versa, An increase level of proliferation response afer secondary exposure was due to CD4+ cells that were effectors in this response. Many theories have been offered proposing a mechanism whereby PRRSV may alter the population of T cell subsets. Parallels with other virus infections give rise to possibility of CD4+ T cell death, perhaps with concurrent CD8+ stimulation or that the virus may act as the level of T cell intra-thymic differentiation (Drew T.W. (2000). Veterinary Research 31, 27-39). Shimizu (Shimizu M., et al., (1996). Veterinary IrrrnrunologicalImrrrunopathology 50, 19-27) proposed that the cause of CD8+ cell proliferation induced as a consequence of PRRSV infection. Based on the findings that PRRSV ORF 5 region has an apoptotic effect on transfected cells (Suarez P., et al., (1996). J Virology 70, 2876-2882).

PRRSV may cause immunosuppression in its host. It was uncertain that whether the decrease in CD8+ cytotoxic T cells correlated with the apoptotic ability of ORF 5 region of PRRSV. Apoptosis is a critical process in the normal functioning of the immune system. Thus the destructive of the cytotoxic effects of CD8+ T cells on target cells are all mediated by apoptosis. The difference of response in T cell population after immunization in mice and pigs showed that there was a variation in pathogenicity of the homologous virus among different hosts.

Pigs like other species have the typical CD4+CD8+ and CD4+CD8+ T lymphocytes in their peripheral blood and secondary lymphoid organs. These cells are shown to have helper and cytolytic functions. However, unlike humans and mice, swine also have a prominent CD4/CD8 double positive (DP) lymphocyte population, comprising between 8% and 64% of the circulating pool of small resting T lymphocytes (Zuckennann F.A-(1999). Veterinary Immunology and Immunopathology 72, 55-66). The relative proportion and the absolute number of this lymphocyte subset in the peripheral blood of swine increase gradually with age. This cell population represents an independent cell lineage with no direct relation to mature CD4 or CD8 single positive T cells. However, dual DP cells could represent memory CD4+ T helper lymphocytes that have acquired. the CD8 antigen upon prior sensitization and retained it afer reversion to small lymphocytes. These cells are capable of helping B cells to produce antibody. And because of the predominance of DP cells in older pigs, it is likely that these play a major role in protective immunity due to the co-expression of CD4 and CD8 might be advantageous for recognition of nominal antigen.

This example shows that DNA immunization with a plasmid encoding GP5 of PRRSV is able to develop both the humoral and cell-mediated immunity in mice and its natural host. Therefore, it appears that the ORF 5 may be a good candidate for a recombinant type vaccine against this regional PRRSV. However, the genomic variability of the ORF 5 genes which was recently reported between North American and European strains (Mardassi H., et al., (1995). Archives of Vriology 140, 1405-1418), indicates that these variabilities may be evaluated significance in terms of the antigenic determinants involved in protection for the production of a vaccine of the present invention.

DNA immunization is not sufficient to inhibit virus persistence and shedding in the respiratory tract after virus challenge (Pirzadeh B. and Dea S. (1998). J General Virology 79, 989-999). And it was found that mucosal immunity is believed to play a role in protection against PRRSV infection, virus persistence and shedding, thus this aspect of immunity against PRRSV was evaluated. Accordingly, efforts to develop a multivalent DNA vaccine for PRRS and associated serological tests under another PRRSV antigen, ORF 5, was attempted, however, and a preliminary humorat immune response obtained. Recently, an infectious clone was generated (Meulenberg J.J,M., el al., (1998). J Virology. 72, 380-387) and it is contemplated that this could be mutagenised at a specific site to introduce a marker or to reduce the virulence and develop a safe and effective marker vaccine for this virus.

### EXAMPLE 9

### Demonstration of a Bivalent Vaccine against Two Pathogens

The example herein describes construction of bivalent vaccine with efficacy against two pathogens. As would be understood by one of ordinary skill in the art, bi- or multivalent vaccines of the present invention may be created using modem molecular cloning technology in light of the disclosures herein:

Laryngotracheitis Virus (ILTV) is a DNA herpes virus that causes a severe upper respiratory disease in chicken. Infectious Bronchitis Virus (IBV) is a member of the coronavirus, a single-stranded RNA virus. They are highly species-specific and both viruses cause significant economic loss in the poultry industry if not under control. In one embodiment of the invention, a bivalent virus was created to both diseases. A recombinant vaccine was created by deleting the TK gene from the ILTV genome and inserting the SI gene subcloned from the 1BV genome. Recombinant virus was generated by transforming chicken embryo kidney cells with the recombinant ILTV-IBV construct. Recombinant virus showed to induce immunity against both ITLV and IBV in chickens challenged with ITLV and IBV. It is possible to administer the vaccine in water and/or mist.

### References

1. World Health Organization. Cumulative number of confirmed human cases of avian influenza A/(H5N1) (www.who.int/csr/disease/avian_influenza/country/cases_table_2008_06_19/en/index.ht ml).
2. Bresson JL, Perronne C, Launay O, et al. Safety and immunogenicity of an inactivated split-virion influenza A/Vietnam/1194/2004 (H5N1) vaccine: phase I randomised trial. Lancet 2006; 367:1657-64.
3. Lin J, Zhang J, Dong X, et al. Safety and immunogenicity of an inactivated adjuvanted whole-virion influenza A (H5N1) vaccine: a phase I randomised controlled trial. Lancet 2006; 368:991-7.
4. Treanor JJ, Campbell JD, Zangwill KM, Rowe T, Wolff M. Safety and immunogenicity of an inactivated subvirion influenza A (H5N1) vaccine. N Engl J Med 2006; 354:1343-51.
5. Treanor JJ, Wilkinson BE, Masseoud F, Hu-Primmer J, Battaglia R, O'Brien D, et al. Safety and immunogenicity of a recombinant hemagglutinin vaccine for H5 influenza in humans. Vaccine2001;19(13-14):1732-7.
6. Kodihalli S, Goto H, Kobasa DL, Krauss S, Kawaoka Y, Webster RG. DNA vaccine encoding hemagglutinin provides protectivebimmunity against H5N1 influenza virus infection in mice. J Virol 1999;73(3):2094-8.
7. Chen Z, Yoshikawa T, Kadowaki S, Hagiwara Y, Matsuo K,Asanuma H, et al. Protection and antibody responses in different strains of mouse immunized with plasmid DNAs encoding influenza virus haemagglutinin, neuraminidase and nucleoprotein. J Gen Virol 1999;80(Pt 10):2559-64.
8. Peggy A. Lalor,Richard J. Webby,Jane Morrow, Denis Rusalov,David C. Kaslow, Alain Rolland, and Larry R. Smith. Plasmid DNA-Based Vaccines Protect Mice and Ferrets against Lethal Challenge with A/Vietnam/1203/04 (H5N1) Influenza Virus. The Journal of Infectious Diseases 2008;197:1643-1652.
9. Gao W, Soloff AC, Lu X, Montecalvo A, Nguyen DC, Matsuoka Y, et al. Protection of mice and poultry from lethal H5N1 avian influenza virus through adenovirus-based immunization. J Virol. 2006;80(4):1959-64.
10. Epstein SL, Kong WP, Misplon JA, Lo CY, Tumpey TM, Xu L, et al. Protection against multiple influenza A subtypes by vaccination with highly conserved nucleoprotein. Vaccine 2005;23(46-47):5404-10 [Epub 2005 Jun 13].
11. Tompkins SM, Zhao ZS, Lo CY, Misplon JA, Liu T, Ye Z, et al. Matrix protein 2 vaccination and protection against influenza viruses, including subtype H5N1. Emerg Infect Dis 2007;13(3):426-35.
12. Toro H, Tang DC, Suarez DL, Sylte MJ, Pfeiffer J, Van Kampen KR. Protective avian influenza in ovo vaccination with non-replicating human adenovirus vector. Vaccine 2007;25(15):2886-91 [Epub 2006 Sep 25].
13. Ke-Qin, Yuka Hoshino, Yoshihiko Toda, et al. Immunogenicity and protective efficacy of orally administered recombinant Lactococcus lactis expressing surface-bound HIV Env. Blood, 2003
14. Robinson K, Chamberlain LM, Schofield KM, Wells JM, Page RWF. Oral vaccination of mice against tetanus with recombinant Lactococcus lactis. Nat Biotechnol. 1997;15:653-657.
15. Gilbert C, Robinson K, Le Page RWF, Wells JM. Heterologous expression of an immunogenic pneumococcal type 3 capsular polysaccharide in Lactococcus lactis. Infect Immun. 2000;68:3251-3260.
16. Hirt H, Erlandsen SL, Dunny GM. Heterologous inducible expression of Enterococcus faecalis pCF10 aggregation substance Asc10 in Lactococcus lactis and Streptococcus gordonii contributes to cell hydrophobicity and adhesion to fibrin. J Bacteriol. 2000;182:2299-2306.
17. Hee-Jeong Cho, Hye-Jeong Shin, In-Kwon Han, et al. Induction of mucosal and systemic immune responses following oral immunization of mice with Lactococcus lactis expressing human papillomavirus type 16 L1. Vaccine, 2007;17: 607-616.
18. Read RC, Naylor SC, Potter CW, Bond J, Jabbal-Gill I, Fisher A, et al. Effective nasal influenza vaccine delivery using chitosan. Vaccine 2005;23:4367-74.
19. Lavelle EC. Lectins and microparticles for enhanced oral vaccination. Methods 2006;38:84-9.
20. Davis SS. The use of soluble polymers and polymer microparticles to provide improved vaccine responses after parenteral and mucosal delivery. Vaccine 2006;24(Suppl. 2):S7-10.
21. Katz JM, Lu X, Young SA, et al. Adjuvant activity of the heat-labile enterotoxin from enterotoxigenic Escherichia coli for oral administration of inactivated influenza virus vaccine. J Infect Dis 1997; 175: 352-63.
22. Lu XH, Tumpey TM, Morken T, et al.Amouse model for the evaluation of pathogenesis and immunity to influenza A (H5N1) viruses isolated from humans. J Virol 1999;73:5903-11.
23. Suguitan AL Jr, McAuliffe J, Mills KL, Jin H, Duke G, Lu B, Luke CJ, Murphy B, Swayne DE, Kemble G, Subbarao K. Live, attenuated influenza A H5N1 candidate vaccines provide broad cross-protection in mice and ferrets. PLoS Med. 2006 Sep;3(9):e375.
24. Steensels M, Van Borm S, Lambrecht B, De Vriese J, Le Gros FX, Bublot M, van den Berg T. Efficacy of an inactivated and a fowlpox-vectored vaccine in Muscovy ducks against an Asian H5N1 highly pathogenic avian influenza viral challeng. Avian Dis. 2007 Mar;51(1 Suppl):325-31.
25. Hoelscher MA, Garg S, Bangari DS, Belser JA, Lu X, et al. Development of adenoviral-vector-based pandemic influenza vaccine against antigenically distinct human H5N1
26. strains in mice. *Lancet.* 2006;367:475-481.
27. David H. Holman, Danher Wang, Nicholas U. Raja, Min Luo, Kevin M. Moore, Jan Woraratanadharm, Nutan Mytle, John Y. Donga, Multi-antigen vaccines based on complex adenovirus vectors induce protective immune responses against H5N1 avian influenza viruses. Vaccine (2008) 26, 2627-2639.
28. Peggy A. Lalor, Richard J. Webby, Jane Morrow, Denis Rusalov, David C. Kaslow, Alain Rolland, and Larry R. Smith. Plasmid DNA-Based Vaccines Protect Mice and Ferrets against Lethal Challenge with A/Vietnam/1203/04 (H5N1) Influenza Virus. The Journal of Infectious Diseases 2008;197:1643-1652.
29. Ge, Q., McManus, M., Nguyen, T., Shen, C.-H., Sharp, P.A., Eisen, H.N., Chen, J. RNA interference of influenza virus production by directly targeting mRNA for degradation and indirectly inhibiting all viralRNA transcription. Proc.Natl. Acad. Sci. 2003; 100: 2718-2723.

## Claims

1. A composition for inducing an immune response to an antigen in a subject, comprising a genetically modified bacterium of the genus *Lactococcus* expressing the antigen hemagglutinin of avian influenza virus H5N1.

2. The composition of claim 1, wherein the bacterium is formulated into microcapsules.

3. The composition of claim 1, wherein the bacterium is complexed with a DNA expressing said antigen.

4. The composition of claim 1, wherein the antigen is capable of binding a glycosylated molecule on the surface of a mucosal cell membrane.

5. The composition of claim 1, wherein the antigen is a chimeric protein.

6. The composition of claim 1 for use in a method of inducing a protective immune response to an antigen in a subject.

7. The composition of claim 6, wherein the immune response is humoral immune response or mucosal immune response.

8. The composition of claim 6, wherein the composition is administered orally.

9. The composition of claim 1 for use as a medicament for inducing an immune response in a subject.

## Patentansprüche

1. Zusammensetzung zur Induktion einer Immunantwort auf ein Antigen in einem Patienten, umfassend ein genetisch modifiziertes Bakterium der Gattung *Lactococcus,* das das Antigen Hämagglutinin des Vogelgrippevirus H5N1 exprimiert.

2. Zusammensetzung nach Anspruch 1, wobei das Bakterium in Mikrokapseln formuliert ist.

3. Zusammensetzung nach Anspruch 1, wobei das Bakterium mit einer DNA komplexiert ist, die das Antigen exprimiert.

4. Zusammensetzung nach Anspruch 1, wobei das Antigen dazu in der Lage ist, ein glykosyliertes Molekül auf der Oberfläche einer Schleimhaut-Zellmembran zu binden.

5. Zusammensetzung nach Anspruch 1, wobei das Antigen ein chimäres Protein ist.

6. Zusammensetzung nach Anspruch 1 zur Verwendung in einem Verfahren zur Induzierung einer schützenden Immunantwort auf ein Antigen in einem Patienten.

7. Zusammensetzung nach Anspruch 6, wobei die Immunantwort eine humorale Immunantwort oder eine Schleimhaut-Immunantwort ist.

8. Zusammensetzung nach Anspruch 6, wobei die Zusammensetzung oral verabreicht wird.

9. Zusammensetzung nach Anspruch 1 zur Verwendung als ein Arzneimittel zur Induktion einer Immunantwort in einem Patienten.

## Revendications

1. Composition permettant d'induire une réponse immunitaire dirigée contre un antigène chez un sujet, comprenant une bactérie génétiquement modifiée du genre *Lactococcus* exprimant l'antigène hémagglutinine du virus de la grippe aviaire H5N1.

2. Composition selon la revendication 1, **caractérisée en ce que** la bactérie est formulée dans des microcapsules.

3. Composition selon la revendication 1, **caractérisée en ce que** la bactérie est complexée avec un ADN exprimant ledit antigène.

4. Composition selon la revendication 1, **caractérisée en ce que** l'antigène capable de se lier à une molécule glycosylée sur la surface d'une membrane de cellule de muqueuse.

5. Composition selon la revendication 1, dans laquelle l'antigène est une protéine chimère.

6. Composition selon la revendication 1, pour son utilisation dans un procédé d'induction d'une réponse immunitaire protectrice dirigée contre un antigène chez un sujet.

7. Composition selon la revendication 6, **caractérisée en ce que** la réponse immunitaire est une réponse immunitaire humorale ou une réponse immunitaire mucosale.

8. Composition selon la revendication 6, dans laquelle la composition est administrée par voie orale.

9. Composition selon la revendication 1, pour son utilisation comme médicament pour induire une réponse immunitaire chez un sujet.
